(19) Europäisches Patentamt
European Patent Office
Office européen des brevets

(11) EP 0 458 363 B1

(12) **EUROPEAN PATENT SPECIFICATION**

(45) Date of publication and mention of the grant of the patent: **07.02.1996 Bulletin 1996/06**

(21) Application number: **91108486.1**

(22) Date of filing: **24.05.1991**

(51) Int. Cl.$^6$: **C07D 473/00**, C07D 239/02, C07D 487/04, C07C 43/13, C07C 43/17, C07C 41/00, C07C 49/35, C07C 45/00, C07C 69/757, C07C 67/00, A61K 31/505

(54) **Fluorinated bis(hydroxymethyl) cyclobutyl purines and pyrimidines**

Fluorierte Bis(hydroxymethyl)cyclobutylpurine und -pyrimidine

Bis(hydroxyméthyl)cyclobutylpurines et pyrimidines fluorurées

(84) Designated Contracting States:
**AT BE CH DE DK ES FR GB GR IT LI LU NL SE**

(30) Priority: **24.05.1990 US 528304**
**14.08.1990 US 567034**

(43) Date of publication of application:
**27.11.1991 Bulletin 1991/48**

(73) Proprietor: **E.R. SQUIBB & SONS, INC.**
**Princeton New Jersey 08543-4000 (US)**

(72) Inventors:
- **Zahler, Robert**
  **Princeton, NJ (US)**
- **Vite, Gregory D.**
  **Lawrenceville, NJ (US)**
- **Goodfellow, Val S.**
  **Morrisville, PA (US)**
- **Ahmad, Saleem**
  **Plainsboro, NJ (US)**

(74) Representative: **VOSSIUS & PARTNER**
**D-81634 München (DE)**

(56) References cited:
**EP-A- 0 335 355**

- **CHEMICAL ABSTRACTS, vol. 75, no. 3, July 19, 1971, Columbus, Ohio, USA U.R. POLISHEHYLZ et al. "Esters of alpha-mercurated fluorocarboxylic acids. II. Reactions with bromine and benzoyl chloride." page 477, abstract-no. 20 552h & Zh. Vses. Khim. Obshchest. 1971, 16(1), 114-15**

Remarks:
The file contains technical information submitted after the application was filed and not included in this specification

EP 0 458 363 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.9.9/3.4

## Description

In EP-A-0 335 355 bis(hydroxymethyl)cyclobutyl purines and pyrimidines are disclosed which possess antiviral activity. The cyclobutyl ring of these compounds is not fluorinated.

CA 75 (1971) Abstract No. 2055h discloses the compound $CHF:C(OEt)OB_z$.

Antiviral activity is exhibited by compounds having the formula 1

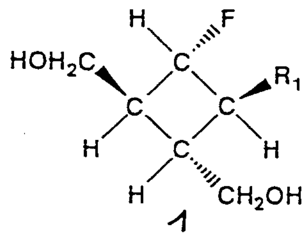

1

and its pharmaceutically acceptable salts wherein $R_1$ is

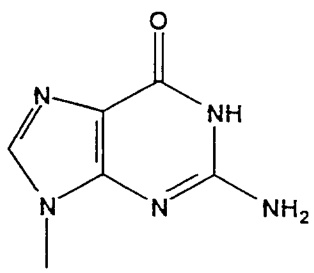

,

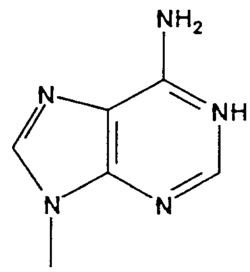

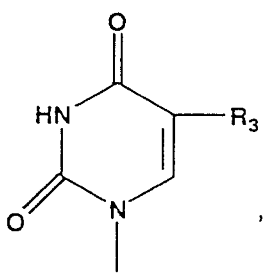

, or

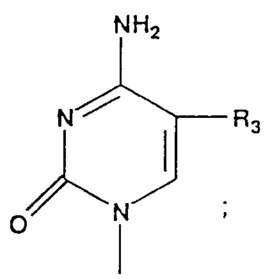

;

$R_3$ is hydrogen, methyl, fluoro, chloro, bromo, iodo, or

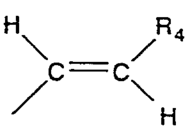

(trans)

and $R_4$ is chloro, bromo, or iodo.

Preferred compounds of formula 1 are when $R_1$ is

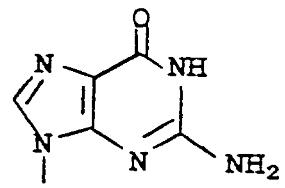

or

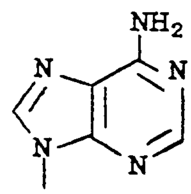

.

The most preferred compound is (1α,2β,3α,4β)-2-amino-9-[2-fluoro-3,4-bis(hydroxymethyl)cyclobutyl]-1,9-dihydro-6H-purin-6-one.

This compound exists as the following two enantiomers: [1S-(1α,2β,3α,4β)]-2-amino-9-[2-fluoro-3,4-bis-(hydroxymethyl)cyclobutyl]-1,9-dihydro-6H-purin-6-one, which is preferred, and [1R-(1α,2β,3α,4β)]-2-amino-9-[2-fluoro-3,4-bis(hydroxymethyl)cyclobutyl]-1,9-dihydro-6H-purin-6-one.

The compounds of formula 1, and the pharmaceutically acceptable salts thereof, are antiviral agents that can be used to treat viral infection in mammalian species such as domesticated animals (e.g., dogs, cats, horses and the like) and humans, and avian species (e.g., chickens and turkeys). The compounds of formula 1 wherein $R_1$ is

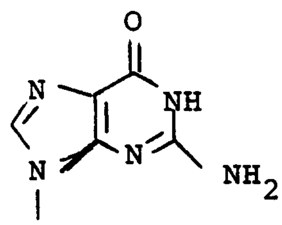

,

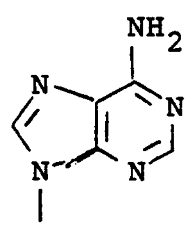

,

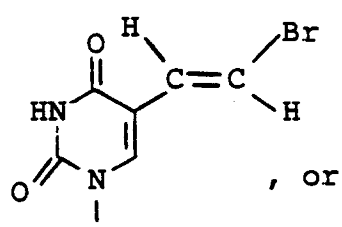

, or

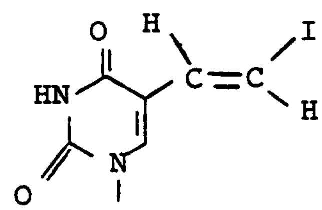

are effective against one or more of the following viruses: herpes simplex virus 1 and 2, varicellazoster virus, cytomegalovirus and murine leukemia virus. They are also believed to be active against a variety of other DNA and retroviruses. Exemplary DNA viruses in addition to those named above include other herpes viruses (e.g., Epstein-Barr virus, pseudorabies virus, human herpes virus 6, and the like), poxviruses (e.g., vaccinia virus, monkey pox and myoma), papovaviruses (e.g., the papilloma viruses), hepatitis B virus, and adenoviruses. Exemplary retroviruses in addition to that named above include human T-cell lymphotrophic viruses -I and -II, feline leukemia virus, and equine infectious anemia virus.

The compounds of formula 1, wherein $R_1$ is

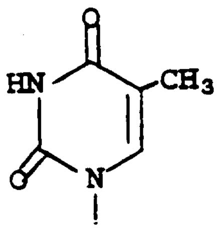 , 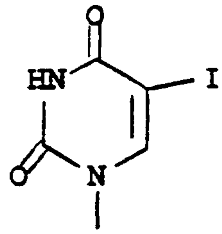 , or 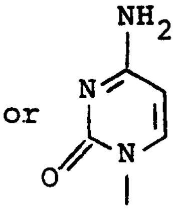 ,

and the enantiomer [1R-(1α,2β,3α,4β)]-2-amino-9-[2-fluoro-3,4-bis(hydroxymethyl)cyclobutyl]-1,9-dihydro-6H-purin-6-one are belived to be active against the various retroviruses and DNA viruses named above with the exception of herpes simplex 1 and 2, varicella-zoster virus, and cytomeglovirus. All of the other compounds of formula 1 are believed to be active against one or more of the following viruses: herpes simplex virus 1 and 2, varicella-zoster virus, cytomegalovirus, human immuno deficiency virus (HIV), and the other retroviruses and DNA viruses described above.

The compounds of this invention may be administered parenterally (for example, by intravenous, intraperitoneal or intramuscular injection), orally or topically.

The compounds may be administered orally or parenterally in an amount effective to treat the infection. The dosage will, of course, depend on the severity of the infection, but will likely be in the range of about 1.0 to 50 mg/kg of body weight. The desired dose may be administered several times daily at appropriate intervals.

For infections of the eye, or other external tissues (e.g., mouth and skin) the compositions may be applied to the infected part of the body of the patient topically as an ointment, cream, aerosol, gel, powder, lotion, suspension or solution (e.g., as in eye drops). The concentration of the compound in the vehicle will, of course, depend on the severity of the infection, but will likely be in the range of about 0.1 to 7% by weight.

A compound of formula 1 wherein $R_1$ is

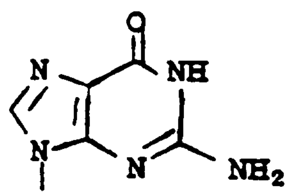

can be prepared from an intermediate of formula

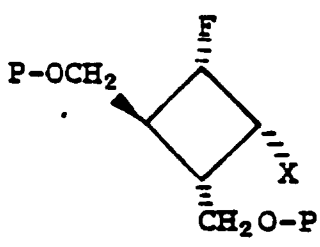

2

wherein P is a protecting group such as benzyl or silyl, and X is a leaving group such as a straight or branched chain lower alkyl of 1 to 5 carbons, phenyl, substituted lower alkyl or substituted phenyl sulfonate well known in the art (e.g., benzenesulfonyloxy, p-toluenesulfonyloxy, methanesulfonyloxy, p-nitrophenylsulfonyloxy, or trifluoromethylsulfonyloxy). The term "silyl" refers to silyl protecting groups well known in the art [e.g., t-butyldimethylsilyl, t-butyldiphenylsilyl, (triphenylmethyl)dimethylsilyl, methyldiisopropylsilyl, or triisopropylsilyl].

Reaction of a compound of formula 2 with a protected form of guanine such as a compound of formula

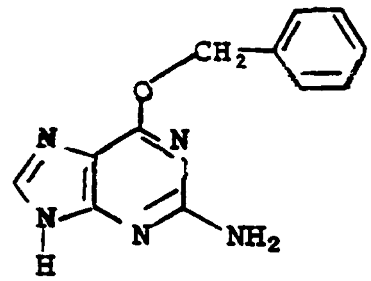

3

in the presence of a base such as potassium carbonate, sodium hydride, or potassium hydride in an aprotic polar solvent such as dimethylformamide, dimethylsulfoxide, or sulfolane (tetramethylene sulfone) yields the corresponding compound of formula

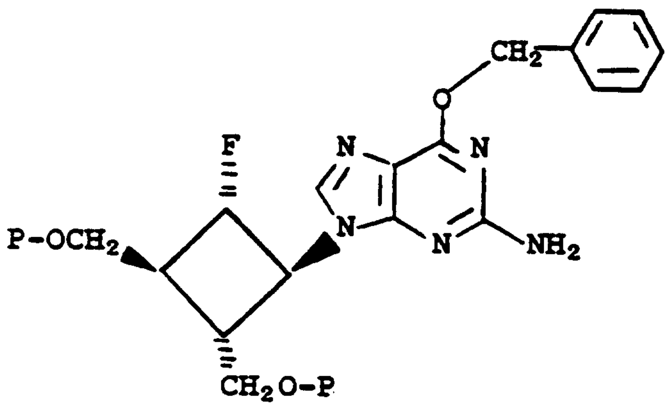

4

Optionally, the reaction can be run in the presence of a metal chelating catalyst such as 18-crown-6 (1,4,7,10,13,16-hexaoxacyclooctadecane) or 15-crown-5 (1,4,7,10,13-pentaoxacyclopentadecane).

Alternatively, treatment of a compound of formula 2 with a tetraalkylammonium salt of a compound of formula 3 (in an aprotic polar solvent such as dimethylformamide, dimethylsulfoxide or sulfolane) yields a compound of formula 4. A tetraalkylammonium salt of a compound of formula 3 can be prepared by treatment of a compound of formula 3 (in an aprotic polar solvent such as dimethylformamide) with a tetraalkylammonium hydroxide such as aqueous tetra-n-butylammonium hydroxide and subsequent azeotropic removal of water.

Alternatively, a tetraalkylammonium salt of a compound of formula 3 can be prepared by treatment of a compound of formula 3 (in a chlorinated hydrocarbon solvent such as methylene chloride) with a tetraalkylammonium hydroxide (such as aqueous tetra-n-butylammonium hydroxide). Removal of the water layer, drying of the organic layer (with a drying agent such as sodium or magnesium sulfate), filtration, and subsequent removal of the volatiles under vacuum provides a tetraalkylammonium salt of a compound of formula 3.

Removal of the protecting groups from a compound of formula 4 yields a compound of formula 1 wherein $R_1$ is

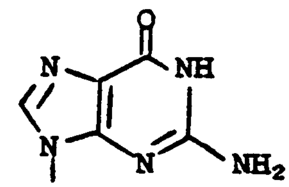

When the group P in compound 4 is a silyl protecting group, removal of the P group can be accomplished using fluoride ion (e.g., tetrabutylammonium fluoride in tetrahydrofuran). The purine O-benzyl protecting group can then be removed with aqueous alcoholic mineral acid or by hydrogenolysis (e.g., palladium hydroxide on carbon in cyclohexene and ethanol). When the protecting group P in 4 is benzyl, removal of all the benzyl protecting groups can be effected using sodium in liquid ammonia, hydrogenolysis (e.g., palladium hydroxide on carbon in cyclohexene and ethanol), or boron trichloride in dichloromethane.

Reaction of a compound of formula 2 with compound

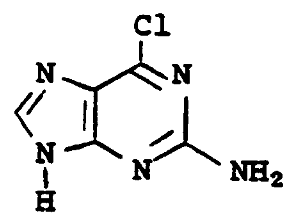

5

under conditions analogous to those used in the preparation of compound 4 provides a compound of formula

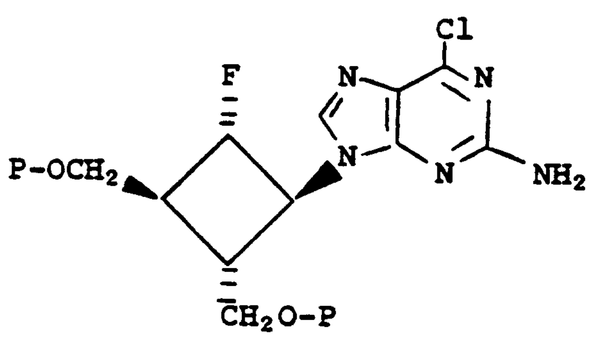

6

Removal of the protecting group P provides a compound of formula 1 wherein $R_1$ is

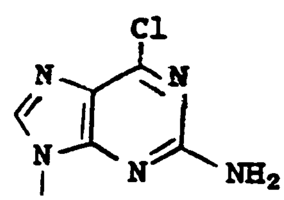

For example, when the protecting group P in 6 is silyl, the protecting group can be removed by treatment with fluoride ion (e.g., tetrabutylammonium fluoride). When the protecting group P in 6 is benzyl, removal of the P group can be performed by treatment with boron trichloride.

Acid hydrolysis (e.g., using hot aqueous hydrochloric acid) of the chloro group of a compound of formula 1 wherein $R_1$ is

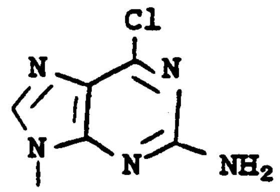

provides a compound of formula 1 wherein $R_1$ is

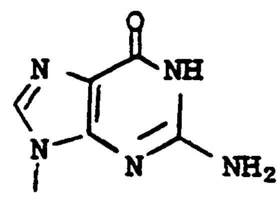

A compound of formula 2 can be prepared as follows: The hydroxyl groups of the known compound of formula 7 [see W.A. Slusarchyk, et al., Tetrahedron Lett., 6453 (1989) and European Patent 335,355-A] can be protected with a protecting group "P" such as benzyl or silyl by methods known in the art, yielding a compound of formula 8. Hydrolysis of the ketal in compound 8 using, for example, p-toluenesulfonic acid in acetone or aqueous sulfuric acid in acetonitrile, provides a compound of formula 9. Treatment of a compound of formula 9 (wherein P, for example, is a benzyl protecting group) with a sterically hindered amide base such as lithium 2,2,6,6-tetramethylpiperidide, lithium diisopropylamide, or lithium bis(trimethylsilyl)amide (preferentially, lithium 2,2,6,6-tetramethylpiperidide), followed by the addition of a fluorinating agent such as perchloryl fluoride [see for example, C.L.J. Wang, P.A. Grieco, F.J. Okuniewicz, Chem.Commun., 48 (1976)] provides compounds of formula 10 as a mixture of (2α,3β,4α)-2-fluoro-3,4-bis[(phenylmethoxy)methyl]cyclobutanone and (2α,3α,4β)-2-fluoro-3,4-bis[(phenylmethoxy)methyl]cyclobutanone. Optionally, perchloryl fluoride can be substituted by other fluorinating agents such as acetyl hypofluorite or N-fluoroalkylsulfonamides [see for example, S. Rozen and M. Brand, Synthesis, 665 (1985); W.E. Barnette, J. Amer. Chem. Soc., 106, 452 (1984)].

Alternatively, a compound of formula 9 can be treated with a sterically hindered amide base, followed by the addition of an enolate trapping agent such as chlorotrimethylsilane to provide a compound of formula 11. Treatment of a compound of formula 11 with a fluorinating agent such as acetyl hypofluorite, trifluoromethyl hypofluorite, 2-fluoropyridinium trifluoromethanesulfonate, or a N-fluoroalkylsulfonamide, provides compounds of formula 10 [see for example, S. Rozen, et al., J. Org. Chem., 50, 4753 (1985); W. J. Middleton and E.M. Bingham, J. Amer. Chem. Soc., 102, 4845 (1980); T. Umenoto, K. Kawada and K. Tomita, Tetrahedron Lett., 4465 (1986); W. E. Barnette, J. Amer. Chem. Soc., 106, 452 (1984)].

Reduction of compounds of formula 10 with a reducing agent such as lithium trisiamylborohydride provides a compound of formula 12 and an equal amount of (1α,2α,3α,4β)-2-fluoro-3,4-bis[(phenylmethoxy)methyl]cyclobutanol which can be separated by chromatography. Alternatively, reducing agents such as lithium aluminum hydride, sodium borohydride, lithium borohydride, lithium tri-sec-butylborohydride or diisobutylaluminum hydride can be used. The compound of formula 12 can be converted to a compound of formula 2 by methods known in the art. For example, treatment of 12 with p-toluenesulfonyl chloride in pyridine, or methanesulfonyl chloride in pyridine, or methylene chloride/triethylamine, yields a compound of formula 2 wherein X is p-toluenesulfonyloxy or methanesulfonyloxy, respectively.

The above description is shown in the following schematic:

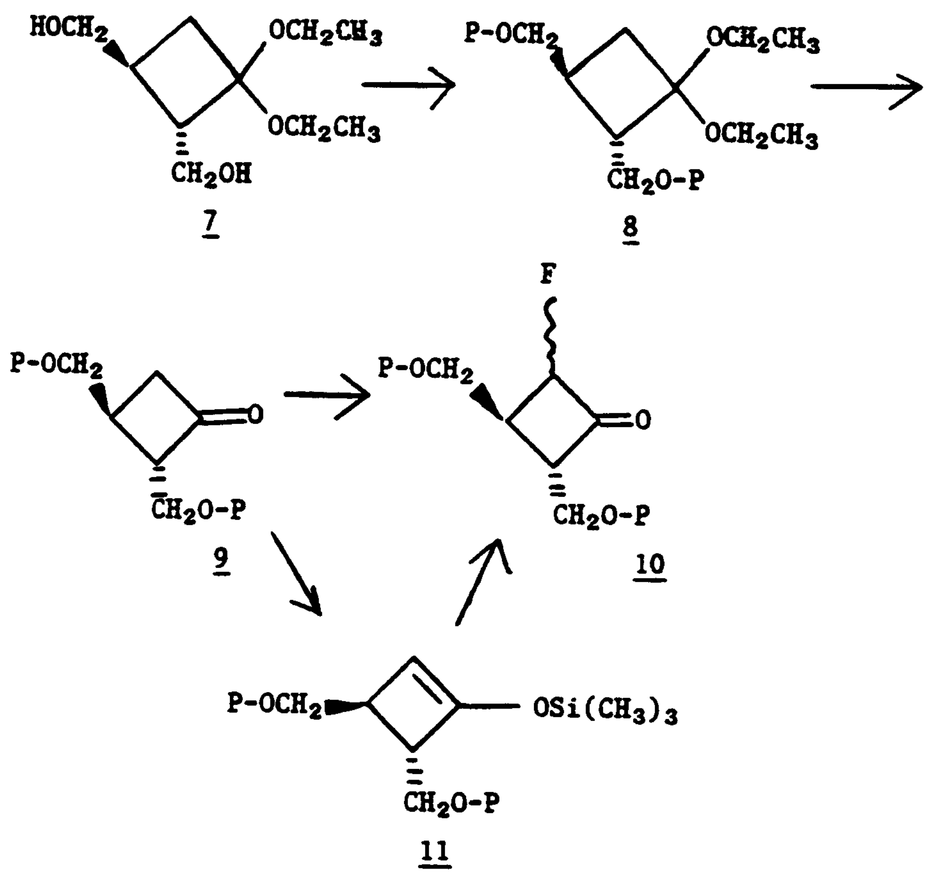

7 8 9 10 11

10

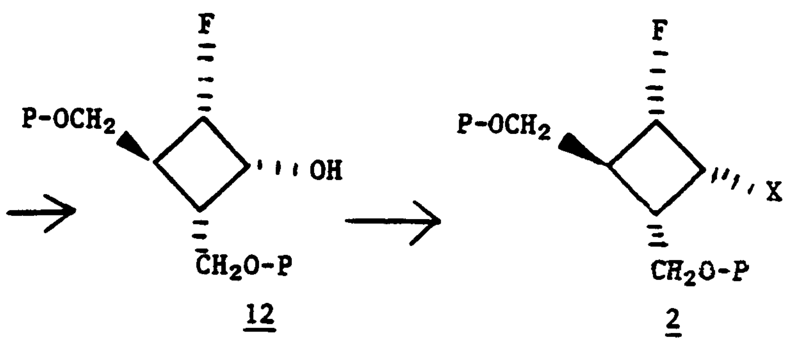

12 2

Reaction of the compound of formula 2 with a compound of formula

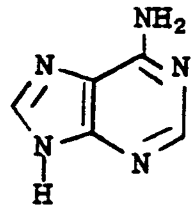

19

by methodology analogous to that used to prepare a compound of formula 4, and subsequent removal of the protecting groups P, yields the corresponding compound of formula 1 wherein $R_1$ is

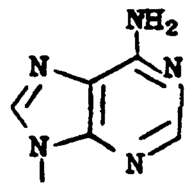

Alternatively, this compound of formula 1 can be prepared by reaction of a compound of formula

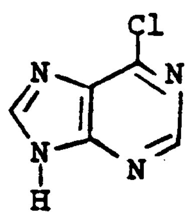

20

with a compound of formula 2 by methods analogous to those used in the preparation of a compound of formula 4. This affords the corresponding compound of formula

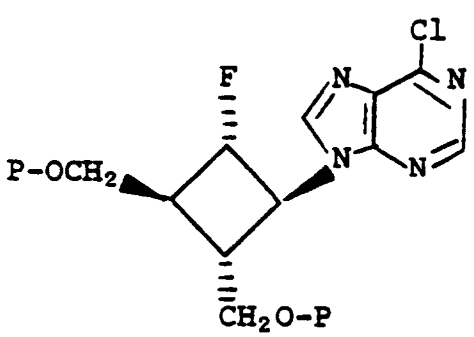

21

Treatment of a compound of formula 21 with hot ammonia in an alcohol (such as, methanol or ethanol) and subsequent deprotection of the protecting groups P yields the corresponding compound of formula 1 wherein $R_1$ is

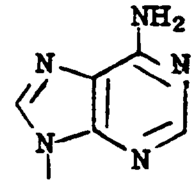

The compound of formula

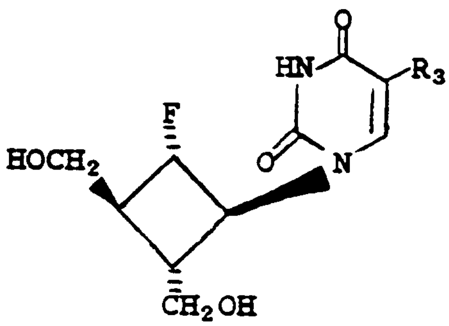

23

wherein $R_3$ is hydrogen, fluoro, methyl, ethyl, n-propyl, 2-chloroethyl, or 2-fluoroethyl can be prepared by reaction of the corresponding compound of formula

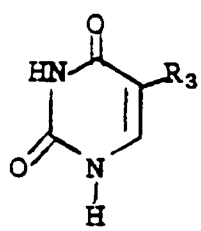

24

with a compound of formula 2 in the presence of a base such as potassium carbonate, sodium hydride, or potassium hydride, in an aprotic polar solvent (e.g., dimethylformamide, dimethylsulfoxide, or sulfolane), in the optional presence

of 18-crown-6 or 15-crown-5, to yield an intermediate of formula

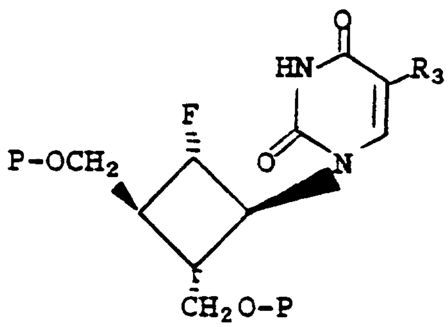

25

Alternatively, treatment of a compound of formula 2 with a mono-tetraalkylammonium salt of a compound of formula 24 in an aprotic polar solvent such as dimethylformamide, dimethylsulfoxide or sulfolane affords a compound of formula 25. A mono-tetraalkylammonium salt of a compound of formula 24 can be prepared by treatment of a compound of formula 24 (in an aprotic polar solvent such as dimethylformamide) with a tetraalkylammonium hydroxide, such as aqueous tetra-n-butylammonium hydroxide, and subsequent azeotropic removal of water.

Removal of the protecting groups P from a compound of formula 25 provides the corresponding compound of formula 23. For example, when P is a silyl group, deprotection can be accomplished with fluoride ion. When P is a benzyl group, deprotection can be accomplished by hydrogenolysis (e.g., palladium hydroxide on carbon in cyclohexene and ethanol) or by treatment with boron trichloride.

The compound of formula 24 wherein $R_3$ is 2-chloroethyl or 2-fluoroethyl can be prepared by methods known in the art [H. Griengl, et al., J. Med. Chem., 30, 1199 (1987); J. Med. Chem., 28, 1679 (1985)].

The compound of formula 23 wherein $R_3$ is fluoro can also be prepared from the corresponding compound 23 wherein $R_3$ is hydrogen and the hydroxy groups are optionally protected with a group such as acetyl by fluorination with trifluoromethyl hypofluorite using methodology known in the art. For example, see M.J. Robins, et al., J. Amer Chem. Soc., 93, 5277 (1971) and Chem. Commun., 18 (1972); T.S. Lin, et al., J. Med. Chem., 26, 1691 (1983).

The compounds of formula 23 wherein $R_3$ is 2-chloroethyl and 2-fluoroethyl can also be prepared from a compound of formula

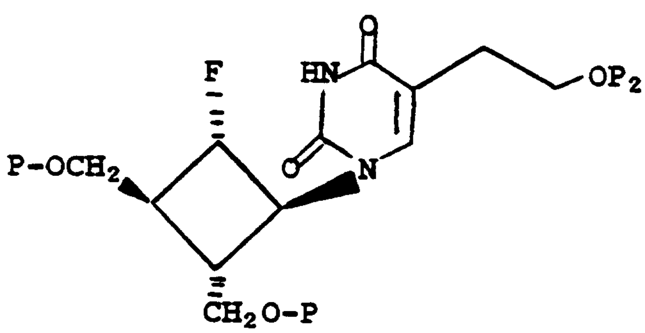

26

wherein $P_2$ and P are different protecting groups wherein $P_2$ can be selectively removed in the presence of P. For example, when $P_2$ is a silyl, trityl or substituted trityl group, P can be a benzyl group. Similarly, when $P_2$ is an acyl or benzyl group, P can be a silyl protecting group. Selective removal of the protecting group $P_2$ yields a compound of formula 25 wherein $R_3$ is 2-hydroxyethyl. Treatment of this compound with triphenylphosphine-carbon tetrachloride and subsequent removal of protecting groups P affords the compound of formula 23 wherein $R_3$ is 2-chloroethyl. Similar treatment using triphenylphosphine-N-bromosuccinimide or triphenylphosphine N-bromosuccinimide-tetrabutylammonium iodide in place of triphenylphosphine-carbon tetrachloride (e.g., see H. Griengl, et al., J. Med. Chem., 28, 1679 (1985)) affords compounds of formula 25 wherein $R_3$ is 2-bromoethyl or 2-iodoethyl, respectively. Subsequent treatment with fluoride ion, followed by removal of protecting groups P, provides the compound of formula 23 wherein $R_3$ is 2-fluoroethyl. When P is a silyl

group, deprotection will occur upon treatment with fluoride ion. Alternatively, treatment of a compound of formula 25, wherein $R_3$ is 2-hydroxyethyl, with diethylaminosulfur trifluoride provides, upon removal of the protecting groups p, a compound of formula 23 wherein $R_3$ is 2-fluoroethyl.

The compound of formula 26 can be prepared by reaction of a compound of formula

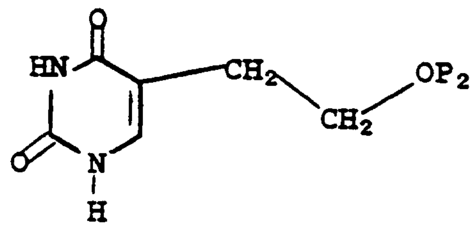

27

with a compound of formula 2 by methods analogous to those used for the preparation of 25 wherein, for example, $R_3$ is hydrogen, methyl or ethyl. The compound of formula 27 can be prepared from the corresponding free alcohol by methods known in the art.

The compound of formula

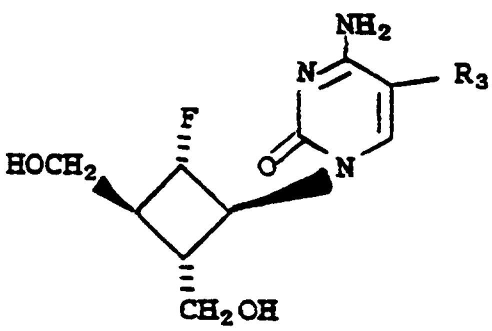

28

wherein $R_3$ is hydrogen, fluoro, methyl, ethyl, n-propyl, 2-chloroethyl, or 2-fluoroethyl can be prepared from the corresponding compound of formula 25 by methods known in the art. See, for example, I. Wempner, et al., in "Synthetic Procedures in Nucleic Acid Chemistry", Vol. 1, W.W. Zorbach and R.S. Tipson, Eds., Interscience Publishers, N.Y., p. 299, 1968; T.S. Lin, et al., J. Med. Chem., 26, 1691 (1983); P. Herdewijn, et al., J. Med. Chem., 28, 550 (1985). Deprotection yields the corresponding compound of formula 28.

Alternatively, the compound of formula 28, wherein $R_3$ is fluoro, hydrogen, methyl, ethyl, n-propyl, 2-chloroethyl, or 2-fluoroethyl, can be prepared by reaction of the corresponding compound of formula

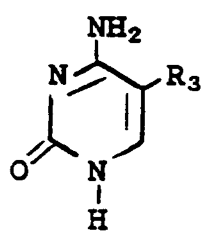

29

with a compound of formula 2 in the presence of a base such as potassium carbonate, sodium hydride, or potassium hydride in an aprotic solvent (e.g., dimethylformamide, dimethylsulfoxide, or sulfolane), in the optional presence of 18-crown-6 or 15-crown-5, and subsequent removal of the protecting groups. Optionally, the amino (-$NH_2$) group in 29 can be protected, e.g., with an acyl group. Removal of this protecting group can be accomplished using sodium methoxide in methanol or methanolic ammonia.

Alternatively, treatment of a compound of formula 2 with a tetraalkylammonium salt of a compound of formula 29 in an aprotic polar solvent such as dimethylformamide, dimethylsulfoxide or sulfolane provides a compound of formula 28. A tetraalkylammonium salt of a compound of formula 29 can be prepared by treatment of a compound of formula 29 (in an aprotic polar solvent such as dimethylformamide) with a tetraalkylammonium hydroxide such as aqueous tetra-n-butylammonium hydroxide and subsequent azeotropic removal of water.

Alternatively, the compound of formula 28 wherein $R_3$ is fluoro can be prepared from the corresponding compound wherein $R_3$ is hydrogen by fluorination with trifluoromethyl hypofluorite using methodology known in the art. Fluorination can also be performed on the compounds of formula 28 wherein $R_3$ is hydrogen and the hydroxyl and/or amino groups are protected, for example, by an acyl group. After fluorination, deprotection using methanolic ammonia or aqueous hydroxide affords the compound of formula 28 wherein $R_3$ is fluoro. See, for example, M.J. Robins, et al., J. Amer. Chem. Soc., 93, 5277 (1971) and Chem. Commun., 18 (1972); T.S. Lin, et al., J. Med. Chem., 26, 1691 (1983).

The compounds of formula 23 and 28 wherein $R_3$ is chloro, bromo, or iodo can be prepared from the corresponding compounds of formula 23 and 28 wherein $R_3$ is hydrogen by methods known in the art. See, for example, "Basic Principals in Nucleic Acid Chemistry", Vol. 1, P.O.P. Ts'O, Ed., Academic Press, N.Y., p. 146, 1974; P.K. Chang in "Nucleic Acid Chemistry" Part 3, L.B. Townsend and R.S. Tipson, Eds., John Wiley and Sons, N.Y., p.46, 1986.

The compounds of formula 23 and 28 wherein $R_3$ is trifluoromethyl can be prepared from the corresponding compounds of formula 23 and 28 wherein $R_3$ is iodo and the hydroxy and amino (-$NH_2$) groups are protected, for example, by an acyl, by treatment with trifluoromethyl iodide and copper according to procedures known in the art. Subsequent deprotection using methanolic ammonia or sodium methoxide in methanol yields the corresponding compound of formulas 23 and 28 wherein $R_3$ is trifluoromethyl. See, for example, Y. Kobayashi, et al., J. Chem. Soc. Perkin 1, 2755 (1980); S. Lin, et al., J. Med. Chem., 26, 1691 (1983).

The compounds of formula 23 and 28 wherein $R_3$ is

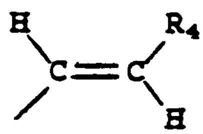

and $R_4$ is chloro, bromo, iodo, hydrogen, methyl or trifluoromethyl can be prepared from the corresponding compounds of formula 23 and 28 wherein $R_3$ is iodo or -HgCl via organopalladium intermediates. The compounds of formula 23 and 28 wherein $R_3$ is -HgCl can be prepared from the corresponding compounds of formula 23 and 28 wherein $R_3$ is hydrogen by methods known in the art. See, for example, M. Ashwell, et al., Tetrahedron, 43, 4601 (1987); M.E. Perlman, et al., J. Med. Chem., 28, 741 (1985); P. Herdewijn, et al., J. Med. Chem., 28, 550 (1985); D.E. Bergstrom, et al., J. Med. Chem., 27, 279 (1984); and references in E. DeClercq, et al., Pharmac. Ther., 26, 1 (1984).

Unless otherwise stated, the stereochemistry shown for the compounds of this invention and intermediates leading to compounds of this invention is relative, not absolute. It is drawn to show that in the compounds of this invention, the base represented by $R_1$ is trans to both the vicinal -$CH_2OH$ substituent and the fluorine atom, and the -$CH_2OH$ substituents are trans to each other.

The compounds of formula 1 can exist as levorotatory or dextrarotatory enantiomers or as mixtures thereof. All of these forms are within the scope of this invention.

For example, the 1S-enantiomer of the compound of formula 1 wherein $R_1$ is

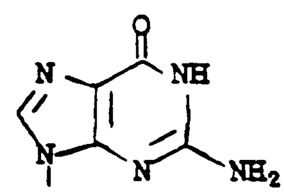

and $R_7$ and $R_8$ are hydrogen, [1S-(1α,2β,3α,4β)]-2-amino-9-[2-fluoro-3,4-bis(hydroxymethyl)-cyclobutyl]-1,9-dihydro-6H-purin-6-one, can be prepared from the 1R-enantiomer of the compound of formula 2 wherein X is p-toluenesulfonoxy and P is benzyl, [1R-(1α,2α,3β,4α)]-2-fluoro-3,4-bis-[(phenylmethoxy)methyl]cyclobutanol,4-methylbenzenesulfonate

ester, using methods analogous to those used in the preparation of the corresponding racemic compound of formula 1. Reaction of [1R-(1α,2α,3β,4α)]-2-fluoro-3,4-bis[(phenylmethoxy)-methyl]cyclobutanol, 4-methylbenzenesulfonate ester, with a compound of formula 3 in the presence of a base, such as potassium carbonate, in an aprotic polar solvent, such as dimethylformamide, and subsequent removal of the benzyl protecting groups yields [1S-(1α,2β,3α,4β)]-2-amino-9-[2-fluoro-3,4-bis(hydroxymethyl)cyclobutyl]-1,9-dihydro-6H-purin-6-one.

[1R-(1α,2β,3α,4β)]-2-Amino-9-[2-fluoro-3,4-bis(hydroxymethyl)cyclobutyl]-1,9-dihydro-6H-purin-6-one can be prepared in an analogous fashion from [1S-(1α,2α,3β,4α)]-2-fluoro-3,4-bis((phenylmethoxy)methyl]cyclobutanol, 4-methylbenzenesulfonate ester.

[1R-(1α,2α,3β,4α)]-2-Fluoro-3,4-bis-[(phenylmethoxy)methyl]cyclobutanol,4-methylbenzeflesulfonate ester, can be prepared from the corresponding alcohol, [1R-(1α,2α,3β,4α)]-2-fluoro-3,4-bis-[(phenylmethoxy)methyl]cyclobutanol, by methods known in the art. [1R-(1α,2α,3β,4α)]-2-fluoro-3,4-bis[(phenylmethoxy)methyl]cyclobutanol can be prepared from (2S-*trans*)-2,3-bis[(phenylmethoxy)-methyl]cyclobutanone by treatment with a sterically hindered base (such as lithium 2,2,6,6-tetramethylpiperidide), followed by addition of a fluorinating agent (such as perchlorylfluoride), and subsequent reduction of the resulting α-fluoro ketone with, for example, lithium trisiamylborohydride. Alternatively, (2S-*trans*)-2,3-bis[(phenylmethoxy)methyl]cyclobutanone can be treated with a sterically hindered base (such as 2,2,6,6-tetramethylpiperidide), followed by the addition of chlorotrimethylsilane, treatment of the resulting enol ether with a fluorinating agent (such as 2-fluoropyridinium trifluoromethanesulfonate), and subsequent reduction of the resulting α-fluoro ketone with, for example, lithium trisiamylborohydride.

[1S-(1α,2α,3β,4α)]-2-Fluoro-3,4-bis[phenylmethoxy)methyl]cyclobutanol,4-methylbenzenesulfonate ester can be prepared in an analogous fashion from (2R-*trans*)-2,3-bis[(phenylmethoxy)methyl]cyclobutanone.

(2S-*trans*)-2,3-Bis[(phenylmethoxy)]methyl]cyclobutanone can be prepared as described below:

Complexation of (-)-dimenthylfumarate with diisobutylaluminum chloride (or diethylaluminum chloride; -78°C in toluene or toluene/hexane), followed by addition of 1,1-dimethoxyethylene, provides, after recrystallization of the crude product, (1S-*trans*)-3,3-dimethoxy-1,2-cyclobutane-dicarboxylic acid, di-(-)-menthyl ester. Reduction of (1S-*trans*)-3,3-dimethoxy-1,2-cyclobutanedicarboxylic acid, di-(-)-menthyl ester with lithium aluminum hydride provides (1S-*trans*)-3,3-dimethoxy-1,2-cyclobutanedimethanol. (1S-*trans*)-3,3-dimethoxy-1,2-cyclobutanedimethanol is then converted to (2S-*trans*)-1,1-dimethoxy-2,3-bis[(phenylmethoxy)-methyl]cyclobutane by methods known in the art. Hydrolysis of (2S-*trans*)-1,1-dimethoxy-2,3-bis-[(phenylmethoxy)methyl]cyclobutane with, for example, aqueous sulfuric acid in acetonitrile provides (2S-*trans*)-2,3-bis[(phenylmethoxy)methyl]cyclobutanone.

(2R-*trans*)-2,3-bis[(phenylmethoxy)methyl]-cyclobutanone can be prepared in an analogous fashion from (+)-dimenthylfumarate.

The intermediate [1R-(1α,2α,3β,4α)]-2-fluoro-3,4-bis[(phenylmethoxy)methyl]cyclobutanol can alternatively be prepared by the procedure shown in the reaction scheme below:

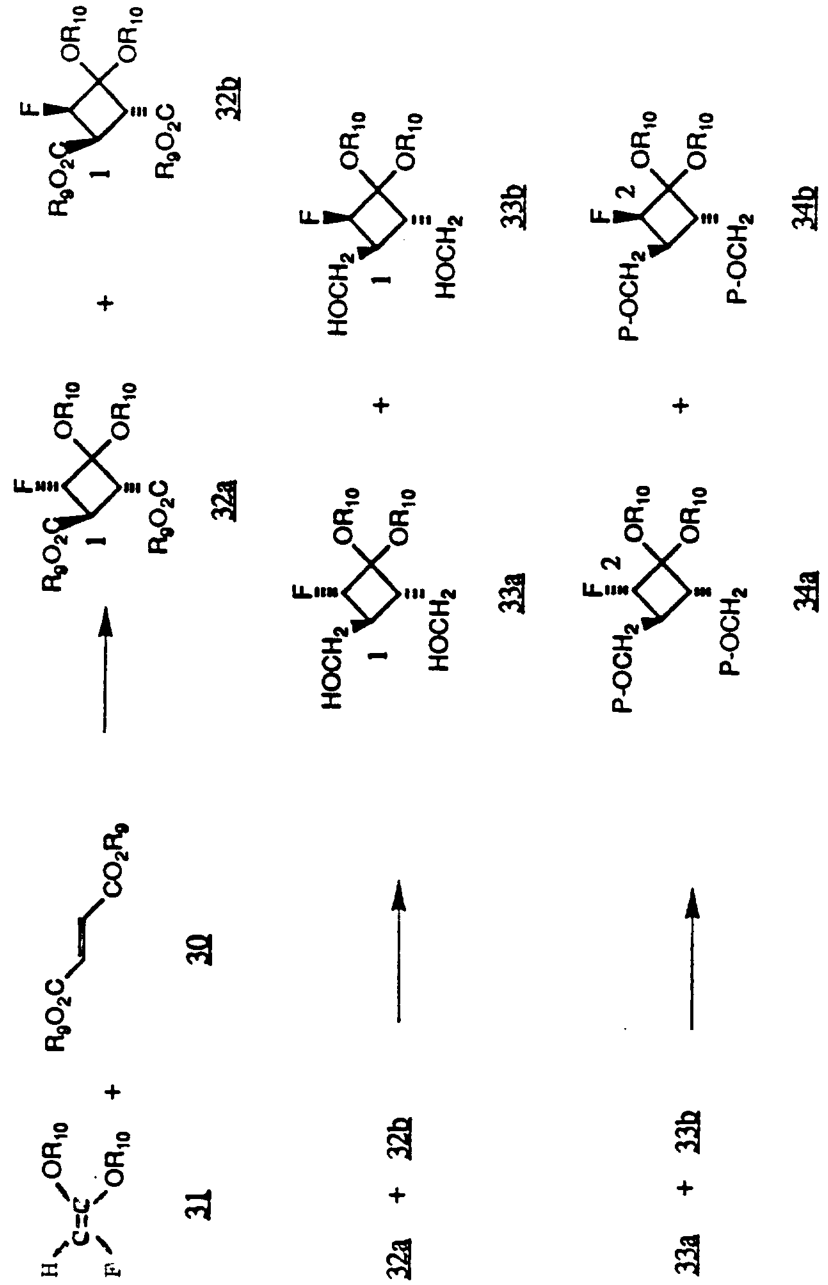

31 + 30 → 32a + 32b

32a + 32b → 33a + 33b

33a + 33b → 34a + 34b

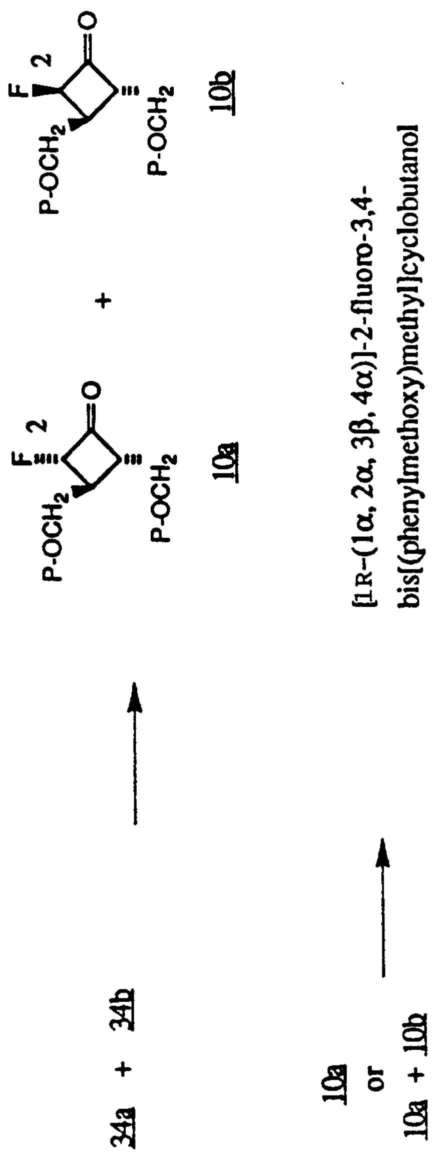

34a + 34b

10a + 10b

10a or 10a + 10b

[1R-(1α, 2α, 3β, 4α)]-2-fluoro-3,4-bis[(phenylmethoxy)methyl]cyclobutanol

$R_{10}$ is lower alkyl of 1 to 5 carbons or both $R_{10}$ groups are joined together by an alkylene group of 2 or 3 carbons; and

P is a protecting group;

and $R_9$ is a group selected from:

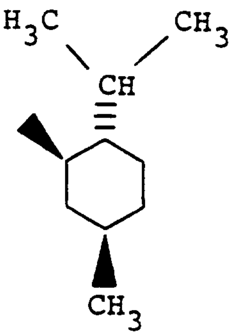
,
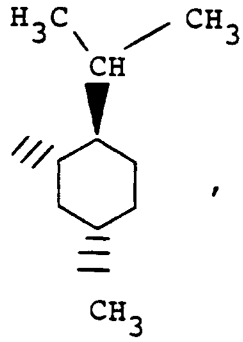
,

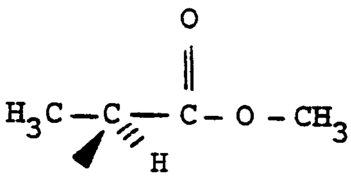
,
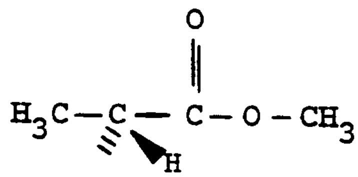
,
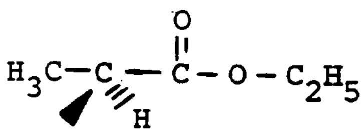
,
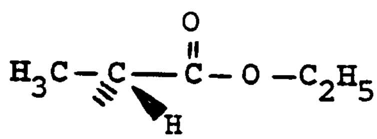
,
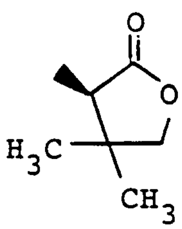
,
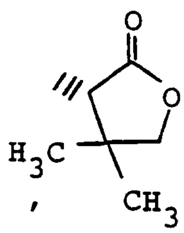
,
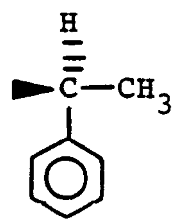
,
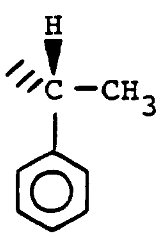
,
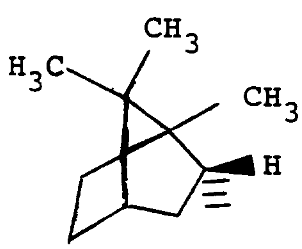
,
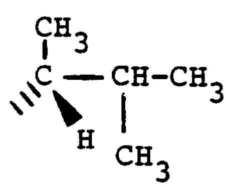
,

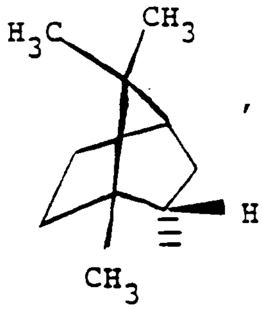 , 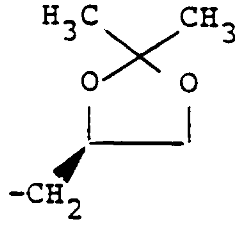 , 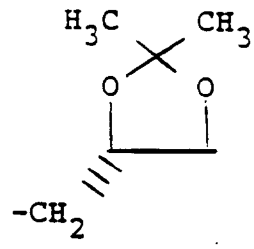 and

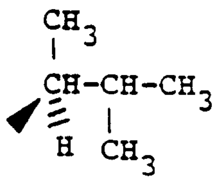 .

These $R_9$ groups are obtained from the optically active alcohols (+)- or (-)-menthol, (+)- or (-)-methyl- or ethyl lactate, (+)- or (-)-pantolactone, (+)- or (-)-α-phenethyl alcohol, (+)- or (-)-borneol, (+)- or (-)-α,β-isopropylideneglycerol, (+)- or (-)-3-methyl-2-butanol and the like (i.e., wherein $R_9OH$ is (+)- or (-)-menthol, (+)- or (-)-methyl or ethyl lactate, (+)- or (-)-pantolactone, (+)- or (-)-α-phenethyl alcohol, (+)- or (-)-borneol, (+)- or (-)-α,β-isopropylideneglycerol, (+)- or (-)-3-methyl-2-butanol) by removal of the hydroxy group.

Preferred $R_{10}$ groups are methyl and ethyl, especially ethyl.

Compounds 32a, 32b, 33a, 33b, 34a, 34b, 10a and 10b are optically active and their absolute stereochemistry is as follows: for 32a, 32b, 33a, and 33b, the absolute configuration at the cyclobutyl 1-position is S; for 34a and 10a, the absolute configuration at the cyclobutyl 2-position is S; for 34b and 10b, the absolute configuration at the cyclobutyl 2-position is R. The relative steroechemistry of these compounds is as depicted in the scheme.

The compounds of formula 32a and 32b can be prepared by reaction of a compound of formula 31 with a compound of formula 30 in the presence of a Lewis acid. Examples of suitable Lewis acids include aluminum compounds such as diethylaluminum chloride, diisobutylaluminum chloride, ethylaluminum dichloride, isopropoxyaluminum dichloride, aluminum trichloride and the like, boron compounds such as boron trifluoride, boron trichloride and the like, titanium compounds such as titanium tetrachloride, dichlorodiisopropoxytitanium and the like, and tin compounds such as tin tetrachloride, tin trichloride and the like. Preferably, the Lewis acid is a di(lower alkyl)aluminum chloride wherein each lower alkyl is of 2 to 4 carbons and most preferably is diethylaluminum chloride. Preferably, the $R_9$ group for the compound of formula 30 is derived from (-)-menthol, i.e., $R_9$ is

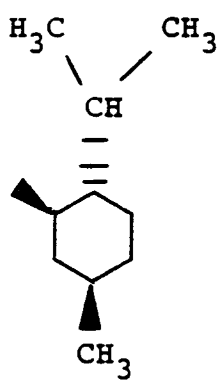 .

The ratio of the resulting epimeric fluoro compounds 32a and 32b may vary according to the conditions of the reaction and the particular $R_9$ and $R_{10}$ groups chosen in the reactants 30 and 31, respectively. For example, in the case wherein

$R_9$ of compound 30 is derived from (-)-menthol, $R_{10}$ of compound 31 is ethyl, and the Lewis acid is diethylaluminum chloride, the ratio of resulting 32a and 32b is approximately 1:5 after recrystallization of the crude product mixture.

The compounds of formulas 33a and 33b can be prepared by reaction of the compounds of formulae 32a and 32b with a reducing agent such as lithium aluminum hydride, lithium borohydride, and the like, in a solvent such as tetrahydrofuran, diethyl ether, and the like, preferably with lithium aluminum hydride in tetrahydrofuran.

The compounds of formulas 34a and 34b can be prepared by protection of the hydroxy groups of compounds of formulas 33a and 33b with benzyl or silyl protecting groups as previously defined. Preferably the protecting group P is a benzyl group.

The compounds of formula 10a and 10b can be prepared by hydrolysis of the compounds of formulas 34a and 34b with an acid catalyst such as sulfuric acid, hydrochloric acid, p-toluenesulfonic acid, and the like, preferably sulfuric acid, in a solvent or solvent mixture such as water, water-acetonitrile, water-dioxane, acetone, and the like, preferably water-acetonitrile.

If in the mixture of isomers 10a and 10b, the isomer 10b is the predominant isomer, then the mixture of 10a and 10b can be treated with a basic catalyst such as N,N-diisopropylethylamine, 1,8-diazabicyclo[5.4.0]undec-7-ene and the like, preferably 1,8-diazabicyclo[5.4.0]undec-7-ene, in a solvent such as tetrahydrofuran, acetonitrile, and the like, preferably acetonitrile, to effect epimerization of the fluorine so that the isomer 10a is the predominant isomer or exclusive isomer of the mixture.

The desired intermediate [1R-(1α,2α,3β,4α)]-2-fluoro-3,4-bis[(phenylmethoxy)methyl]cyclobutanol can then be prepared by reduction of 10a or the mixture of 10a and 10b with a reducing agent, preferably lithium trisiamylborohydride, followed by chromatography on silica gel.

The starting material of formula 31 in the above procedure can be prepared by the method shown in the reaction scheme below:

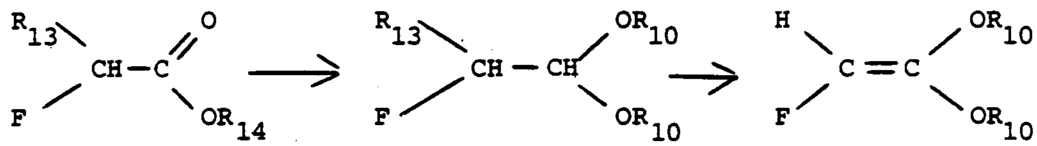

35 36 31

wherein $R_{14}$ is a lower alkyl of 1 to 5 carbons and $R_{13}$ is chloro, bromo, or iodo.

A compound of formula 36 can be prepared by reducing a compound of formula 35 with diisobutylaluminum hydride, lithium aluminum hydride, and the like, preferably with diisobutylaluminum hydride, followed by treatment of the crude product with an acid catalyst such as sulfuric acid, hydrochloric acid, and the like, preferably hydrochloric acid, in the presence of a lower alkyl alcohol or ethylene glycol or propylene glycol, preferably ethyl alcohol. In the compound of formula 35, $R_{13}$ is preferably chloro and $R_5$ is preferably ethyl. The compounds of formula 35 are either commercially available or can be prepared by methods known in the art. The compound of formula 31 can be prepared by treatment of the compound of formula 36 with a hindered base such as potassium t-butoxide, lithium diisopropylamide, and the like, preferably potassium t-butoxide.

The starting materials of formula 30 wherein $R_9$ is as defined above are commercially available (e.g., the compound of formula 30 wherein $R_9$ is the group obtained by removal of the hydroxy group from (-)-menthol is available from Aldrich Chemical Company) or can be readily prepared by methods known in the art (see e.g., Heathcock, C.H., et al., J. Med. Chem., 32, 197(1989); Scharf, H.D., Chem. Ber., 119, 3492, (1986); Helmchem, G., et al., DE 3702084).

Analogously, by selection of an appropriate $R_9$ the above procedure can be utilized to prepare the intermediate [1S-(1α,2α,3β,4α)]-2-fluoro-3,4-bis[(phenylmethoxy)methyl]cyclobutanol. In particular, when $R_9$ is the group obtained by removal of the hydroxy from (+)-menthol, i.e.,

$R_9$ is

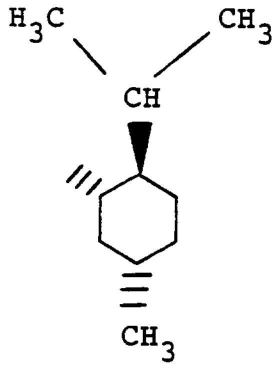

,

the [1S-(1α,2α,3β,4α)] enantiomer is obtained.

The separate [1S-(1α,2β,3α,4β)]- and [1R-(1α,2β,3α,4β)]-enantiomeric forms of the remaining compounds of formula 1 can be prepared from [1R-(1α,2α,3β,4α)]- and [1S-(1α,2α,3β,4α)]-2-fluoro-3,4-bis[(phenylmethoxy)methyl]cyclobutanol, respectively, using methods analogous to those used in the preparation of the corresponding racemic compounds of formula 1.

The compounds of formula 1 wherein $R_1$ is

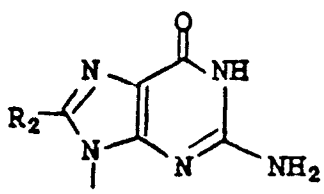

,

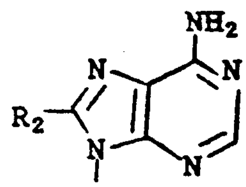

wherein $R_2$ = H and

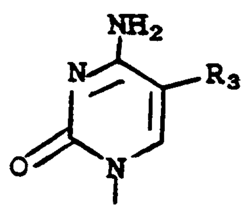

,

can form acid addition salts with inorganic or organic acids. Illustrative are the halide (e.g., chloride and bromide), alkylsulfonate, sulfate, phosphate and carboxylate salts.

The compounds of formula 1 wherein $R_1$ is

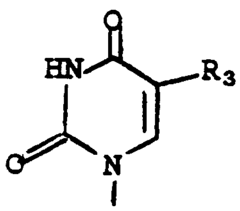

,

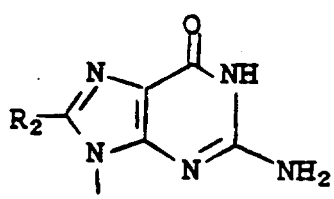

wherein $R_2$ is hydrogen can form basic salts with inorganic and organic bases. Illustrative are alkali metal salts (e.g., sodium and potassium), alkaline earth metal salts (e.g., calcium and magnesium), ammonium and substituted ammonium salts.

The following examples are specific embodiments of this invention. Unless otherwise noted, the compounds exemplified have been prepared as racemic mixtures.

Example 1

(1α,2β,3α,4β)-2-Amino-9-[2-fluoro-3,4-bis-(hydroxymethyl)cyclobutyl]-1,9-dihydro-6H-purin-6-one

A. *trans*-3,3-Diethoxy-1,2-cyclobutanedimethanol

To a suspension of 1.97 g (52.0 mmole) of lithium aluminum hydride in 150 ml of diethyl ether at 0°C was added over 15 minutes a solution of 10.0 g (34.7 mmole) of *trans*-3,3-diethoxy-1,2-cyclobutanedicarboxylic acid, diethyl ester [W.A. Slusarchyk, et al., Tetrahedron Lett., 6453 (1989) and European Patent 335,355-A] in 100 ml of anhydrous diethyl ether. The mixture was warmed to room temperature and stirred for 16 hours. The reaction was quenched by the addition of sodium sulfate decahydrate followed by the addition of 10 ml of 3M NaOH. The solids were removed by suction filtration and washed with ethyl acetate. Rotary evaporation of the solvent provided 7.21 g of the title compound as a viscous, colorless oil.

B. *trans*-1,1-Diethoxy-2,3-bis[(phenylmethoxy)methyl]cyclobutane

Sodium hydride (3.33 g, 83.3 mmole, 60% dispersion in mineral oil) was washed with pentane and then suspended in 50 ml of dry dimethylformamide. The suspension was cooled to 0°C and 10.0 ml of benzyl bromide was added to the reaction flask. To the resulting mixture was added a solution of 7.36 g (35 mmole) of *trans*-3,3-diethoxy-1,2-cyclobutanedimethanol in 20 ml of dry dimethylformamide. The mixture was warmed to room temperature and stirred for 8 hours. The mixture was diluted with diethyl ether (300 ml) and washed with saturated aqueous $NaHCO_3$ (2 x 150 ml). The aqueous layer was washed with diethyl ether (2 x 50 ml) and the combined organic layers were dried over $Na_2SO_4$. The solvent was removed by rotary evaporation and the residue was taken up in diethyl ether and dried over $Na_2SO_4$. Rotary evaporation of the solvent gave 14.1 g of residue which was purified by flash chromatography on silica gel (0% to 5% ethyl acetate - petroleum ether) affording 10.4 g of title compound that was homogenous by TLC.

C. *trans*-2,3-bis[(phenylmethoxy)methyl]cyclobutanone

To a solution of 10.4 g (27.0 mmol) of *trans*-1,1-diethoxy-2,3-bis[(phenylmethoxy)methyl]cyclobutane in 300 ml of acetonitrile was added 100 ml of 0.5M $H_2SO_4$. The solution was stirred overnight at room temperature. Next, the solution was diluted with ethyl acetate (1200 ml) and washed in succession with water (400 ml), saturated aqueous $NaHCO_3$ (400 ml), and brine (400 ml). The organic layer was dried over $Na_2SO_4$, and the solvent was removed by rotary evaporation. Toluene was added to the residue, followed by rotary evaporation in order to effect a toluene-water azeotrope. Flash chromatography on silica gel (10% to 20% ethyl acetate - petroleum ether) provided 7.85 g of pure cyclobutanone.

D. (2α,3β,4α)-2-Fluoro-3,4-bis[(phenylmethoxy)methyl]cyclobutanone

To a solution of 0.41 ml (2.4 mmol) of dry 2,2,6,6-tetramethylpiperidine in 4 ml of dry tetrahydrofuran at -78°C was added 0.84 ml of n-butyllithium (2.0 mmol; 2.4M in hexane). The resulting solution was stirred at -78°C for 20 minutes. To this was added, via cannula over 2 minutes, a cold (-78°C) solution of 0.500 g (1.51 mmol) of *trans*-2,3-[bis(phenylmethoxy)methyl]cyclobutanone in 2 ml of dry tetrahydrofuran. The resulting solution was stirred for 30 minutes at -78°C and then warmed to -35°C. Perchloryl fluoride* (~5 g) was bubbled through the reaction mixture at a rapid rate for 30 seconds. The mixture was purged with Argon for 5 minutes at -35°C and for 10 minutes at 0°C. The mixture was quenched by dropwise addition of 8 ml of 1M KI, and the mixture was stirred for 15 minutes at room temperature. The reaction contents were extracted with diethyl ether and the combined organic layers were washed with 5% aqueous sodium bisulfite. The organic layer was dried over $Na_2SO_4$, and the solvent was removed by rotary evaporation. The residue was dried by azeotropic rotary evaporation with toluene to provide 0.57 g of crude product containing an ~1:1 ratio of (2α,3β,4α)-2-fluoro-3,4-bis[(phenylmethoxy)methyl]cyclobutanone and its C2-epimer, (2α,3α,4β)-2-fluoro-3,4-bis[(phenylmethoxy)methyl]cyclobutanone.

E. (1α,2α,3β,4α)-2-Fluoro-3,4-bis[(phenylmethoxy)methyl]cyclobutanol

To a solution of 19.3 ml (19.3 mmol) of lithium trisiamylborohydride (1.0 M in tetrahydrofuran) in 12 ml of dry tetrahydrofuran at -78°C was added, via cannula over 3 minutes, a cold (-78°C) solution of 3.55 g (~9.7 mmol) of a crude

*Caution: On two occasions violent explosions occurred, as has been documented for reactions involving organic solutions of $FC10_3$ [see C.L.J. Wang, P.A. Grieco, F.J. Okuniewicz, Chem. Commun., 468 (1976); C.M. Sharts, W.A. Sheppard, Org. Reactions, 21, 225 (1974); W. Adcock, T.C. Khor, J. Organometal. Chem., 91, C20 (1975)]. Proper precautions should be taken to avoid injury.

~1:1 mixture of (2α,3β,4α)-2-fluoro-3,4-bis[(phenylmethoxy)methyl]cyclobutanone and (2α,3α,4β)-2-fluoro-3,4-bis[(phenylmethoxy)methyl]cyclobutanone in 12 ml of dry tetrahydrofuran. The mixture was stirred at -78°C for 30 minutes, and then warmed to 0°C and stirred for 30 minutes. The reaction contents were quenched by the slow addition of saturated aqueous $NaHCO_3$ (60 ml) and 30% $H_2O_2$(12 ml). The mixture was warmed to room temperature and stirred for 15 minutes. Next, the mixture was diluted with brine (75 ml) and extracted with ethyl acetate (300 ml, then 2 x 100 ml). The combined organic layers were washed with brine (100 ml) and dried over $Na_2SO_4$. Rotary evaporation of the solvent provided 3.37 g of crude material. Flash chromatography on silica gel (20% to 30% ethyl acetate-hexane) gave in order of elution 644 mg of (1α,2α,3β,4α)-2-fluoro-3,4-bis[(phenylmethoxy)methyl]cyclobutanol, 556 mg of an unidentified material, and 694 mg of (1α,2α,3α,4β)-2-fluoro-3,4-bis[(phenylmethoxy)methyl]cyclobutanol.

F. <u>(1α,2α,3β,4α)-2-Fluoro-3,4-bis[(phenylmethoxy)methyl]cyclobutanol, 4-methylbenzenesulfonate ester</u>

To a solution of 340 mg (1.03 mmol) of (1α,2α,3β,4α)-2-fluoro-3,4-bis[(phenylmethoxy)methyl]cyclobutanol in 1 ml of dry pyridine was added 972 mg (5.10 mmol) of p-toluenesulfonyl chloride. The mixture was heated at 60°C for 7 hours. The mixture was cooled to room temperature, diluted with diethyl ether (80 ml), and washed with water (4 x 25 ml), saturated aqueous $NaHCO_3$ (25 ml), and brine (25 ml). The organic layer was dried over $Na_2SO_4$, and rotary evaporation of the solvent gave 1.49 g of crude material. Flash chromatography on silica gel (10% to 20% ethyl acetate-hexane) gave 465 mg of the title compound as a white solid.

G. <u>(1α,2β,3α,4β)-9-[2-Fluoro-3,4-bis[(phenylmethoxy)methyl]cyclobutyl]-6-(phenylmethoxy)-9H-purin-2-amine</u>

To a solution of 450 mg (0.929 mmol) of (1α,2α,3β,4α)-2-fluoro-3,4-bis[(phenylmethoxy)methyl]cyclobutanol, 4-methylbenzenesulfonate ester in 5 ml of dry dimethylformamide were added 1.12 g (4.64 mmol) of 2-amino-6-(phenylmethoxy)-9H-purine, 963 mg (6.97 mmol) of $K_2CO_3$ (powdered and dried under high vacuum at 80°C for 18 hours) and 1.23 g (4.64 mmol) of 18-crown-6. The mixture was heated at 110°C for 18 hours. The mixture was cooled to room temperature and filtered. The solvent was removed by rotary evaporation. The residue was dissolved in ethyl acetate and adsorbed onto silica gel. The ethyl acetate was removed by rotary evaporation and flash chromatography on silica gel (20% to 50% ethyl acetate-hexane) afforded 202 mg of the title compound.

H. <u>(1α,2β,3α,4β)-2-Amino-9-[2-fluoro-3,4-bis(hydroxymethyl)cyclobutyl]-1,9-dihydro-6H-purin-6-one</u>

To a solution of 158 mg (0.285 mmol) of (1α,2β,3α,4β)-9-[2-fluoro-3,4-bis[(phenylmethoxy)methyl]cyclobutyl]-6-(phenylmethoxy)-9H-purin-2-amine in 14 ml of 95% ethyl alcohol were added 7 ml of cyclohexene and 80 mg of $Pd(OH)_2/C$ (20%). The mixture was heated at reflux for 15 hours. The hot mixture was filtered through celite which was then rinsed with 50% aqueous methyl alcohol (150 ml). Rotary evaporation of the filtrate gave 85 mg of a white solid. The solid was suspended in water and chromatographed (CHP-20P resin, Mitsubishi Chemical Co., 35 - 150μ; 0% to 20% acetonitrile-water) to afford 62.9 mg of material which was suspended in water and freeze-dried to provide 52.6 mg of (1α,2β,3α,4β)-2-amino-9-[2-fluoro-3,4-bis-(hydroxymethyl)cyclobutyl]-1,9-dihydro-6H-purin-6-one as a white solid, mp 220°C (dec). $^1H$ NMR (270 MHz; DMSO-$d_6$) δ: 2.16 - 2.33 (m, 2H), 3.45 - 3.55 (m, 2H), 3.55 - 3.70 (m, 2H), 4.53 - 4.68 (m, 1H), 4.74 (t, 1H, J = 5.3 Hz), 4.81 (t, 1H, J = 5.3 Hz), 5.08 (ddd, 1H, J = 55.7 Hz, J = 7.0 Hz, J = 7.0 Hz), 6.43 (bs, 2H), 7.80 (s, 1H), 10.59 (bs, 1H).

<u>Example 2</u>

<u>(1α,2β,3α,4β)-3-(6-Amino-9H-purin-9-yl)-4-fluoro-1,2-cyclobutanedimethanol</u>

A. <u>(1α,2β,3α,4β)-9-[2-Fluoro-3,4-bis[(phenyl methoxy)methyl]cyclobutyl]-9H-purin-6-amine</u>

To a solution of 579 mg (1.19 mmol) of (1α,2α,3β,4α)-2-fluoro-3,4-bis[(phenylmethoxy)methyl]cyclobutanol, 4-methylbenzenesulfonate ester in 12 ml of dry dimethylformamide were added 484 mg (3.58 mmol) of adenine (dried under vacuum at 80°C for 24 hours), 658 mg (4.76 mmol) of $K_2CO_3$ (powdered and dried under vacuum at 80°C for 24 hours), and 946 mg (3.58 mmol) of 18-crown-6. The mixture was heated at 135°C for 5 hours. The mixture was cooled to room temperature and filtered. The solids were washed with ethyl acetate and the solvent removed by rotary evaporation. The residue was flash chromatographed on silica gel (1% to 5% $CH_3OH$-$CHCl_3$) to afford 297 mg of the title compound.

B. <u>(1α,2β,3α,4β)-3-(6-Amino-9H-purin-9-yl)-4-fluoro-1,2-cyclobutanedimethanol</u>

To a solution of 262 mg (0.586 mmol) of (1α,2β,3α,4β)-9-[2-fluoro-3,4-bis[(phenylmethoxy)methyl]cyclobutyl]-9H-purin-6-amine in 20 ml of 95% ethyl alcohol were added 10 ml of cyclohexene and 130 mg of $Pd(OH)_2/C$ (20%). The

mixture was heated at reflux for 16 hours, and another 70 mg of catalyst was added. Reflux was continued for an additional 24 hours at which time TLC (3:1 $CHCl_3$-$CH_3OH$) indicated that the reaction was complete. The hot mixture was filtered through celite which was rinsed with hot methyl alcohol (100 ml) and hot 50% aqueous methyl alcohol (100 ml). Rotary evaporation of the filtrate gave 160 mg of crude product. Chromatography (CHP-20P resin, 0% to 20% acetonitrile-water) afforded 137 mg of a white solid which was suspended in water and freeze-dried to provide 137 mg of (1α,2β,3α,4β)-3-(6-amino-9H-purin-9-yl)-4-fluoro-1,2-cyclobutanedimethanol as a white solid, mp 198 - 201°C.
$^1$H NMR (270 MHz; DMSO-$d_6$) δ: 2.15 - 2.35 (m, 1H), 2.35 - 2.48 (m, 1H), 3.50 - 3.60 (m, 2H), 3.60 - 3.70 (m,2H), 4.70 - 4.90 (m, 3H), 5.28 (ddd, 1H, J = 55.7 Hz, J = 7.0 Hz, J = 7.0 Hz), 7.21 (bs, 2H), 8.14 (s, 1H), 8.24 (s, 1H).

Example 3

(1α,2β,3α,4β)-1-[2-Fluoro-3,4-bis(hydroxymethyl) cyclobutyl]-5-methyl-2,4(1H,3H)-pyrimidinedione

A. (1α,2β,3α,4β)-1-[2-Fluoro-3,4-bis[(phenylmethoxy)methyl]cyclobutyl]-5-methyl-2,4(1H,3H)-pyrimidinedione

To a suspension of 1.39 g (11.0 mmol) of thymine in 25 ml of dimethylformamide at room temperature was added 6.5 ml (40% wt; 10.0 mmol) of aqueous tetrabutylammonium hydroxide. The mixture was stirred at room temperature for 1 hour. The solvents were removed *in vacuo* at 55°C. The resulting residue was dissolved in approximately 10 ml of dry dimethylformamide and the solvent was removed *in vacuo* (this procedure was repeated three times to remove water). The resulting tetrabutylammonium salt, which appeared as a colorless solid, was dried overnight under high vacuum (0.1 mm Hg). The salt was dissolved in 7 ml of dry dimethylformamide to give approximately 10 ml of solution. Then, 7.5 ml (~7.5 mmol) of the resulting solution was added to 623 mg (1.29 mmol) of (1α,2α,3β,4α)-2-fluoro-3,4-bis[(phenylmethoxy)methyl]cyclobutanol, 4-methylbenzenesulfonate ester. The mixture was stirred at room temperature for 5 minutes to dissolve the tosylate and then heated at 100°C for 6 hours. The reaction mixture was cooled to room temperature and allowed to stir for 30 minutes. Acetic acid (0.86 ml, 15 mmol) was added and the volatiles were removed by rotary evaporation to give a gummy residue. The residue was suspended in 50 ml each of water and ethyl acetate and then stirred in the presence of 2.5 g of slightly damp AG-MP-50 ($Na^+$) resin for 30 minutes at room temperature. The solids were removed by filtration, and the filter cake was thoroughly washed with additional quantities of both water and ethyl acetate. The ethyl acetate layer was separated and dried over $Na_2SO_4$. The solvent was removed by rotary evaporation to give 639 mg of crude product. Flash chromatography on silica gel (1:1 ethyl acetate-hexane) afforded 284 mg of the title compound.

B. (1α,2β,3α,4β)-1-[2-Fluoro-3,4-bis(hydroxymethyl)cyclobutyl]-5-methyl-2,4(1H,3H)-pyrimidinedione

To a solution of 270 mg (0.616 mmol) of (1α,2β,3α,4β)-1-[2-fluoro-3,4-bis[(phenylmethoxy)methyl]cyclobutyl]-5-methyl-2,4(1H,3H)-pyrimidinedione in 20 ml of 95% ethyl alcohol were added 10 ml of cyclohexene and 200 mg of $Pd(OH)_2/C$ (20%). The mixture was heated at reflux under argon for 24 hours. The hot mixture was filtered through celite which was rinsed with hot methyl alcohol (100 ml) and hot 50% aqueous methyl alcohol (100 ml). Rotary evaporation of the filtrate gave 175 mg of crude product. Flash chromatography on silica gel (0% to 20% isopropyl alcohol-chloroform) afforded 102 mg of a white solid which was suspended in water and freeze-dried to provide 92 mg of (1α,2β,3α,4β)-1-[2-fluoro-3,4-bis(hydroxymethyl)cyclobutyl]-5-methyl-2,4(1H,3H)-pyrimidinedione as a white solid, mp 50 - 55°C. $^1$H NMR (270 MHz, DMSO-$d_6$) δ: 1.72 (s, 3H), 1.95 - 2.15 (m, 2H), 3.35 - 3.45 (m, 2H), 3.45 - 3.55 (m, 2H), 4.48 - 4.68 (m, 3H), 4.90 (ddd, 1H, J = 55.7 Hz, J = 7.0 Hz, J = 7.0 Hz), 7.48 (d, 1H, J = 1.2 Hz), 11.18 (bs, 1H).

Example 4

(1α,2β,3α,4β)-1-[2-Fluoro-3,4-bis(hydroxymethyl)cyclobutyl]-5-iodo-2,4(1H,3H)pyrimidinedione

A. (1α,2β,3α,4β)-1-[2-Fluoro-3,4-bis[(phenylmethoxy)methyl]cyclobutyl]-2,4(1H,3H)pyrimidinedione

To a suspension of 1.14 g (10.2 mmol) of uracil in 25 ml of dimethylformamide at room temperature was added 6.1 ml of aqueous tetrabutylammonium hydroxide (40% wt; 9.3 mmol). The mixture was stirred at room temperature for 1 hour. The solvents were removed *in vacuo* at 55°C. The resulting residue was dissolved in approximately 5 ml of dry dimethylformamide and the solvent was removed *in vacuo* (this procedure was repeated four times to remove water). The resulting tetrabutylammonium salt, which appeared as a colorless solid, was dried overnight under high vacuum (0.1 mm Hg). The salt was suspended in 9 ml of dry dimethylformamide and 623 mg (1.29 mmol) of (1α,2α,3β,4α)-2-fluoro-3,4-bis[(phenylmethoxy)methyl]cyclobutanol, 4-methylbenzenesulfonate ester was added. The resulting mixture was heated at 100°C for 6 hours (when the temperature reached 55°C, the tetrabutylammonium salt dissolved yielding a homogeneous mixture). The reaction mixture was cooled to room temperature and allowed to stir for 30 minutes.

Acetic acid (1.11 g, 18.5 mmol) was added and the volatiles were removed by rotary evaporation to give a gummy residue. The residue was suspended in 62 ml each of water and ethyl acetate, and stirred for 45 minutes at room temperature. Next, 13.0 g of slightly damp AG-MP-50 ($Na^+$) resin was added and the mixture stirred for 30 minutes at room temperature. The resin and precipitated uracil were removed by filtration and the filter cake was thoroughly washed with additional quantities of both water and ethyl acetate. The ethyl acetate layer was separated and dried over $Na_2SO_4$. The solvent was removed by rotary evaporation, and purification of the residue by flash chromatography on silica gel (1:2 ethyl acetate-hexane) afforded 253 mg of the title compound as a viscous oil.

B. <u>(1α,2β,3α,4β)-1-[2-Fluoro-3,4-bis(hydroxymethyl)cyclobutyl]-2,4(1H,3H)-pyrimidinedione</u>

To a solution of 253 mg (0.60 mmol) of (1α,2β,3α,4β)-1-[2-fluoro-3,4-[bis(phenylmethoxy)methyl]cyclobutyl]-2,4(1H,3H)-pyrimidinedione in 20 ml of 95% ethyl alcohol were added 202 mg of $Pd(OH)_2/C$ and 9.5 ml of cyclohexene. The reaction mixture was heated at reflux for 4.5 hours. TLC (20% $CH_3OH$-$CH_2Cl_2$) showed no starting material remaining. The mixture was cooled slightly, filtered through celite, and washed with 50% aqueous methyl alcohol (100 ml). The solvent was removed by rotary evaporation, and purification of the residue by chromatography (CHP-20P resin; 0% to 10% $CH_3CN$-$H_2O$) afforded 111 mg of the title compound as a clear oil upon evacuation of the volatiles.

C. <u>(1α,2β,3α,4β)-1-[2-Fluoro-3,4-bis(hydroxymethyl)cyclobutyl]-5-iodo-2,4(1H,3H)pyrimidinedione</u>

To a solution of 83 mg (0.34 mmol) of (1α,2β,3α,4β)-1-[2-fluoro-3,4-bis(hydroxymethyl)cyclobutyl]-2,4(1H,3H)-pyrimidinedione in 5 ml of dioxane and $HNO_3$ (0.40 ml, 0.8N) was added 129 mg (0.51 mmol) of iodine. The mixture was heated at reflux for 3 hours. TLC (6:3:1 $CHCl_3$-$CH_3OH$-$NH_4OH$, silica) showed no starting material remaining. The mixture was cooled to room temperature and concentrated *in vacuo* to yield a dark red residue that was stored at -20°C for 48 hours. After warming to room temperature, the crude material was suspended in $H_2O$ and chromatographed (CHP-20P resin; 10% to 20% $CH_3CN$-$H_2O$). The purified material was suspended in water and freeze-dried to afford 110 mg of (1α,2β,3α,4β)-1-[2-fluoro-3,4-bis(hydroxymethyl)cyclobutyl]-5-iodo-2,4(1H,3H)-pyrimidinedione as an opaque solid, mp 211 - 213°C.
$^1H$ NMR (400 MHz; DMSO-$d_6$) δ: 2.00 - 2.20 (m, 2H), 3.35 - 3.65 (m, 4H), 4.53 - 4.66 (m, 1H), 4.70 (t, 1H, J = 5.3 Hz), 4.75 (t, 1H, J = 5.1 Hz), 5.01 (ddd, 1H, J = 55.7 Hz, J = 7.0 Hz, J = 7.0 Hz), 8.14 (s, 1H), 11.64 (s, 1H).

<u>Example 5</u>

<u>[1S-(1α,2β,3α,4β)]-2-Amino-9-[2-fluoro-3,4-bis(hydroxymethyl)cyclobutyl]-1,9-dihydro-6H-purin-6-one</u>

A. <u>(1S-trans)-3,3-Dimethoxy-1,2-cyclobutanedicarboxylic acid, di-(-)-menthyl ester</u>

Di-(-)-menthylfumarate (100 g) was dissolved in 400 ml dry toluene and cooled to -75°C under argon. To this solution was added with stirring diisobutylaluminum chloride (99.5 ml) over 15 minutes. The resulting orange mixture was stirred at -75°C for 15 minutes and 1,1-dimethoxyethylene (24.7 g; Wiley Organics) was added over 15 minutes. After stirring the reaction mixture for 10 minutes at -78°C, methanol (30 ml) was added over 15 minutes and the mixture was stirred for 30 minutes. Hexane (250 ml) was added over 5 minutes followed by the addition of 20% aqueous sodium hydroxide (40 ml) over 15 minutes at -60°C to -40°C. The reaction mixture was slowly (over 45 minutes) allowed to warm to 10°C and anhydrous magnesium sulfate (40 g) was added. The mixture was allowed to come to room temperature, was filtered, and the filtrate was concentrated *in vacuo* to afford an oil (119.5 g) which solidified under vacuum. The crude product was recrystallized from methanol-water (95:5) to afford 102 g of the title compound (isomerically pure as judged by HPLC) as a white solid, m.p. 89°C, $[\alpha]_D$ = -28.5° (c = 1, $CHCl_3$).

Alternatively, the title compound can be prepared by using diethylaluminum chloride instead of diisobutylaluminum chloride, as described below.

A solution of diethylaluminum chloride (1M in hexane, 5.1 ml) was added dropwise to a stirred solution of di(-)-menthyl fumarate (1.0 g) in 5 ml toluene under nitrogen at -78°C. The reaction mixture was stirred at that temperature for 15 minutes, followed by the addition of 0.247 g 1,1-dimethoxyethylene (Wiley Organics). The reaction mixture was stirred at -78°C for 30 minutes, then was carefully added to a mixture of 50 ml hexane and 20 ml saturated aqueous sodium bicarbonate solution.

The organic phase was washed with additional saturated sodium bicarbonate solution (2 x 20 ml), water (3 x 20 ml), and was dried (magnesium sulfate) and concentrated *in vacuo* giving 1.23 g of a thick oil. The crude mixture was recrystallized from methanol-water (95:5) affording 0.98 g of the title compound pure by HPLC and NMR.

B. (1S-trans)-3,3-Dimethoxy-1,2-cyclobutanedimethanol

To a suspension of 2.09 g (55 mmol) of lithium aluminum hydride in 365 ml of tetrahydrofuran was added over 10 minutes a solution of 17.5 g (36.4 mmol) of (1S-trans)-3,3-dimethoxy-1,2-cyclobutanedicarboxylic acid, di-(-)-menthyl ester in 75 ml of anhydrous tetrahydrofuran. The mixture was heated at 55°C for 1 hour and then cooled to 0°C. The reaction was quenched by the addition of 2.1 ml of water over 5 minutes followed by the addition of 2.1 ml of 15% aqueous sodium hydroxide. The reaction mixture was diluted with 6.3 ml of water and allowed to stir for 30 minutes. Magnesium sulfate (25 g) was added to the reaction mixture and stirring was continued for an additional 30 minutes. The solids were removed by filtration through a pad of celite and the filter cake was washed several times with additional ethyl acetate (2 x 150 ml). The solvents were removed *in vacuo*, and the residue was placed under vacuum to remove any traces of solvent. The residue was dissolved in 100 ml of heptane and washed with water (4 x 25 ml). The combined aqueous layers were extracted with 25 ml of heptane. The aqueous portion was saturated with ammonium sulfate (45 g) and extracted with ethyl acetate (4 x 50 ml). The combined ethyl acetate extracts were dried over anhydrous magnesium sulfate and the solvent was removed *in vacuo* to provide 6.28 g of the title compound as a colorless oil.

C. (2S-trans)-1,1-Dimethoxy-2,3-bis[(phenylmethoxy)methyl]cyclobutane

Sodium hydride (3.5 g, 88 mmol, 60% dispersion in mineral oil) was suspended in 50 ml of dry dimethylformamide. The suspension was cooled to 0°C, and 10.5 ml (88.3 mmol) of benzyl bromide was added to the reaction flask. To the resulting mixture was added a solution of 7.73 g (43.7 mmol) of (1S-trans)-3,3-dimethoxy-1,2-cyclobutanedimethanol in 20 ml of dry dimethylformamide. The mixture was warmed to room temperature and stirred overnight. The reaction was incomplete by TLC analysis. Additional quantities of sodium hydride (350 mg) and benzyl bromide (1.05 ml) were added. The reaction mixture was stirred for 2 hours longer, at which time TLC indicated that the reaction was complete. The mixture was diluted with diethyl ether (300 ml) and washed with saturated aqueous sodium bicarbonate (2 x 150 ml). The aqueous layer was washed with diethyl ether (100 ml) and the combined organic layers were dried over $Na_2SO_4$. The solvent was removed by rotary evaporation and the residue was purified by flash chromatography (0% to 5% ethyl acetate-hexane) to afford 12.2 g of title compound that was homogenous by TLC.

D. (2S-trans)-2,3-Bis[(phenylmethoxy)methyl]cyclobutanone

To a solution of 12.0 g (33.7 mmol) of (2S-trans)-1,1-dimethoxy-2,3-bis[(phenylmethoxy)methyl]cyclobutane in 340 ml of acetonitrile was added 112 ml of 0.5 M $H_2SO_4$. The solution was stirred at room temperature overnight. Then the solution was diluted with ethyl acetate (1200 ml) and washed in succession with water (400 ml), saturated aqueous sodium bicarbonate, and brine (400 ml). The organic layer was dried over sodium sulfate, and the solvent was removed by rotary evaporation. Flash chromatography (10% to 20% ethyl acetate-hexane) of the crude material provided 7.65 g of the title compound.

E. [2S-(2α,3β,4α)]-2-Fluoro-3,4-bis[(phenylmethoxy)methyl]cyclobutanone

To a solution of 0.71 ml (4.2 mmol) of dry 2,2,6,6-tetramethylpiperidine in 8 ml of dry tetrahydrofuran at -78°C was added 1.75 ml of n-butyllithium (3.86 mmol; 2.22 M in hexane). The resulting solution was stirred at -78°C for 30 minutes. To this was added, via cannula over 1-2 minutes, a cold (-78°C) solution of 1.00 g (3.22 mmol) of (2S-trans)-2,3-bis[(phenylmethoxy)methyl]cyclobutanone in 2 ml of dry tetrahydrofuran. The resulting solution was stirred for 30 minutes at -78°C and then warmed to -35°C. Perchloryl fluoride* (~2.5 g) was bubbled through the reaction mixture at a steady rate for 15 seconds. The mixture was purged with Argon for 5 minutes at -35°C and for 5 minutes at 0°C. The mixture was quenched by the dropwise addition of 15 ml of 1M aqueous KI and the mixture was stirred for 15 minutes at room temperature. The reaction contents were extracted with diethyl ether and the combined organic layers were washed with 5% aqueous sodium thiosulfate. The organic layer was dried over $Na_2SO_4$ and the solvent was removed by rotary evaporation. The residue was dried by azeotropic rotary evaporation with toluene to provide 1.13 g of crude fluoroketone.

F. [1R-(1α,2α,3β,4α)]-2-Fluoro-3,4-bis[(phenylmethoxy)methyl]cyclobutanol

To a solution of 26 ml (26 mmol) of lithium trisiamylborohydride (1.0 M in tetrahydrofuran) in 20 ml of dry tetrahydrofuran at -78°C was added, via cannula over a few minutes, a cold (-78°C) solution of 4.30 g (~12.9 mmol) of crude

*Caution: On two occasions violent explosions occurred, as has been documented for reactions involving organic solutions of $FC1O_3$ [see C.L.J. Wang, P.A. Grieco, F.J. Okuniewicz, Chem. Commun., 468 (1976); C.M. Sharts, W.A. Sheppard, Org. Reactions, 21, 225 (1974); W. Adcock, T.C. Khor, J. Organometal. Chem., 91, C20 (1975)]. Proper precautions should be taken to avoid injury.

[2S-(2α, 3β,4α)]-2-fluoro-3,4-bis[(phenylmethoxy)methyl]cyclobutanone in 15 ml of dry tetrahydrofuran. The mixture was stirred at -78°C for 30 minutes and then warmed to 0°C and stirred for 30 minutes. The mixture was warmed to room temperature briefly and then cooled to 0°C. The mixture was quenched by the slow addition of saturated aqueous sodium bicarbonate (80 ml) and 30% hydrogenperoxide (16 ml). The mixture was warmed to room temperature and stirred for 20 minutes. This quenched reaction mixture was combined with that from a ~10.7 mmol reaction. The resulting mixture was diluted with brine (200 ml) and extracted with ethyl acetate (600 ml, then 2 x 100 ml). The combined organic layers were washed with brine (100 ml) and dried over $Na_2SO_4$. Rotary evaporation of the solvent provided 7.68 g of crude material. Flash chromatography (20% to 50% ethyl acetate-hexane) gave 2.38 g of crude desired alcohol, which was rechromatographed (20% ethyl acetate-hexane) affording 1.29 g of the title compound as a viscous oil.

G. <u>[1R-(1α,2α,3β,4α)]-2-Fluoro-3,4-bis[(phenylmethoxy)methyl]cyclobutanol, 4-methylbenzenesulfonate ester</u>

To a solution of 1.22 g (3.69 mmol) of [1R-(1α,2α,3β,4α)]-2-fluoro-3,4-bis[(phenylmethoxy)methyl]cyclobutanol in 3.6 ml of dry pyridine was added 3.52 g (18.4 mmol) of p-toluenesulfonyl chloride. The mixture was heated at 60°C for 7 hours. The mixture was cooled to room temperature, diluted with diethyl ether (250 ml) and washed with water (4 x 100 ml), saturated aqueous sodium bicarbonate (100 ml) and brine (100 ml). The organic layer was dried over $Na_2SO_4$, and rotary evaporation of the solvent gave 5.4 g of crude material. Flash chromatography (10 to 20% ethyl acetate-hexane) afforded 0.842 g of the title compound as a white solid.

H. <u>[1S-(1α,2β,3α,4β)]-9-[2-Fluoro-3,4-bis[(phenylmethoxy)methyl]cyclobutyl]-6-(phenylmethoxy)-9H-purin-2-amine</u>

To a solution of 832 mg (1.72 mmol) of [1R-(1α,2α,3β,4α)]-2-fluoro-3,4-bis[(phenylmethoxy)methyl]cyclobutanol, 4-methylbenzenesulfonate ester in 9 ml of dry dimethylformamide were added 2.1 g (8.6 mmol) of 2-amino-6-(phenylmethoxy)-9H-purine, 1.78 g (12.9 mmol) of $K_2CO_3$ (powdered and dried under vacuum at 75°C for 18 hours), and 2.3 g (8.6 mmol) of 18-crown-6. The mixture was heated at 110°C for 18 hours. The mixture was cooled to room temperature and filtered. The solvent was removed by rotary evaporation, and the residue was purified by flash chromatography (20% to 50% ethyl acetate-hexane) to afford 268 mg of the title compound.

I. <u>[1S-(1α,2β,3α,4β)]-2-Amino-9-[2-fluoro-3,4-bis(hydroxymethyl)cyclobutyl]-1,9-dihydro-6H-purin-6-one</u>

To a solution of 265 mg (0.479 mmol) of [1S-(1α,2β,3α,4β)]-9-[2-fluoro-3,4-bis[(phenylmethoxy)methyl]cyclobutyl]-6-(phenylmethoxy)-9H-purin-2-amine in 31 ml of 95% ethyl alcohol were added 15 ml of cyclohexene and 133 mg of $Pd(OH)_2/C$ (20%). The mixture was heated at reflux for 22 hours. The hot mixture was filtered through celite, which was rinsed with 50 ml of hot ethyl alcohol. Rotary evaporation of the solvent gave 118 mg of a white solid. The solid was dissolved in 8 ml of 10% acetonitrile-water and chromatographed (CHP-20P resin; 0% to 40% acetonitrile-water). The solvent was removed by rotary evaporation and the product was freeze-dried to provide 86 mg of [1S-(1α,2β,3α,4β)]-2-amino-9-[2-fluoro-3,4-bis-(hydroxymethyl)cyclobutyl]-1,9-dihydro-6H-purin-6-one as a white solid, mp 234°C (dec). $[\alpha]^{22}_D$ -8.0 (c = 1, $H_2O$); $^1H$ NMR (270 MHz; DMSO-$d_6$) δ: 2.15 - 2.35 (m, 2H), 3.45 - 3.55 (m, 2H), 3.55 - 3.65 (m, 2H), 4.53 - 4.67 (m, 1H), 4.78 (t, 1H, J = 5.3 Hz), 4.84 (t, 1H, J = 5.3 Hz), 5.08 (ddd, 1H, J = 55.7 Hz, J = 7.0 Hz, J = 7.0 Hz), 6.44 (bs, 1H), 7.81 (s, 1H), 10.57 (bs, 1H).

<u>Example 6</u>

<u>[1S-(1α,2β,3α,4β)]-4-Amino-1-[2-fluoro-3,4-bis-(hydroxymethyl)cyclobutyl]-2(1H)-pyrimidinone</u>

A. <u>Chlorofluoroacetaldehyde diethyl acetal</u>

To a stirred solution of 100 g. (709 mmol) of ethyl chlorofluoroacetate in 550 mL dry toluene at -75° to -78°C under argon was added dropwise 780 mL of a solution of diisobutylaluminum hydride (1M in toluene, 780 mmol). The reaction mixture was stirred at that temperature for 30 minutes, then transferred via cannula to a stirred solution of 230 g. (6.3 moles) anhydrous HCl in absolute ethanol (1000 mL) at -78°C. The mixture was allowed to come to room temperature and was fractionally distilled using a 2 foot long Vigreux column until ca. 1800 mL distillate had been collected. The remaining material was flash distilled (slowly increasing the vacuum from 150 mm to 0.1 mm until no more liquid remained in the pot) and collected separately. The flash distillate was combined with 25 mL ethanol and subjected to a second fractional distillation under atmospheric pressure, affording 55 g. of the title compound as a colorless liquid, boiling point 152 - 154°C. Partial $^1H$ NMR (270 MHz, $CDCl_3$)δ5.94 (dd, J = 5.3, 50.4 Hz, H-CF).

B. 2-Fluoro-1,1-diethoxyethene

To a stirred suspension of 50.0 g.(445.6 mmol) potassium t-butoxide in 250 mL tetrahydrofuran at 0°C under argon was added dropwise a solution of 55.0g. (321.6 mmol) chlorofluoroacetaldehyde diethyl acetal in 50 mL dry tetrahydrofuran. The reaction mixture was stirred at 35°C for 12 hours, refluxed for one hour, and then fractionally distilled using a 1 foot long Vigreux column until 250 mL of solvent had been collected. The remaining material was flash distilled (slowly increasing the vacuum from 150 mm to 0.1 mm until no more liquid remained in the pot) and collected separately. The flash distillate was subjected to a second fractional distillation under atmospheric pressure, affording 29.5 g. of the title compound as a colorless liquid, boiling point 123 - 126°C. Partial $^1H$ NMR (270 MHz, $CDCl_3$)δ6.35 (d, J=77.4 Hz, 1H, H-CF).

C. [1S-(1α,2β,4α)]-4-Fluoro-3,3-diethoxy-1,2-cyclobutanedicarboxylic acid, bis-(-)-menthyl ester

To a stirred solution of 29.03 g. (73.95 mmol) of (-)-dimenthyl fumarate (Aldrich Chemical Co.) in 400 mL dry toluene at -78°C under argon was added dropwise 175 mL (175 mmol) of a solution of diethylaluminum chloride (1M in hexane). The resulting dark orange reaction mixture was stirred at that temperature for 15 minutes followed by the addition of the freshly prepared 2-fluoro-1,1-diethoxyethene dropwise over 5 minutes at -78°C. The reaction mixture was stirred at that temperature for 30 minutes, quenched by sequential dropwise addition of methanol (10 mL), 20% NaOH (10 mL), hexane (100 mL) and magnesium sulfate (20 g). The mixture was slowly allowed to come to room temperature, filtered and the filtrate concentrated in vacuo, affording a thick colorless oil. The crude mixture was recrystallized twice, first from pentane, followed by hexane, affording 13.9 g. of title compound along with its epimer [1S-(1α,2β,4β)]-4-fluoro-3,3-diethoxy-1,2-cyclobutanedicarboxylic acid, bis-(-)-menthyl ester (ratio ca. 5:1 by NMR). Partial $^1H$ NMR of the title compound (270 MHz, $CDCl_3$)δ5.06 (dd, J = 8.0, 54.2 Hz; 1H, H-CF), 4,72 (dt, J = 4.7, 11.1 Hz, 2H), 3.78 - 3.37 (m, 4H).

D. [1S-(1α,2β,4α)]-4-Fluoro-3,3-diethoxy-1,2-cyclobutanedimethanol

A solution of 13.8 g. (26.2 mmol) of the mixture of [1S-(1α,2β,4α)]-4-fluoro-3,3-diethoxy-1,2-cyclobutanedicarboxylic acid, bis-(-)-menthyl ester and its epimer [1S-(1α,2β,4β)]-4-fluoro-3,3-diethoxy-1,2-cyclobutanedicarboxylic acid, bis-(-)-menthyl ester (ratio about 5:1) in 50 mL dry tetrahydrofuran was added dropwise under argon to a stirred suspension of 1.49 g (39.3 mmol) lithium aluminum hydride in 100 mL tetrahydrofuran at 0°C. The mixture was stirred at room temperature for 2 hours, quenched at 0°C by sequentially adding 1.5 mL water, 4.5 mL 1N NaOH and 1.5 mL water. The solids were removed by filtration and the filtrate was concentrated in vacuo. The crude product was subjected to flash chromatography on silica gel (hexane-ethyl acetate) giving 5.1 g. of a colorless oil containing the title compound and its epimer [1S-(1α,2β,4β)]-4-fluoro-3,3-diethoxy-1,2-cyclobutanedimethanol (ratio about 5:1 by NMR). Partial $^1H$ NMR of the title compound (270 MHz, $CDCl_3$)δ5.02 (dd, J = 6.6, 54.0 Hz, 1H, H-CF).

E. [2R-(2α,3α,4β)-2-Fluoro-1,1-diethoxy-3,4-bis[(phenylmethoxy)methyl]cyclobutane

To a stirred suspension of 2.252 g. (56.3 mmol, 60% in oil) of ether-washed NaH in 10 mL dimethylformamide under argon at 10-15°C was added sequentially 5.62 mL (47.3 mmol) benzyl bromide and a dimethylformamide solution (20 mL) containing 5.0 g (22.5 mmol) of the mixture of [1S-(1α,2β,4β)]-4-fluoro-3,3-diethoxy-1,2-cyclobutanedimethanol and [1S-(1α,2β,4α)]-4-fluoro-3,3-diethoxy-1,2-cyclobutanedimethanol (ratio about 1:5). The reaction mixture was stirred at room temperature for 12 hours, diluted with ether and washed with water. The organic phase was dried over magnesium sulfate and concentrated in vacuo. The crude oil was subjected to flash chromatography on silica gel (hexane to hexane-ether 5:1) affording 8.5 g of the title compound and its epimer [2S-(2α,3β,4α)-2-fluoro-1,1-diethoxy-3,4-bis[(phenylmethoxy)methyl]cyclobutane (ratio about 5:1). Partial $^1H$ NMR of the title compound (270 MHz, $CDCl_3$)δ4.95 (dd, J = 5.9, 53.9 Hz, 1H, H-CF).

F. [2R-(2α,3α,4β)]-2-Fluoro-3,4-bis[(phenylmethoxy)methyl]cyclobutanone

A solution of 8.4 g. (20.9 mmol) of the mixture of [2R-(2α,3α,4β)-2-fluoro-1,1-diethoxy-3,4-bis[(phenylmethoxy)methyl]cyclobutane and its epimer [2S-(2α,3β, 4α)-2-fluoro-1,1-diethoxy-3,4-bis[(phenylmethoxy)methyl]cyclobutane (ratio about 5:1) in 150 mL acetonitrile and 75 mL 0.5 M sulfuric acid was refluxed for 3 hours. The solution was allowed to come to room temperature, diluted with ethyl acetate and washed with saturated sodium bicarbonate. The organic layer was dried ($MgSO_4$) and concentrated in vacuo, giving 6.8 g. of the title compound and its epimer, [2S-(2α,3β,4α)]-2-fluoro-3,4-bis[(phenylmethoxy)methyl]cyclobutanone (ratio by NMR = about 5:1). Partial $^1H$ NMR of the title compound (270 MHz, $CDCl_3$)δ 5.58 (ddd, J = 3.51, 9.38, 53.35 Hz, 1H, H-CF).

G. [2S-(2α,3β,4α)-2-Fluoro-3,4-bis[(phenylmethoxy)methyl]cyclobutanone

To a stirred solution of 6.8 g. (20.73 mmol) of the mixture of [2R-(2α,3α,4β)-2-fluoro-3,4-bis[(phenylmethoxy)methyl]cyclobutanone and its epimer [2S-(2α,3β,4α)-2-fluoro-3,4-bis[(phenylmethoxy)methyl]cyclobutanone (ratio about 5:1) in 300 mL dry acetonitrile was added 0.31 mL (2.07 mmol) 1,8-diazabicyclo[5.4.0]undec-7-ene over 10 minutes at 0°C. The mixture was allowed to come to room temperature and was stirred for 3 hours (monitored by $^1$H NMR). The mixture was diluted with 500 mL ethyl acetate, washed with a 1:1 mixture of 20% sulfuric acid and saturated sodium sulfate solutions (3 x 500 mL). The organic phase was concentrated approximately to 100 mL, diluted with 250 mL methylene chloride, dried ($MgSO_4$) and concentrated in vacuo to afford 6.8 g. of the title compound and its epimer [2R-(2α,3α,4β)-2-fluoro-3,4-bis[(phenylmethoxy)methyl]cyclobutanone (ratio about ≧ 5:1). Partial $^1$H NMR of the title compound (270 MHz, $CDCl_3$) δ 5.43 (ddd, J = 3.5, 9.4, 53.6 Hz, 1H, H-CF).

H. [1R-(1α,2α,3β,4α)-2-Fluoro-3,4-bis[(phenylmethoxy)methyl]cyclobutanol

A solution of 22.8 mℓ(22.8 mmol) of lithium trisiamylborohydride (1M in tetrahydrofuran) was added dropwise under argon at -78°C. to a stirred solution of 6.8 g. (20.7 mmol, crude from the previous step) of the mixture of [2S-(2α,3β,4α)-2-fluoro-3,4-bis[(phenylmethoxy)methyl]cyclobutanone and [2R-(2α,3α,4β)-2-fluoro-3,4-bis[(phenylmethoxy)methyl]cyclobutanone (ratio about 5:1) in 200 mL dry tetrahydrofuran. The reaction mixture was stirred at -78°C for 15 minutes, 0°C for 15 minutes and quenched with 14 mL saturated sodium bicarbonate solution and 7 mL 30% hydrogen peroxide. The mixture was stirred at 0°C for 30 minutes, diluted with ethyl acetate and washed sequentially with saturated sodium bicarbonate and brine. The organic phase was dried ($MgSO_4$) and concentrated in vacuo, affording a gummy residue which was subjected to flash chromatography on silica gel (hexane to 10 - 20% ethyl acetate in hexane) to give 4.81 g. of the title compound as a colorless oil. Partial $^1$H NMR (270 MHz, $CDCl_3$)δ 4.78 (td, J = 6.5, 54.0 Hz).

I. [1R-(1α,2α,3β,4α)-2-Fluoro-3,4-bis[(phenylmethoxy)methyl]cyclobutanol, 4-methylbenzenesulfonate ester

A mixture of 13.87 g. (72.73 mmol) tosyl chloride and 4.8 g. (14.55 mmol) of [1R-(1α,2α,3β,4α)-2-fluoro-3,4-bis[(phenylmethoxy)methyl]cyclobutanol in 10 mL dry pyridine was heated at 70°C for 20 hours. TLC (silica gel; hexane-ethyl acetate 3:1) indicated incomplete reaction. Additional tosyl chloride (6.94 g., 36.4 mmol) was added to the reaction mixture which was heated at 100°C for 5 hours. The mixture was cooled to room temperature, diluted with ethyl acetate, washed with water and then brine. The organic phase was dried ($MgSO_4$), concentrated in vacuo and the residue flash chromatographed on silica gel (hexane to 5 - 20% ethyl acetate in hexane) affording 2.3 g. of the title compound and 2.4 g. of unreacted [1R-(1α,2α,3β,4α)-2-fluoro-3,4-bis[(phenylmethoxy)methyl]cyclobutanol (eluted after the tosylate). Partial $^1$H NMR (270 MHz, $CDCl_3$) δ 7.85 (d, J = 8.2 Hz, 2H) 7.38 - 7.19 (s, 12 H), 5.08 (m, 1H, HC-OTs), 4.80 (td, J = 6.5, 52.2 Hz), 4.48 (s, 2H, benzyl), 4.42 (AB quartet, J = 11.7 Hz, 2H, benzyl).

J. [1S-(1α,2β,3α,4β)]-1-[2-Fluoro-3,4-bis[(phenylmethoxy)methyl]cyclobutyl]-2,4(1H,3H)-pyrimidinedione

To a suspension of 4.63 g. (41.3 mmol) of uracil in 10 mL dimethylformamide was added 21.4 mL (36.2 mmol) of a 1.5 M aqueous solution of tetrabutylammonium hydroxide. The mixture was stirred at room temperature for 10 minutes, concentrated in vacuo, and the residue redissolved and concentrated 4 times from 35 mL dry dimethylformamide. The resulting gummy uracil tetrabutylammonium salt was dried overnight at 65°C. under vacuum (0.1 mm.). To a solution of this salt in 30 mL dry dimethylformamide was added 2.3 g. (5.2 mmol) [1R-(1α,2α, 3β,4α)-2-fluoro-3,4-bis[(phenylmethoxy)methyl]cyclobutanol, 4-methylbenzenesulfonate ester and the mixture was stirred at 100°C for 12 hours. The reaction mixture was cooled to room temperature followed by the addition of acetic acid (2.48 g., 41.3 mmol) and concentration in vacuo. The residue was dissolved in 150 mL water and 150 mL ethyl acetate and the resulting solution was stirred for 2 hours with 150 g. AGMP-50 resin ($Na^+$ form). The mixture was filtered and the aqueous layer of the filtrate extracted with 3 x 100 mL ethyl acetate. The combined organic phase was dried ($MgSO_4$), concentrated in vacuo and the residue subjected to flash chromatography on silica gel (hexane to 1:1 hexane - ethyl acetate) affording 1.35 g. of the title compound as a pale gummy solid. Partial $^1$H NMR (270 MHz, $CDCl_3$) δ 7.19 (d, J = 8.2, 1H), 5.13 (td, J = 6.5, 54.5 Hz, 1H, H-CF).

K. [1S-(1α,2β,3α,4β)]-4-Amino-1-[2-fluoro-3,4-bis[(phenylmethoxy)methyl]cyclobutyl]-2(1H)pyrimidinone

To a stirred solution of 230 mg. (0.54 mmol) of [1S-(1α,2β,3α,4β)]-1-[2-fluoro-3,4-bis[(phenylmethoxy)methyl]cyclobutyl]-2,4(1H,3H)-pyrimidinedione in dry pyridine (1.5 mL) under argon was added 0.239 mL (1.47 mmol) p-chlorophenyl dichlorophosphate. The reaction mixture was stirred at room temperature for 10 minutes and 207 mg. (2.99 mmol) 1,2,4-triazole was added, stirred at room temperature for 4 days and concentrated in vacuo. The residue

was dissolved in methylene chloride, washed with water and saturated sodium bicarbonate. The organic phase was dried over magnesium sulfate and concentrated in vacuo.

To the resulting triazole derivative dissolved in 5 mL dioxane was added 5 mL 29% ammonium hydroxide and the mixture was stirred at room temperature for 24 hours. The mixture was concentrated in vacuo, redissolved in methylene chloride and washed with 5% NaOH followed by water. The organic layer was dried over magnesium sulfate, concentrated in vacuo and the residue subjected to flash chromatography on silica gel (2-10% ethanol in ethyl acetate) affording 0.14 g. of the title compound as a gummy solid. Partial $^1$H NMR (270 MHz, $CDCl_3$) δ 7.30 (m, 12 H), 7.22 (d, J = 7.62 Hz, 1H), 5.65 (d, J = 7.62 Hz, 1H), 5.18 (td, J = 6.55, 55.7 Hz, 1H, H-CF).

L. [1S-(1α,2β,3α,4β)]-4-Amino-1-[2-fluoro-3,4-bis(hydroxymethyl)cyclobutyl]-2(1H)-pyrinidinone

A mixture of 0.14 g. (0.332 mmol) [1S-(1α,2β, 3α,4β)]-4-amino-1-[2-fluoro-3,4-bis[(phenylmethoxy)methyl]cyclobutyl]-2(1H)-pyrimidinone, 20% palladium hydroxide on carbon (200 mg), and cyclohexene (7.5 mL) in 15 mL 95% ethanol was heated under reflux for 4 hours. The mixture was allowed to come to room temperature and filtered through celite. The filtrate was concentrated in vacuo and the residue was subjected to CHP-20 chromatography (water to 1% acetonitrile in water) affording 60 mg. of the title compound as a white solid, m.p. 145 - 150°C, $[\alpha]_D$ = -6.5° (water, C = 2). $^1$H NMR (270 MHz, DMSO-$d_6$) δ 8.70 (br s, 1H), 8.01 (br s, 1H), 7.89 (d, J = 7.0 Hz, 1H), 5.97 (d, J = 7.0 Hz, 1H), 4.98 (td, J = 7.0, 55.1 Hz, 1H, H-CF), 4.68 - 4.53 (m, 1H in a deuterium exchanged spectrum in presence of $D_2O$, 3H in the original unchanged spectrum), 3.66 - 3.48 (m, 4H), 2.25 - 2.07 (m., 2H)

## Example 7

[1S-(1α(E),2β,3α,4β)-1-[2-Fluoro-3,4-bis(hydroxymethyl)cyclobutyl]-5-(2-bromoethenyl)-2,4(1H,3H)pyrimidinedione

A. [1S-(1α,2β,3α,4β)]-1-[2-Fluoro-3,4-bis(hydroxymethyl)cyclobutyl]-2,4(1H,3H)-pyrimidinedione

To a stirred solution of 1.35 g. (3.2 mmol) of [1S-(1α,2β,3α,4β)]-1-[2-fluoro-3,4-bis[(phenylmethoxy)methyl]cyclobutyl]-2,4(1H,3H)-pyrimidinedione in 95% ethanol (100 mL) was added sequentially cyclohexene (50 mL) and 20% palladium hydroxide on carbon (1 g). The mixture was heated under reflux for 4 hours, allowed to come to room temperature, filtered through celite and concentrated in vacuo. The residue was subjected to CHP-20 chromatography (water to 1% acetonitrile-water), affording 0.62 g. of the title compound as a colorless gummy solid. Partial $^1$H NMR (270 MHz, DMSO-$d_6$) δ 11.31 (br s, 1H, NH), 7.68 (d, J = 8.2 Hz, 1H), 5.62 (d, J = 8.2 Hz, 1H), 4.95 (td, J = 6.4, 55.7 Hz, 1H, H-CF).

B. [1S-(1α,2β,3α,4β)]-1-[2-Fluoro-3,4-bis(hydroxymethyl)cyclobutyl]-5-iodo-2,4(1H,3H)pyrimidinedione

A mixture of 0.62 g. (2.54 mmol) [1S-(1α,2β, 3α,4β)]-1-[2-fluoro-3,4-bis(hydroxymethyl)cyclobutyl]-2,4(1H,3H)pyrimidinedione, 0.968 g. (3.81 mmol) iodine, and 3 mL 0.8 N nitric acid in 30 mL dioxane was heated under reflux for 4 hours. The mixture was allowed to come to room temperature and was adjusted to pH 7 using saturated sodium bicarbonate solution. The mixture was concentrated and the residue subjected to CHP-20 chromatography (water to 0 - 20% acetonitrile-water) affording 0.53 g. of the title compound as a white solid. Partial $^1$H NMR (270 MHz, DMSO-$d_6$) δ 11.68 (br s, 1H, NH), 8.15 (s, 1H, C6-H), 5.11 (td, J = 6.5, 55.1 Hz, 1H, H-CF).

C. [1S-[1α(E),2β,3α,4β]]-3-[1-[2-Fluoro-3,4-bis(hydroxymethyl)cyclobutyl]-1,2,3,4-tetrahydro-2,4-dioxo-5-pyrimidinyl]-2-propenoic acid, methyl ester

To 2.5 mL of deoxygenated, anhydrous dioxane under argon was added sequentially 33 mg (0.135 mmol) palladium acetate, 71 mg (0.27 mmol) triphenylphosphine, and 0.565 mL (4.05 mmol) triethylamine. The mixture was stirred at 70° C for 15 minutes. A solution of 0.5 g. (1.35 mmol) [1S-(1α,2β,3α,4β)]-1-[2-fluoro-3,4-bis(hydroxymethyl)cyclobutyl]-5-iodo-2,4(1H,3H)pyrimidinedione in 2.5 mL of deoxygenated dioxane was added to the dark brown reaction mixture, followed by the addition of 0.487 mL (5.4 mmol) methyl acrylate. The reaction mixture was stirred at 70°C. for 4 hours, allowed to come to room temperature, filtered through celite and concentrated in vacuo. The residue was triturated from methylene chloride affording 400 mg. of the title compound as a light brownish solid. Partial $^1$H NMR (270 MHz, DMSO-$d_6$) δ 11.65 (br s, 1H,NH), 8.34 (s, 1H, C6-H), 7.43 (d, J = 16 Hz, 1H), 6.90 (d, J = 16 Hz,1H).

D. [1S-[1α(E),2β,3α,4β]]-3-[1-[2-Fluoro-3,4-bis(hydroxymethyl)cyclobutyl]-1,2,3,4-tetrahydro-2,4-dioxo-5-pyrimidinyl]-2-propenoic acid

A solution of 390 mg. (1.19 mmol) [1S-[1α(E), 2β,3α,4β]]-3-[1-[2-fluoro-3,4-bis(hydroxymethyl)cyclobutyl]-1,2,3,4-tetrahydro-2,4-dioxo-5-pyrimidinyl]-2- propenoic acid methyl ester in 8 mL 2N KOH was stirred at room temperature for 1 hour. The reaction mixture was concentrated in vacuo and the residue dissolved in ice cold 10% HCl (2 mL). The pH was adjusted to 1.7 with additional 10% HCl. The mixture was kept in the refrigerator for 3 hours after which the solids were filtered, washed with cold water and dried to afford 232 mg. of crude product. The combined filtrate was concentrated to one half volume and kept overnight in a refrigerator. Filtration afforded an additional 35 mg. product. This was combined with the previous batch and recrystallized from water, affording 233 mg. of the title compound. Partial $^1H$ NMR (270 MHz, DMSO-$d_6$) δ 12.13 (br s, 1H, carboxylic acid), 11.63 (br s, 1H,NH), 8.27 (s, 1H, H-C6), 7.36 (d, J = 15.8 Hz, 1H), 6.82 (d, J = 15.8 Hz, 1H), 4.93 (td, J = 5.9, 55.7 Hz, 1H, H-CF).

E. [1S-(1α(E),2β,3α,4β)]-1-[2-Fluoro-3,4-bis(hydroxymethyl)cyclobutyl]-5-(2-bromoethenyl)-2,4(1H,3H)-pyrimidinedione

To a stirred suspension of 90 mg. (0.287 mmol) of [1S-[1α(E),2β,3α,4β]]-3-[1-[2-fluoro-3,4-bis(hydroxymethyl)cyclobutyl]-1,2,3,4-tetrahydro-2,4-dioxo-5-pyrimidinyl]-2-propenoic acid and 86.1 mg. (0.86 mmol) potassium bicarbonate in 1 mL dry dimethylformamide was added a solution of 51 mg. (0.287 mmol) N-bromosuccinimide in dry dimethylformamide (0.51 mL) over 15 minutes. The reaction mixture was stirred under argon for 1 hour at room temperature, filtered and concentrated in vacuo. The residue was subjected to CHP-20 chromatography (25 mL slurry) using a 0 to 33% acetonitrile-water continuous gradient (600 mL) to afford a white residue. Recrystallization of this material from water gave 60 mg of the title compound, m.p. 185 - 187°C (dec.), $[\alpha]_D$ = -11.6° (methanol, C = 0.32). $^1H$ NMR (270 MHz, DMSO-$d_6$) δ 11.57 (br s, 1H, NH), 7.94 (s, 1H, H-$C_6$), 7.28 (d, J = 13.2 Hz, 1H, bromovinyl), 7.16 (d, J = 13.2 Hz, 1H, bromovinyl), 4.92 (td, J = 6.6, 55.4 Hz, 1H, H-CF), 4.80 - 4.62 (m, 3H), 3.55 (m, 2H), 3.48 (m,2H), 2.22 - 2.02 (m, 2H).

Example 8

[1S-(1α(E),2β,3α,4β)]-1-[2-Fluoro-3,4-bis(hydroxymethyl)cyclobutyl]-5-(2-iodoethenyl)-2,4(1H,3H)pyrimidinedione

A mixture of 70 mg. (0.223 mmol) [1S-[1α(E), 2β,3α,4β]]-3-[1-[2-fluoro-3,4-bis(hydroxymethyl)cyclobutyl]-1,2,3,4-tetrahydro-2,4-dioxo-5-pyrimidinyl]-2-propenoic acid and 48.3 mg. (0.446 mmol) potassium acetate in 0.5 mL dimethylformamide was stirred under argon at room temperature for 15 minutes followed by the addition of a solution of 50.2 mg. (0.223 mmol) N-iodosuccinimide in 0.5 mL dimethylformamide over 15 minutes. The reaction mixture was heated at 50°C for 2.5 hours, filtered and the filtrate concentrated in vacuo. The crude product was subjected to CHP-20 chromatography (using a 0 to 30% acetonitrile in water gradient) affording 60 mg. of the title compound as a white solid m.p. 93 - 96°C, $[\alpha]_D$ = -6.5° (methanol, C = 0.333). $^1H$ NMR (270 MHz, DMSO-$d_6$) δ 11.55 (br s, 1H, NH), 7.93 (s, 1H, H-C6), 7.24 (d, J = 14.5 Hz, 1H, iodovinyl), 7.16 (d, J = 14.5 Hz, 1H, iodovinyl), 4.92 (td, J = 6.6, 55.4 Hz, 1H, H-CF), 4.80 - 4.60 (m, 3H), 3.55 (m, 2H), 3.50 (m, 2H), 2.18 -2.02 (m, 2H).

Example 9

[1R-(1α,2β,3α,4β)]-2-Amino-9-[2-fluoro-3,4-bis(hydroxymethyl)cyclobutyl]-1,9-dihydro-6H-purin-6-one

A. [1R-(1α,2β,4α)]-4-Fluoro-3,3-diethoxy-1,2-cyclobutanedicarboxylic acid, bis-(+)-menthyl ester

To a solution of 22.7 g. (58 mmol) (+)-dimenthylfumerate in 115 ml. of dry degassed toluene at -78°C under an argon atmosphere was added 114 ml (1.0 M, 1.14 mmol) of a solution of diethylaluminum chloride in hexanes dropwise over 30 minutes keeping the reaction temperature between -74° and -78°C. After the addition was complete the reaction was stirred at -78°C. for an additional 30 minutes. 2-Fluoro-1,1-diethoxyethene (8.6 g., 64 mmol) was added over a 5 minute period and then the reaction was stirred for 1.5 hours at -78°C. A saturated solution of sodium bicarbonate (150 ml) was added and the resulting mixture was allowed to warm to room temperature. The aqueous mixture was extracted with 250 ml. of hexanes, washed with water and dried over sodium sulfate. The solvent was evaporated in vacuo yielding 34.6 g. of crude material. The crude material was crystallized from pentane yielding 10.2 g. of the title compound as a colorless solid.

B. [1R-(1α,2β,4α)]-4-Fluoro-3,3-diethoxy-1,2-cyclobutanedimethanol

A solution of 8.6 g. (16.3 mmol) [1R-(1α,2β, 4β)]-4-fluoro-3,3-diethoxy-1,2-cyclobutanedicarboxylic acid, bis-(+)-menthyl ester in 40 ml. of dry tetrahydrofuran was added dropwise to a suspension of 0.928 g. (24.6 mmol) lithium aluminum hydride in 150 ml. of dry tetrahydrofuran under an argon atmosphere at room temperature. After the addition was complete the reaction was heated at 50°C. for 1.5 hours. Water (1 ml.) was carefully added and the reaction mixture stirred for 15 minutes. Additions of 1.0 ml. of a 15% sodium hydroxide solution and 3.4 ml. of water were made and the reaction stirred for 30 minutes at room temperature. Magnesium sulfate (24.5 g.) was added and the mixture stirred for 15 minutes. The solids were removed by filtration and the filter cake was washed with ethyl acetate. The organic solution was concentrated in vacuo to afford the crude product which was column chromatographed on silica gel (250 ml.) eluting with 25% ethyl acetate/hexanes (1000 ml.) followed by 60% ethyl acetate/hexanes (2000 ml.). The appropriate fractions were combined and the solvents removed in vacuo yielding 3.25 g. of the title compound as a colorless oil.

C. [2S-(2α,3α,4β)]-2-Fluoro-1,1-diethoxy-3,4-bis[(phenylmethoxy)methyl]cyclobutane

A solution of 3.22 g. (14.5 mmol) of [1R-(1α,2β,4α)]-4-fluoro-3,3-diethoxy-1,2-cyclobutanedimethanol in 3.5 ml. of dry dimethylformamide was added over a 5 minute period to a stirred mixture of sodium hydride (60% in oil, 1.16 g., 29 mmol) and 3.8 ml. (31.9 mmol) benzyl bromide in 5 ml. of dry dimethylformamide at 0°C under an argon atmosphere. The reaction mixture was allowed to slowly warm to ambient temperature and was stirred overnight. Additional amounts of benzyl bromide (0.380 ml., 55 mg.) and 60% sodium hydride (120 mg.) were added. The reaction mixture was then allowed to stir for 5 hours. The mixture was diluted with ether and washed with saturated sodium bicarbonate (3 x 40 ml.). The aqueous portions were back extracted with ether (2 x 50 ml.) and the combined ether extracts were dried over anhydrous magnesium sulfate. The solvent was removed in vacuo yielding the crude product as an oily residue which was column chromatographed on silica gel (800 ml.) eluting with hexanes (1500 ml.) followed by 5% ethyl acetate/hexanes (3000 ml.). The appropriate fractions were combined and the solvents removed in vacuo yielding 4.3 g. of the title compound.

D. [2S-(2α,3α,4β)]-2-Fluoro-3,4-bis[(phenylmethoxy)methyl]cyclobutanone

A solution of 4.1 g. (10.2 mmol) of [2S-(2α,3α,4β)]-2-fluoro-1,1-diethoxy-3,4-bis[(phenylmethoxy)methyl]cyclobutane in a mixture of 60 ml. of acetonitrile and 30 ml. of 0.5 M aqueous sulfuric acid was heated at 90°C. for 3 hours. The mixture was cooled to room temperature and diluted with 800 ml. of ethyl acetate. The mixture was washed with 600 ml. of a saturated solution of sodium bicarbonate and 600 ml. of brine. The aqueous mixture was back extracted with ethyl acetate and the combined ethyl acetate extracts were dried over anhydrous magnesium sulfate. The ethyl acetate was removed in vacuo yielding 3.2 g. of the title compound as a colorless oily residue.

E. [2R-(2α,3β,4α)-2-fluoro-3,4-bis[(phenylmethoxy)methyl]cyclobutanone

A solution of 3.2 g. (9.7 mmol) of [2S-(2α,3α,4β)]-2-fluoro-3,4-bis[(phenylmethoxy)methyl]cyclobutanone in 775 ml. of acetonitrile was added over a 10 minute period to a solution of 0.221 ml. (9.7 mmol) 1,8-diazabicyclo[5.4.0]undec-7-ene in acetonitrile at 0°C. under an argon atmosphere. The reaction mixture was stirred at 0° C. under an argon atmosphere for 15 minutes and was then allowed to warm to ambient temperature and stirred for 4 hours. The reaction mixture was diluted with 3500 ml. of ethyl acetate and washed with a (1:1) mixture of 20% sulfuric acid/saturated sodium sulfate solution (3 x 3500 ml). The ethyl acetate mixture was dried over anhydrous magnesium sulfate and the solvent removed in vacuo yielding 3.05 g. of the title compound (ratio about ≧ 5:1) as a colorless oily residue.

F. [1S-(1α,2α,3β,4α)-2-Fluoro-3,4-bis[(phenylmethoxy)methyl]cyclobutanol

To a solution of 3.0 g. (9.1 mmol) of the mixture of [2R-(2α,3β,4α)-2-fluoro-3,4-bis[(phenylmethoxy)methyl]cyclobutanone and its epimer [2S-(2α,3α,4β)-2-fluoro-3,4-bis[(phenylmethoxy)methyl]cyclobutanone (ratio about ≧ 5:1) in 275 ml. of dry tetrahydrofuran at -78°C. under an argon atmosphere was added a solution of lithium trisiamylborohydride in hexanes (1.0 M, 14 ml.). The reaction mixture was stirred at -78°C for 15 minutes and then allowed to warm to room temperature. A saturated aqueous sodium bicarbonate solution (275 ml.) was added followed by 3 ml. of a 30% hydrogen peroxide solution. The reaction mixture was stirred for 15 minutes and then extracted with 400 ml. of ethyl acetate. The ethyl acetate extract was washed with a solution of brine and dried over anhydrous magnesium sulfate. The solvent was removed in vacuo yielding 2.5 g. of crude product as an oily residue which was column chromatographed on silica gel (250 ml.) eluting with 10% ethyl acetate/hexanes (500 ml.), 15% ethyl acetate/hexanes and 20% ethyl acetate/hexanes (1000 ml.). The appropriate fractions were combined and the solvents removed in vacuo to afford 840 mg. of the title compound as a colorless oil.

G. [1S-(1α,2α,3β,4α)-2-Fluoro-3,4-bis[(phenylmethoxy)methyl]cyclobutanol, 4-methylbenzenesulfonate ester

A solution of 800 mg. (2.4 mmol) of [1S-(1α,2α,3β,4α)-2-fluoro-3,4-bis[(phenylmethoxy)methyl]cyclobutanol and 2.29 g. (12 mmol.) p-toluenesulfonyl chloride in 2.3 ml. of dry pyridine was heated at 60°C under an argon atmosphere and then stirred overnight at ambient temperature. Additional p-toluenesulfonyl chloride (458 mg., 2.4 mmol.) was added and the reaction was reheated at 65°C. for an additional 1 hour. The reaction was cooled to room temperature and diluted with 80 ml. of ether. The mixture was washed with water (3 x 50 ml.), a saturated solution of sodium bicarbonate (75 ml.) and brine (75 ml.). The mixture was dried over anhydrous sodium sulfate and the ether removed in vacuo yielding the crude product as a colorless oily residue. The crude product was column chromatographed on silica gel (500 ml.) eluting with 10% ethyl acetate/hexanes (3000 ml.) followed by eluting with 20% ethyl acetate/hexanes (4000 ml.). The appropriate fractions were combined and the solvents removed in vacuo yielding 1.1 g. of the title compound as a colorless solid.

H. [1R-(1α,2β,3α,4β)]-9-[2-Fluoro-3,4-bis[(phenylmethoxy)methyl]cyclobutyl]-6-(phenylmethoxy)-9H-purin-2-amine

A mixture of 1.1 g. (2.77 mmol) of [1S-(1α,2α,3β,4α)-2-fluoro-3,4-bis[(phenylmethoxy)methyl]cyclobutanol, 4-methylbenzenesulfonate ester, 2.74 g. (11.35 mmol.) O-benzylguanine and 3.0 g. (11.35 mmol) 18-crown-6 in 9 ml. of dry dimethylformamide was heated at 110°C. under an argon atmosphere for 20 hours. The reaction mixture was cooled to room temperature and was filtered and the solids washed with methanol. The solvents were evaporated in vacuo yielding a brown oily residue which was column chromatographed on silica gel (400 ml.) eluting with hexanes (1200 ml.), 20% ethyl acetate/hexanes (1200 ml.) and 40% ethyl acetate/hexanes (2000 ml.). The appropriate fractions were combined and the solvents removed in vacuo yielding 248 mg. of the title compound as a colorless solid.

J. [1R-(1α,2β,3α,4β)]-2-amino-9-[2-fluoro-3,4-bis(hydroxymethyl)cyclobutyl]-1,9-dihydro-6H-purin-6-one

A mixture of 245 mg. (0.443 mmol) of [1R-(1α, 2β,3α,4β)]-9-[2-fluoro-3,4-bis[(phenylmethoxy)methyl]cyclobutyl]-6-(phenylmethoxy)-9H-purin-2-amine, 135 mg. of 20% palladium hydroxide, and 15 ml. cyclohexene were dissolved in 30 ml. of 95% ethanol and the mixture heated at reflux for 18 hours. The reaction mixture was cooled to room temperature, filtered through celite and the filter cake washed with additional ethanol. The solvents were removed in vacuo yielding the crude product as a colorless oil. The crude material was column chromatographed on CHP-20 resin eluting with water (100 ml.) followed by eluting with a 40% acetonitrile-water/water gradient (600 ml.). The appropriate fractions were combined and the solution concentrated in vacuo to remove acetonitrile. The resulting aqueous solution was lyophilized yielding 94 mg. (75%) of the title compound as a colorless solid, m.p. 235°C. (dec.), $[\alpha]_D^{22}$ = +8.4 (c = 1, water).

## Example 10

Treatment of Viral Infection in Cell Culture *in Vitro*

Assays were performed in cell culture systems to determine the concentrations of compounds that are effective in preventing several kinds of viral infections. The assays are described below, and-the results are presented in Table 1.

Abbreviations:

HSV-1 (herpes simplex virus type 1, strain Schooler), HSV-2 (herpes simplex virus type 2, strain 186), VZV (varicella zoster virus, strain ELLEN), HCMV (human cytomegalovirus, strain AD 169), MuLV (murine leukemia virus, strain CAS).

Cell Culture Assays:

HSV-1, HSV-2, HCMV, and VZV antiviral assays: Virus was adsorbed to WI-38 cell culture monolayers in 6 well culture plates (Costar, Cambridge, MA) for 1 hour prior to addition of maintenance medium containing duplicate dilutions of the test compound. Inhibition of plaque development was evaluated on fixed and stained monolayers after 4 days incubation at 37°C for HSV-1 and HSV-2 and after 6-7 days incubation at 37°C for HCMV and VZV. $ID_{50}$ values were determined from the drug concentration which conferred at least a 50% plaque reduction compared to virus controls.

MuLV antiviral assay: Antiviral assays using MuLV were performed with some modification, as described by Rowe et al. and Shannon et al.. SC-1 cells were planted at approximately 2 x $10^5$ cell per well in 6 well plates. After overnight incubation at 37°C, the cell cultures were sensitised with DEAS-Dextran for one hour at 37°C, rinsed and inoculated with MuLV. Cultures were re-fed with growth medium containing different concentrations of the test compound. After three more days at 37°C, cultures were re-fed with fresh medium plus test compounds and incubated at 37°C for an additional 3 days. Cultures were then washed to remove medium, ultraviolet light irradiated, and planted with approxi-

mately 5 x $10^5$ XC cells per well in cell growth medium containing the appropriate concentration of the test compound. The cultures were then incubated for an additional 4 days, with a re-feed using growth medium containing test compound at the second day following XC cell overlay. Finally the cultures were rinsed, stained and syncytial plaques were counted.

References:

Rowe, W.P., Pugh, W.E., and Hartley, J.W., (1970), Plaque Assay Techniques for Murine Leukemia Viruses, Virology, 42: 1136-1139.

Shannon, W.M., Brockman, R.W., Westbrook L., Shaddix, S., and Shabel, F.M., (1974), Inhibition of Gross Leukemia Virus-Induced Plaque Formation in XC Cells by 3-Deazauridine, J. Natl. Cancer Inst., 52:199-205.

Table 1

| Compound | ID$_{50}$(μM) for the following viruses | | | | |
|---|---|---|---|---|---|
| | HSV-1 | HSV-2 | VZV | HCMV | MuLV |
| I | 0.7 - 1.8 | 0.7 | 1.8 - 3.5 | 3.5 - 35 | 3.5 - 7 |
| II | 0.7 - 1.8 | 0.4 - 0.7 | ≤0.7 | 18 - 35 | ND* |
| III | 37 | 7.5 - 19 | 37 | 37 | ND* |
| IV | >286 | >286 | 28.6-286 | >286 | ND* |
| V | >252 | >252 | 2.5-25.2 | >252 | ND* |
| *ND = not determined | | | | | |

Compound I is (1α,2β,3α,4β)-2-Amino-9-[2-fluoro-3,4-bis(hydroxymethyl)cyclobutyl]-1,9-dihydro-6H-purin-6-one.
Compound II is [1S-(1α,2β,3α,4β)]-2-Amino-9-[2-fluoro-3,4-bis(hydroxymethyl)cyclobutyl]-1,9-dihydro-6H-purin-6-one.
Compound III is (1α,2β,3α,4β)-3-(6-Amino-9H-purin-9-yl)-4-fluoro-1,2-cyclobutanedimethanol
Compound IV is [1S-(1α(E),2β,3α,4β)]-1-[2-fluoro-3,4-bis(hydroxymethyl)cyclobutyl]-5-(2-bromoethenyl)-2,4-(1H,3H)-pyrimidinedione.
Compound V is [1S-(1α(E),2β,3α,4β)]-1-[2-fluoro-3,4-bis(hydroxymethyl)cyclobutyl]-5-(2-iodoethenyl)-2,4-(1H,3H)-pyrimidinedione.

## Claims

**1.** A compound having the formula

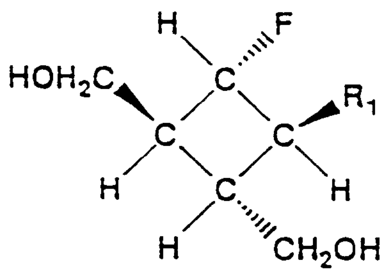

and its pharmaceutically acceptable salts wherein $R_1$ is

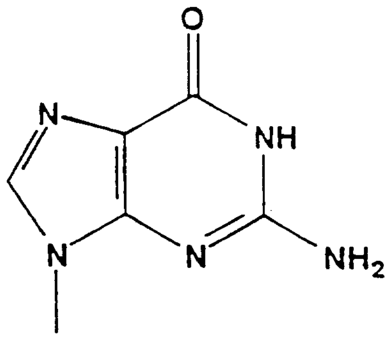

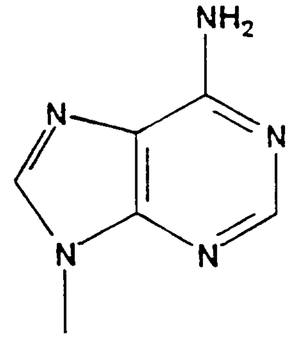

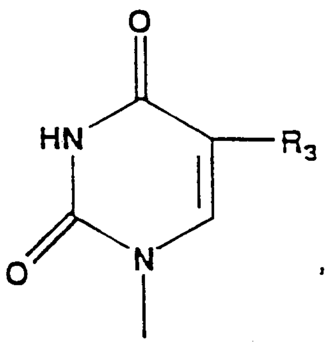

, or

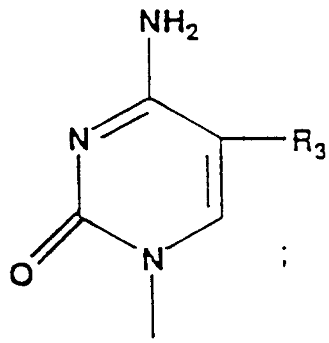

;

$R_3$ is hydrogen, methyl, fluoro, chloro, bromo, iodo, trifluoromethyl, ethyl, n-propyl, 2-fluoroethyl, 2-chloroethyl, or

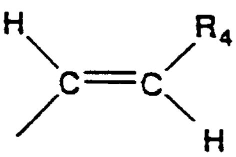

(trans)

;

and $R_4$ is chloro, bromo, iodo, hydrogen, methyl, or trifluoromethyl.

2. A compound in accordance with claim 1 wherein $R_1$ is

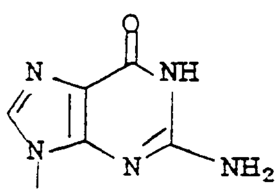

or

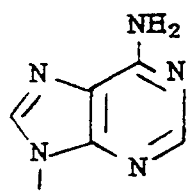

.

3. A compound in accordance with claim 2 wherein $R_1$ is

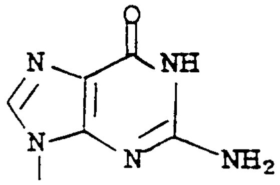

.

4. A compound in accordance with claim 2 wherein $R_1$ is

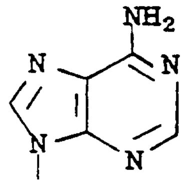

.

5. A compound in accordance with Claim 1, (1α,2β,3α,4β)-2-amino-9-[2-fluoro-3,4-bis(hydroxymethyl)cyclobutyl]-1,9-dihydro-6H-purin-6-one.

6. A compound in accordance with claim 5, [1S-(1α,2β,3α,4β)]-2-amino-9-[2-fluoro-3,4-bis(hydroxymethyl)cyclobutyl]-1,9-dihydro-6H-purin-6-one.

7. A compound in accordance with Claim 5, [1R-(1α,2β,3α,4β)]-2-amino-9-[2-fluoro-3,4-bis-(hydroxymethyl)cyclobutyl]-1,9-dihydro-6H-purin-6-one.

8. A compound in accordance with Claim 1, (1α,2β,3α,4β)-3-(6-amino-9H-purin-9-yl)-4-fluoro-1,2-cyclobutanedimethanol.

9. A compound in accordance with Claim 1, (1α,2β,3α,4β)-1-[2-fluoro-3,4-bis(hydroxymethyl)cyclobutyl]-5-methyl-2,4-(1H,3H)-pyrimidinedione.

10. A compound in accordance with Claim 1, (1α,2β,3α,4β)-1-[2-fluoro-3,4-bis(hydroxymethyl)cyclobutyl]-5-iodo-2,4(1H,3H)-pyrimidinedione.

11. A compound in accordance with Claim 1 wherein $R_1$ is

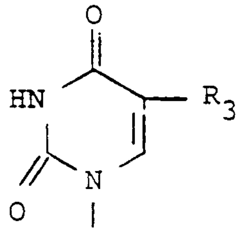

;

$R_3$ is

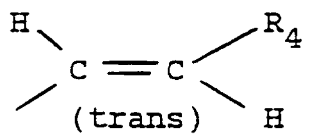

;

and $R_4$ is Br or I.

**12.** A compound in accordance with Claim 1, [1S-(1α(E), 2β,3α,4β)]-1-[2-fluoro-3,4-bis(hydroxymethyl)cyclobutyl]-5-(2-iodoethenyl)-2,4(1H,3H)pyrimidinedione.

**13.** A compound in accordance with Claim 1, [1S-(1α(E),2β,3α,4β)]-1-[2-fluoro-3,4-bis(hydroxymethyl)cyclobutyl]-5-(2-bromoethenyl)-2,4(1H,3H)pyrimidinedione.

**14.** A compound in accordance with Claim 1, [1S-(1α,2β,3α,4β)]-4-amino-1-[2-fluoro-3,4-bis(hydroxymethyl)cyclobutyl]-2(1H)pyrimidinone.

**15.** A compound of the formula

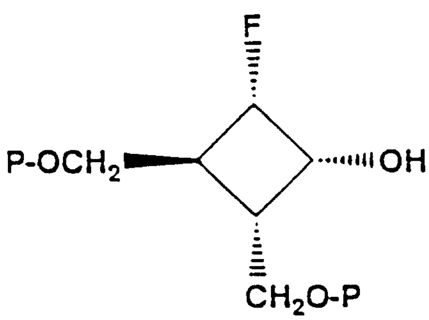

wherein P is a hydroxy protecting group selected from benzyl, t-butyldimethylsilyl, t-butyldiphenylsilyl, (triphenylmethyl)dimethylsilyl, methyldiisopropylsilyl and triisopropylsilyl and the absolute stereochemistry at the position bearing the secondary hydroxy group is R.

**16.** A compound of the formula

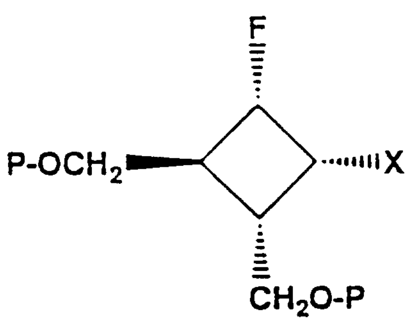

wherein X is a leaving group selected from benzenesulfonyloxy, p-toluenesulfonyloxy, methanesulfonyloxy, p-nitrophenylsulfonyloxy, and trifluoromethylsulfonyloxy; and P is a hydroxy protecting group selected from benzyl, t-butyldimethylsilyl, t-butyldiphenylsilyl, (triphenylmethyl)dimethylsilyl, methyldiisopropylsilyl, and triisopropylsilyl; and the absolute stereochemistry at the position bearing the group X is R.

**17.** A process for the preparation of the optically active compound 10a

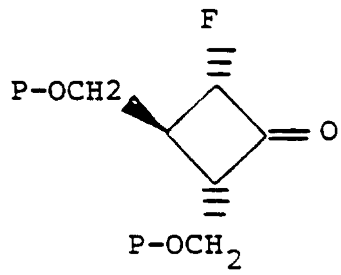

10a

wherein P is a hydroxy protecting group selected from benzyl, t-butyldimethylsilyl, t-butyldiphenylsilyl, (triphenylmethyl)dimethylsilyl, methyldiisopropylsilyl, and triisopropylsilyl, which comprises

(a) reacting a compound of the formula 30

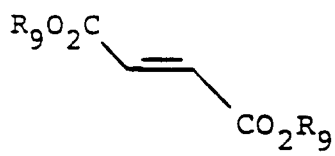

30

with a compound of the formula 31

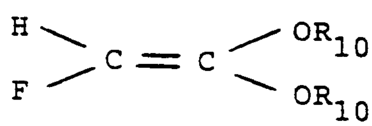

31

in the presence of a Lewis acid to yield a mixture of isomers of the formulas 32a and 32b

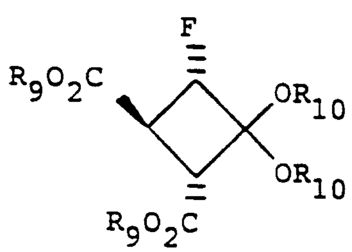

and

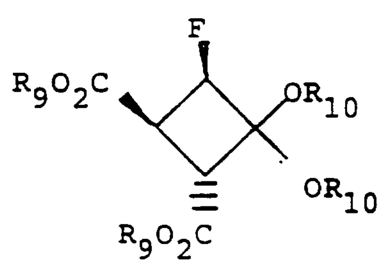

32a

32b

wherein $R_9$ is

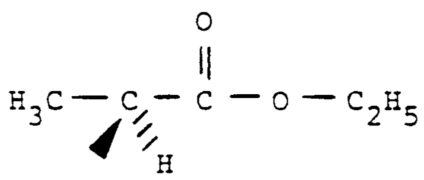 , 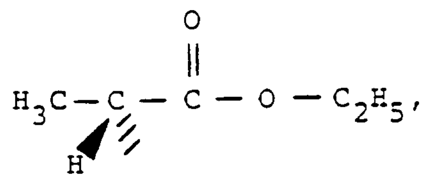

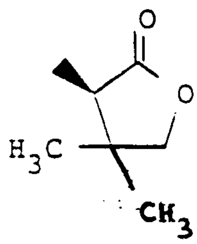 , 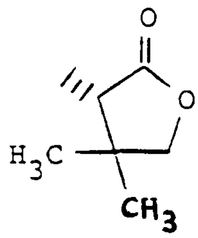 , 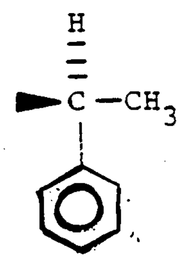 .

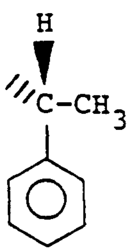 , 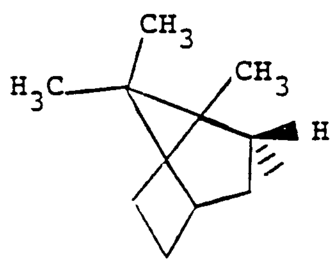 , 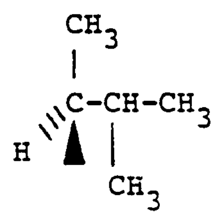 ,

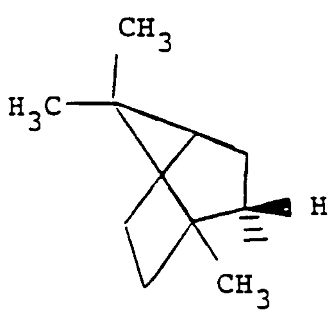 , 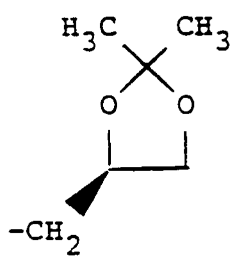 , 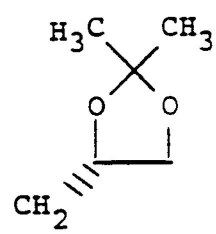 or

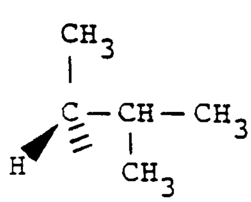 ;

and

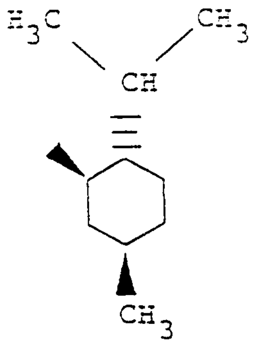 , 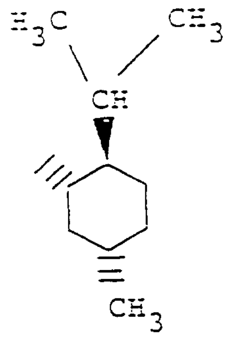 ,

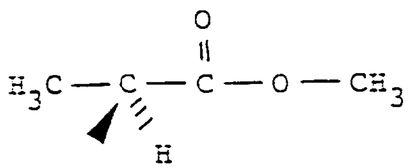 , 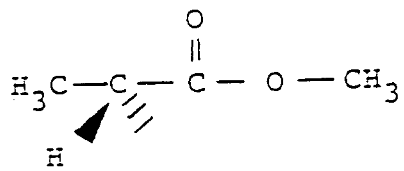 ,

$R_{10}$ is lower alkyl of 1 to 5 carbons or both $R_{10}$ groups are joined together by an alkylene group of 2 or 3 carbons;

(b) reacting the mixture of 32a and 32b with a reducing agent to form the corresponding mixture of compounds of the formula 33a and 33b

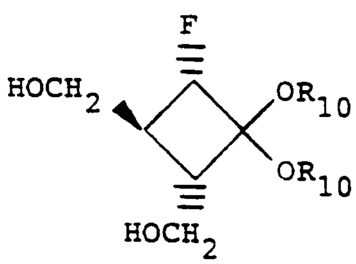

33a

and

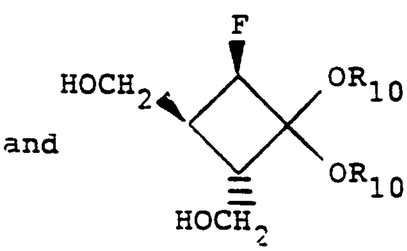

33b

(c) protecting the hydroxy groups of the mixture of compounds 33a and 33b with a group (P) to form the corresponding mixture of compounds of the formula 34a and 34b

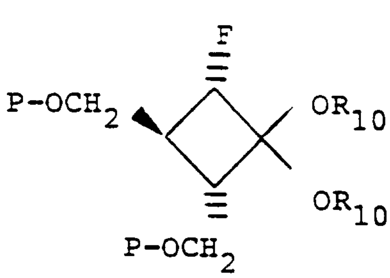

34a

and

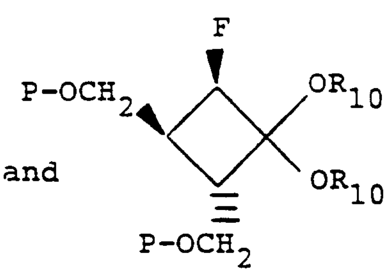

34b

(d) hydrolyzing the mixture of compounds of formula 34a and 34b using an acid catalyst to form the mixture of compounds of formula 10a and 10b

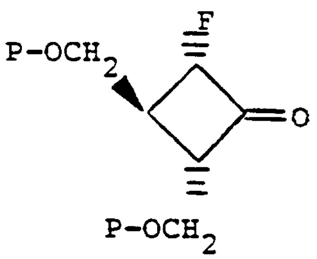

10a

and

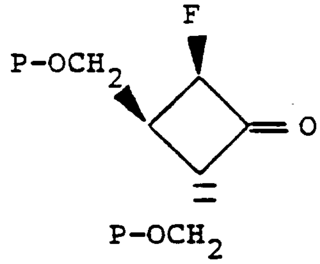

10b

(e) treating the mixture of isomers 10a and 10b with a basic catalyst to effect epimerization of the fluorine so that 10a becomes the predominant or exclusive isomer of the mixture.

**18.** The process according to Claim 17 wherein the Lewis acid is selected from the group consisting of diethylaluminum chloride, diisobutylaluminum chloride, ethylaluminum dichloride, isopropoxyaluminum dichloride, aluminum trichloride, boron trifluoride, boron trichloride, titanium tetrachloride, dichlorodiisopropoxytitanium, tin tetrachloride, and tin trichloride.

**19.** The process according to Claim 17 wherein the Lewis acid is a di(lower alkyl)aluminum chloride wherein each lower alkyl is of 2 to 4 carbons, and $R_{10}$ is methyl or ethyl.

**20.** The process according to Claim 17 wherein
$R_9$ is

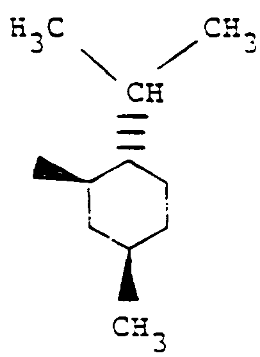

;

$R_{10}$ is ethyl; and
the Lewis acid is diethylaluminum chloride.

**21.** A compound having the formula 32a or 32b

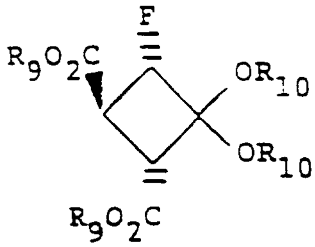

32a

or

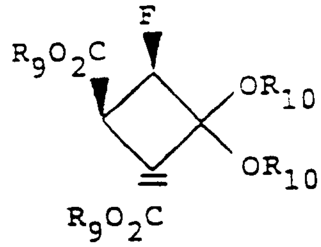

32b

wherein $R_9$ and $R_{10}$ are as defined in claim 17.

**22.** A compound according to Claim 21 wherein
$R_9$ is

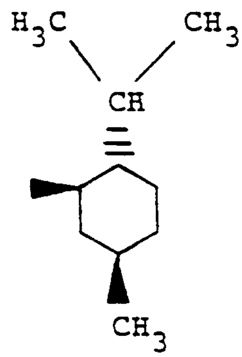

;

and $R_{10}$ is ethyl.

**23.** A compound according to Claim 22 [1S-(1α,2β,4α)]-4-fluoro-3,3-diethoxy-1,2-cyclobutanedicarboxylic acid, bis-(-)-menthyl ester or [1S-(1α,2β,4β)]-4-fluoro-3,3-diethoxy-1,2-cyclobutanedicarboxylic acid, bis-(-)-menthyl ester.

**24.** A compound having the formula 10a or 10b

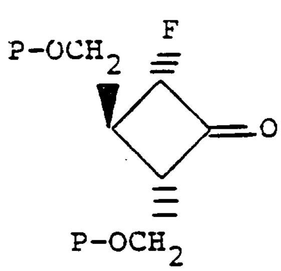

10a

or

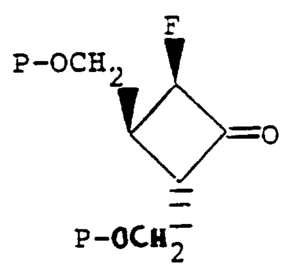

10b

wherein P is as defined in claim 17 and wherein the absolute stereochemistry at the 2-position of 10a is S and the absolute stereochemistry at the 2-position is 10b is R.

**25.** A compound according to Claim 24, [2R-(2α,3α,4β)-2-fluoro-3,4-bis[(phenylmethoxy)methyl]cyclobutanone [2S-(2α,3β,4α)-2-fluoro-3, 4-bis[(phenylmethoxy)methyl]cyclobutanone.

**26.** A pharmaceutical composition comprising a compound of any of Claims 1 to 14.

**27.** An antiviral agent comprising a compound of any of Claims 1 to 14.

**28.** Use of a compound of any of Claims 1 to 14 for the preparation of an antiviral agent.

**29.** A process for preparing compounds of formula 1 according to claim 1

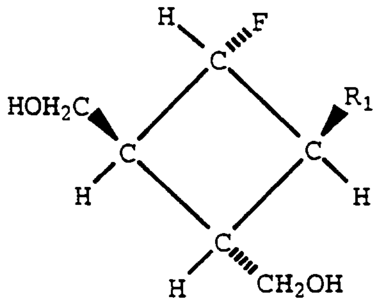

1

and pharmaceutically acceptable salts comprising

a) when $R_1$ is

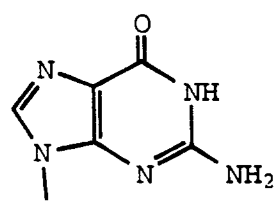

reacting an intermediate of formula 2

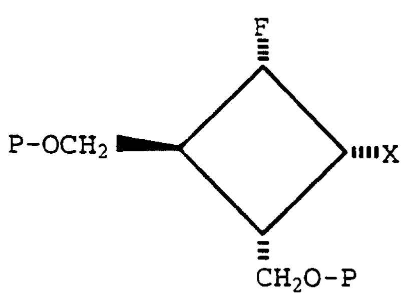

2

wherein X is a leaving group selected from benzenesulfonyloxy, p-toluenesulfonyloxy, methanesulfonyloxy, p-nitrophenylsulfonyloxy, and trifluoromethylsulfonyloxy; and

P is a hydroxy protecting group selected from benzyl, t-butyldimethylsilyl, t-butyldiphenylsilyl, (triphenylmethyl) dimethylsilyl, methyldiisopropylsilyl and triisopropylsilyl; with a benzyl protected guanine of formula 3

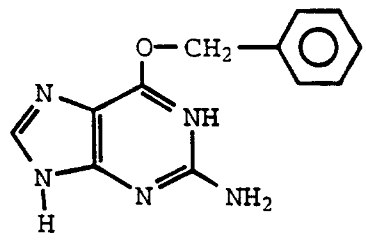

3

followed by removal of the P hydroxy protecting groups and the guanine benzyl protecting group;

b) when $R_1$

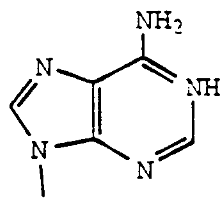 , 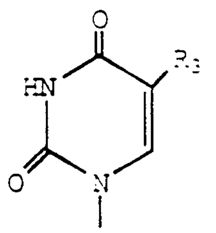 , or 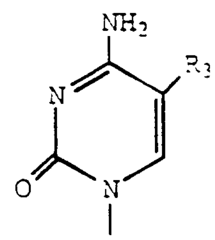 ,

and $R_3$ is hydrogen, methyl, fluoroethyl, n-propyl, 2-chloroethyl, or 2-fluoroethyl, reacting an intermediate of formula 2 with a compound of the formula

$R_1$-H

followed by removal of the P hydroxy protecting groups;

c) when $R_1$ is

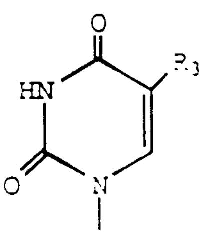 , or 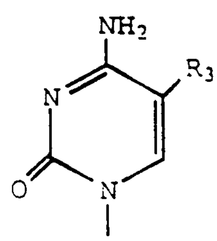

and $R_3$ is chloro, bromo or iodo, reacting a compound of formula 1 wherein $R_1$ is

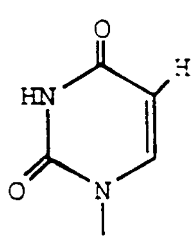 or 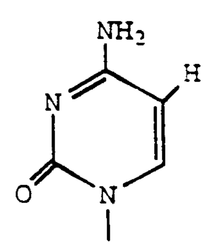

with a source of chlorine, bromine, or iodine; and

d) when $R_1$ is

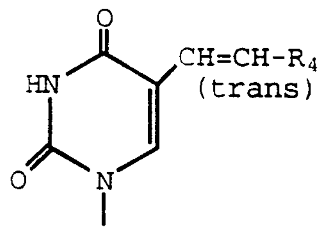
or
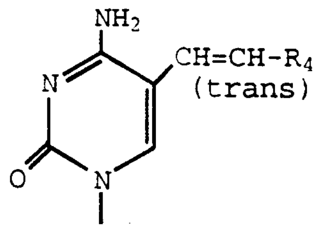

and $R_4$ is chloro, bromo or iodo, hydrogen, methyl or trifluoromethyl, reacting a compound of formula 1 wherein $R_1$ is

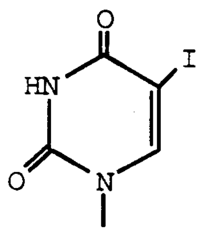
or
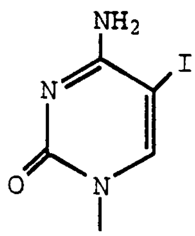

with an organopalladium compound by methods known in the art

## Patentansprüche

1. Verbindung der Formel

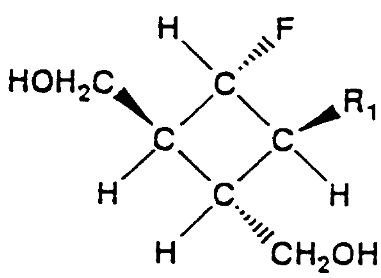

und ihre pharmazeutisch verträglichen Salze, in der $R_1$

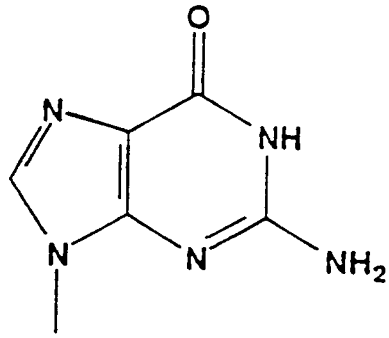

,

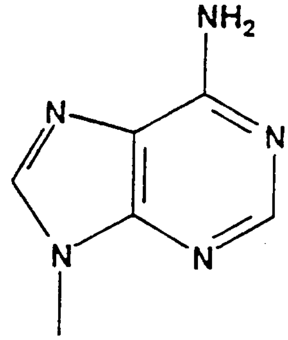

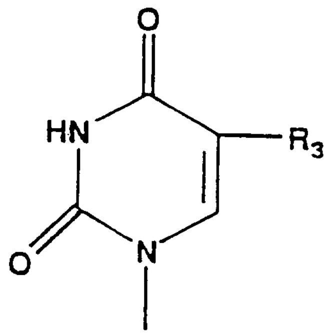

oder

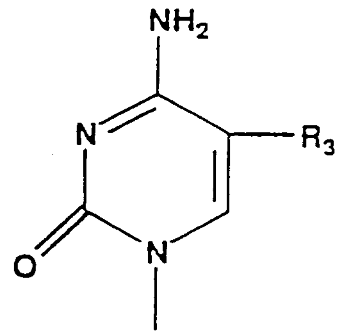

ist, $R_3$ ein Wasserstoffatom, eine Methylgruppe, ein Fluor-, Chlor-, Brom- oder Jodatom, eine Trifluormethyl-, Ethyl-, n-Propyl-, 2-Fluorethyl-, 2-Chlorethylgruppe oder

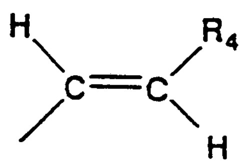

(trans)

ist und

$R_4$ ein Chlor-, Brom-, Jod- oder Wasserstoffatom, eine Methyl- oder Trifluormethylgruppe ist.

2. Verbindung nach Anspruch 1, wobei $R_1$

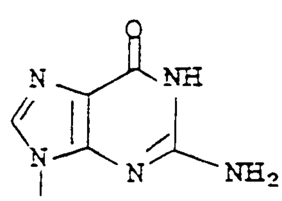

oder

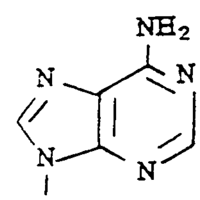

ist.

3. Verbindung nach Anspruch 2, wobei $R_1$

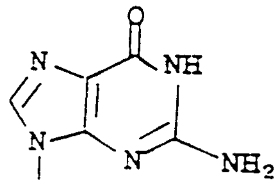

ist.

4. Verbindung nach Anspruch 2, wobei $R_1$

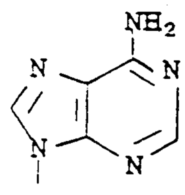

ist.

5. Verbindung nach Anspruch 1, nämlich (1α,2β,3α,4β)-2-Amino-9-[2-fluor-3,4-bis(hydroxymethyl)cyclobutyl]-1,9-dihydro-6H-purin-6-on.

6. Verbindung nach Anspruch 5, nämlich [1S-(1α,2β,3α,4β)]-2-Amino-9-[2-fluor-3,4-bis(hydroxymethyl)cyclobutyl]-1,9-dihydro-6H-purin-6-on.

7. Verbindung nach Anspruch 5, nämlich [1R-(1α,2β,3α,4β)]-2-Amino-9-[2-fluor-3,4-bis(hydroxymethyl)cyclobutyl]-1,9-dihydro-6H-purin-6-on.

8. Verbindung nach Anspruch 1, nämlich (1α,2β,3α,4β)-3-(6-Amino-9H-purin-9-yl)-4-fluor-1,2-cyclobutandimethanol.

9. Verbindung nach Anspruch 1, nämlich (1α,2β,3α,4β)-1-[2-Fluor-3,4-bis(hydroxymethyl)cyclobutyl]-5-methyl-2,4-(1H,3H)-pyrimidindion.

10. Verbindung nach Anspruch 1, nämlich (1α,2β,3α,4β)-1-[2-Fluor-3,4-bis(hydroxymethyl)cyclobutyl]-5-jod-2,4(1H,3H)-pyrimidindion.

11. Verbindung nach Anspruch 1, wobei $R_1$

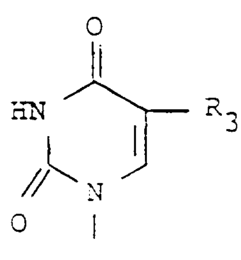

ist, $R_3$

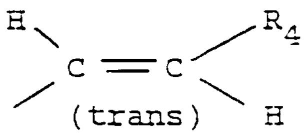

ist und $R_4$ Br oder I ist.

**12.** Verbindung nach Anspruch 1, nämlich [1S(1α(E),2β,3α,4β)]-1-[2-Fluor-3,4-bis(hydroxymethyl)cyclobutyl]-5-(2-jodethenyl)-2,4(1H,3H)-pyrimidindion.

**13.** Verbindung nach Anspruch 1, nämlich [1S(1α(E),2β,3α,4β)]-1-[2-Fluor-3,4-bis(hydroxymethyl)cyclobutyl]-5-(2-bromethenyl)-2,4(1H,3H)-pyrimidindion.

**14.** Verbindung nach Anspruch 1, nämlich [1S-(1α,2β,3α,4β)]-4-Amino-1-[2-fluor-3,4-bis(hydroxymethyl)cyclobutyl]-2(1H)pyrimidinon.

**15.** Verbindung der Formel

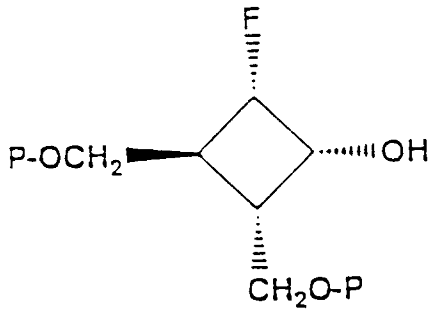

in der P eine Hydroxylschutzgruppe, ausgewählt aus Benzyl-, tert-Butyldimethylsilyl-, tert-Butyldiphenylsilyl-, (Triphenylmethyl)dimethylsilyl-, Methyldiisopropylsilyl- und Triisopropylsilylgruppen ist und die absolute Stereochemie an der die sekundäre Hydroxylgruppe tragenden Stellung R ist.

**16.** Verbindung der Formel

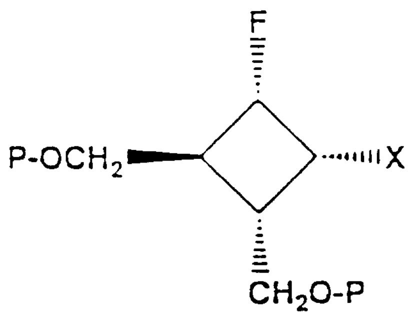

in der X eine Abgangsgruppe, ausgewählt aus Benzolsulfonyloxy-, p-Toluolsulfonyloxy-, Methansulfonyloxy-, p-Nitrophenylsulfonyloxy- und Trifluormethylsulfonyloxygruppen ist, und P eine Hydroxylschutzgruppe, ausgewählt aus Benzyl-, tert-Butyldimethylsilyl-, tert-Butyldiphenylsilyl-, (Triphenylmethyl)dimethylsilyl-, Methyldiisopropylsilyl- und Triisopropylsilylgruppen ist und die absolute Stereochemie an der die Gruppe X tragenden Stellung R ist.

**17.** Verfahren zur Herstellung der optisch aktiven Verbindung 10a

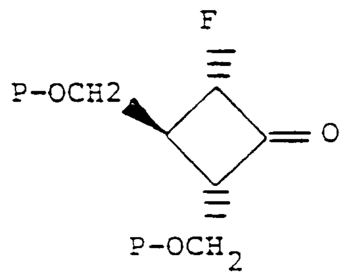

10a

wobei P eine Hydroxylschutzgruppe, ausgewählt aus Benzyl-, tert-Butyldimethylsilyl-, tert-Butyldiphenylsilyl-, (Triphenylmethyl)dimethylsilyl-, Methyldiisopropylsilyl- und Triisopropylsilylgruppen, ist, umfassend

(a) die Umsetzung einer Verbindung der Formel 30

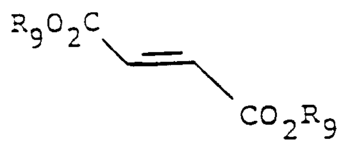

30

mit einer Verbindung der Formel 31

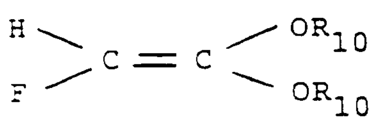

31

in Gegenwart einer Lewis-Säure zum Erhalt eines Gemisches von Isomeren der Formeln 32a und 32b

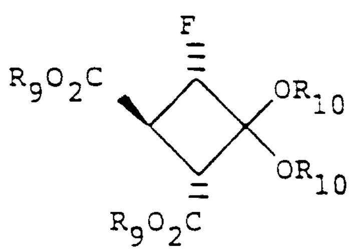

und

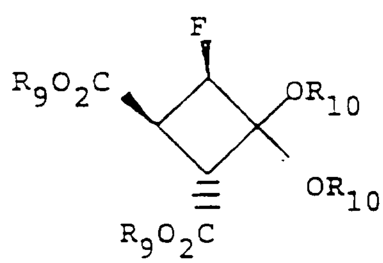

32a

32b

in denen $R_9$

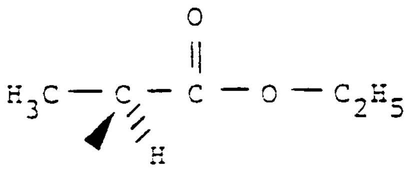 , 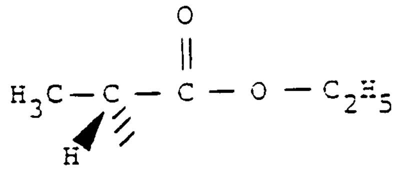 ,

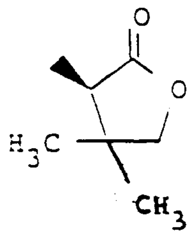 , 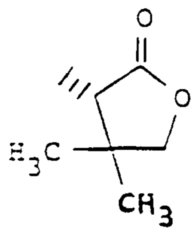 , 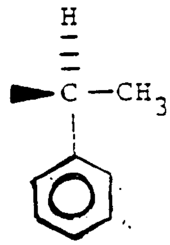 .

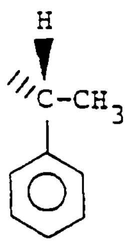 , 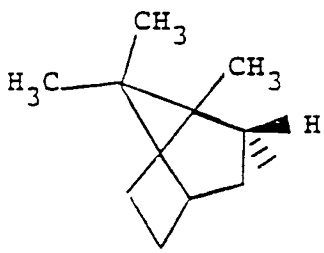 , 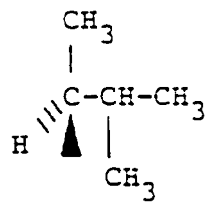 ,

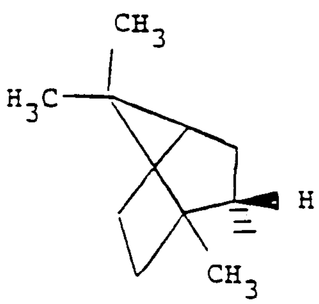 , 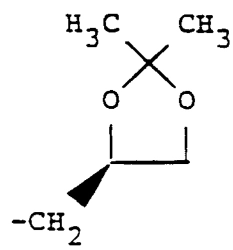 , 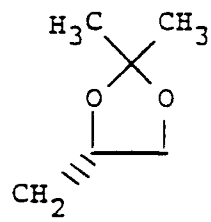 oder

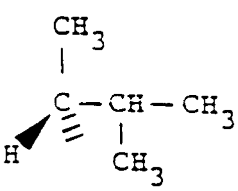 und 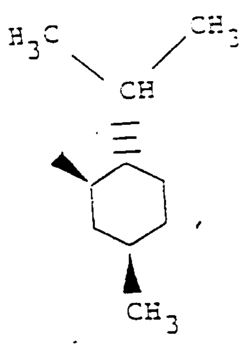 , 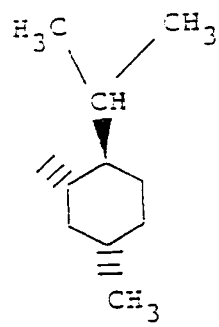 ,

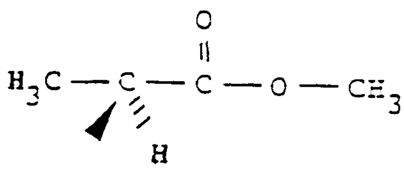 , 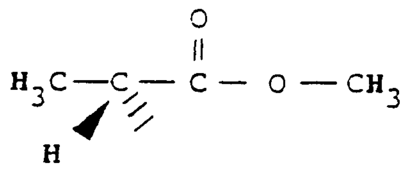 ,

$R_{10}$ ein Niederalkylrest mit 1 bis 5 Kohlenstoffatomen ist oder beide Reste $R_{10}$ zusammen einen Alkylenrest mit 2 oder 3 Kohlenstoffatomen bilden,

(b) Umsetzung des Gemisches von 32a und 32b mit einem Reduktionsmittel, wobei das entsprechende Gemisch der Verbindungen der Formel 33a und 33b gebildet wird

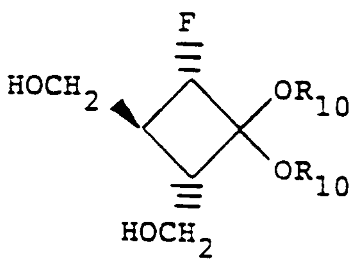

33a

und

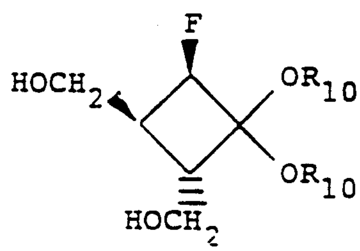

33b

(c) Schützen der Hydroxylgruppen des Gemisches der Verbindungen 33a und 33b mit einer Gruppe (P), wobei das entsprechende Gemisch der Verbindungen der Formel 34a und 34b gebildet wird

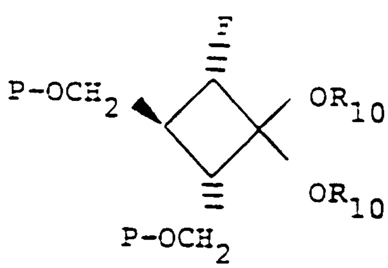

34a

und

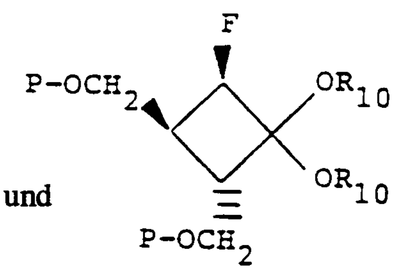

34b

(d) Hydrolysieren des Gemisches der Verbindungen der Formel 34a und 34b unter Verwendung eines saueren Katalysators, wobei das Gemisch der Verbindungen der Formel 10a und 10b gebildet wird

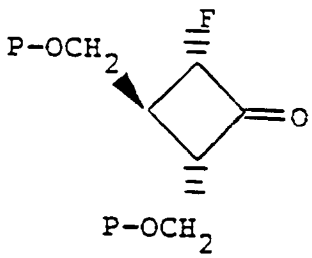

10a

und

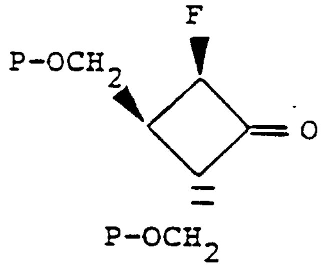

10b

(e) Umsetzung des Gemisches der Isomeren 10a und 10b mit einem basischen Katalysator, um die Epimerisierung des Fluoratoms zu bewirken, sodaß 10a das vorherrschende oder ausschließliche Isomer des Gemisches wird.

**18.** Verfahren nach Anspruch 17, wobei die Lewis-Säure aus Diethylaluminiumchlorid, Diisobutylaluminiumchlorid, Ethylaluminiumdichlorid, Isopropoxyaluminiumdichlorid, Aluminiumtrichlorid, Bortrifluorid, Bortrichlorid, Titantetrachlorid, Dichlordiisopropoxytitan, Zinntetrachlorid und Zinntrichlorid ausgewählt wird.

**19.** Verfahren nach Anspruch 17, wobei die Lewis-Säure ein Di(niederalkyl)aluminiumchlorid ist, in dem jeder Niederalkylrest 2 bis 4 Kohlenstoffatome aufweist, und $R_{10}$ eine Methyl- oder Ethylgruppe ist.

**20.** Verfahren nach Anspruch 17, wobei
$R_9$ ist,

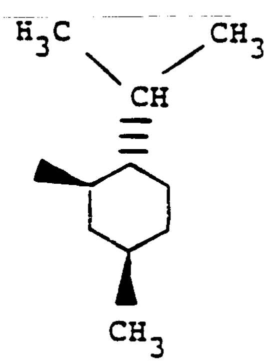

$R_{10}$ eine Ethylgruppe ist und die Lewis-Säure Diethylaluminiumchlorid ist.

**21.** Verbindung der Formel 32a oder 32b

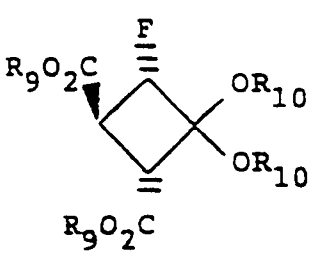

32a

oder

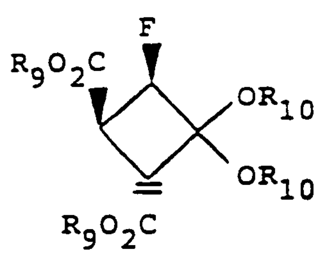

32b

in denen $R_9$ und $R_{10}$ die in Anspruch 17 angegebene Bedeutung haben.

**22.** Verbindung nach Anspruch 21, wobei $R_9$ ist, und

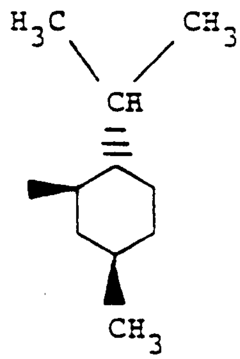

$R_{10}$ eine Ethylgruppe ist.

**23.** Verbindung nach Anspruch 22, nämlich [1S-(1α,2β,4α)]-4-Fluor-3,3-diethoxy-1,2-cyclobutandicarbonsäure-bis-(-)-menthylester oder [1S-(1α,2β,4β)]-4-Fluor-3,3-diethoxy-1,2-cyclobutandicarbonsäure-bis-(-)-menthylester.

**24.** Verbindung der Formel 10a oder 10b

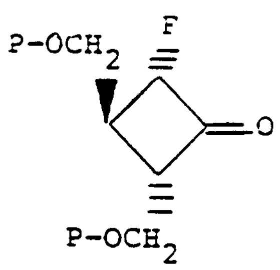

oder

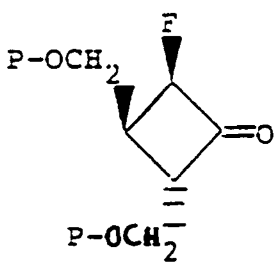

10a    10b

in denen P die in Anspruch 17 angegebene Bedeutung hat und in denen die absolute Stereochemie an der 2-Stellung von 10a S und die absolute Stereochemie an der 2-Stellung von 10b R ist.

**25.** Verbindung nach Anspruch 24, nämlich [2R-(2α,3α,4β)-2-Fluor-3,4-bis[(phenylmethoxy)methyl]cyclobutanon, [2S-(2α,3β,4α)-2-Fluor-3,4-bis[(phenylmethoxy)methyl]cyclobutanon.

**26.** Arzneimittel, umfassend eine Verbindung nach einem der Ansprüche 1 bis 14.

**27.** Antivirales Mittel, umfassend eine Verbindung nach einem der Ansprüche 1 bis 14.

**28.** Verwendung einer Verbindung nach einem der Ansprüche 1 bis 14 zur Herstellung eines antiviralen Mittels.

**29.** Verfahren zur Herstellung von Verbindungen der Formel 1 nach Anspruch 1

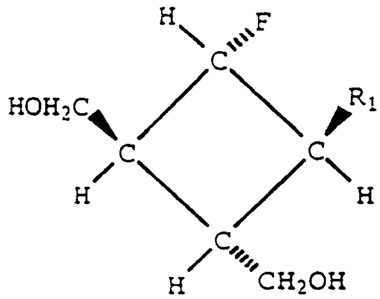

1

und pharmazeutisch verträglichen Salzen, umfassend

a) wenn $R_1$

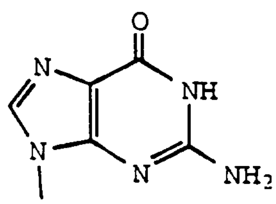

ist,
die Umsetzung eines Zwischenprodukts der Formel 2

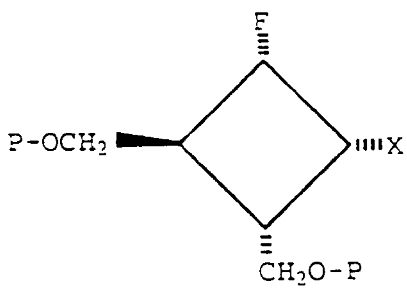

2

in der X eine Abgangsgruppe, ausgewählt aus Benzolsulfonyloxy-, p-Toluolsulfonyloxy-, Methansulfonyloxy-, p-Nitrophenylsulfonyloxy- und Trifluormethylsulfonyloxygruppen, ist, und
P eine Hydroxylschutzgruppe, ausgewählt aus Benzyl-, tert-Butyldimethylsilyl-, tert-Butyldiphenylsilyl-, (Triphenylmethyl)dimethylsilyl-, Methyldiisopropylsilyl- und Triisopropylsilylgruppen, ist,

mit einem mit einer Benzylgruppe geschützten Guanin der Formel 3

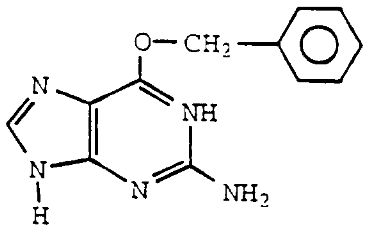

3

gefolgt von Entfernen der Hydroxylschutzgruppen P und der Guanin-Benzylschutzgruppe;

b) wenn $R_1$

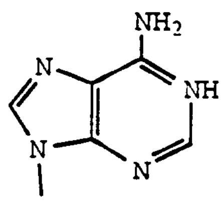 , 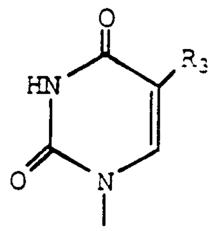 oder 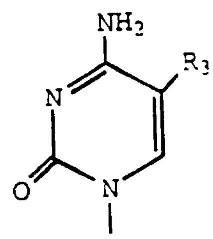

ist

und $R_3$ ein Wasserstoffatom, eine Methylgruppe, ein Fluoratom, eine Ethyl-, n-Propyl-, 2-Chlorethyl- oder 2-Fluorethylgruppe ist, Umsetzung eines Zwischenprodukts der Formel 2 mit einer Verbindung der Formel

$R_1$-H

gefolgt von Entfernen der Hydroxylschutzgruppen P,

c) wenn $R_1$

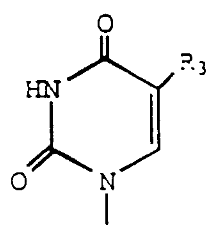 oder 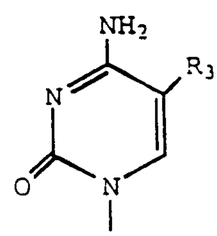

ist und $R_3$ ein Chlor-, Brom- oder Jodatom ist, Umsetzung einer Verbindung der Formel 1, in der $R_1$

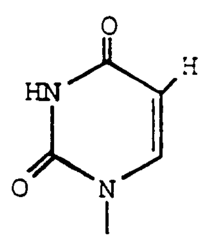 oder 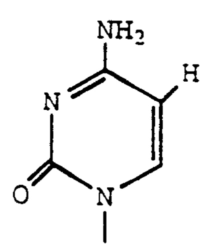

ist, mit einer Chlor-, Brom- oder Jodquelle, und

d) wenn $R_1$

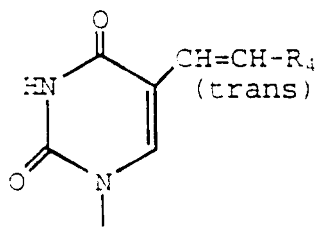

oder

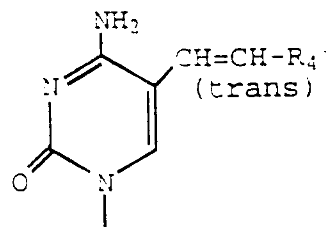

ist und $R_4$ ein Chlor-, Brom-, Jod- oder Wasserstoffatom, eine Methyl- oder Trifluormethylgruppe ist, Umsetzung einer Verbindung der Formel 1, in der $R_1$

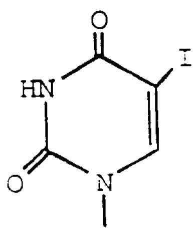

oder

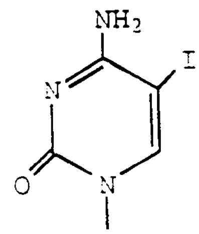

ist
mit einer Organopalladiumverbindung mit auf dem Fachgebiet bekannten Verfahren.

**Revendications**

1. Composé ayant pour formule

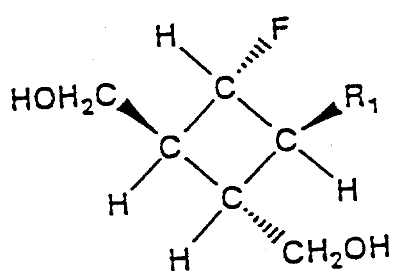

et ses sels pharmaceutiquement acceptables, formule dans laquelle $R_1$ est

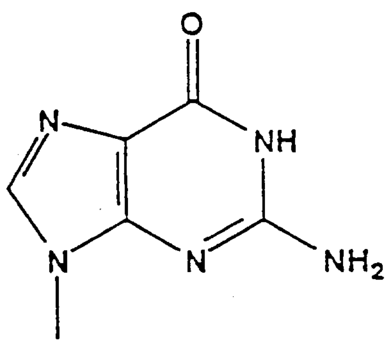 , 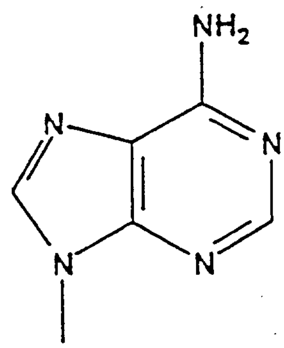

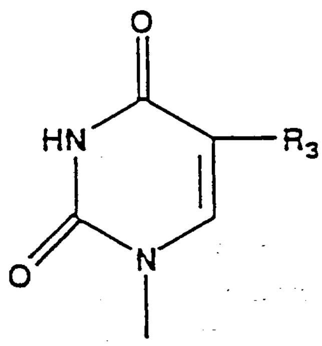 ou 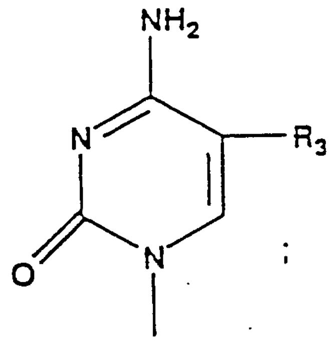 ;

$R_3$ est l'hydrogène, le groupe méthyle, le fluor, le chlore, le brome, l'iode ou le groupe trifluorométhyle, éthyle, n-propyle, 2-fluoréthyle, 2-chloréthyle, ou

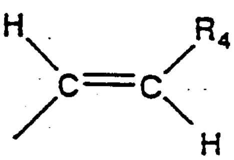 ;

(trans)

et

$R_4$ est le chlore, le brome, l'iode, l'hydrogène, ou le groupe méthyle ou trifluorométhyle.

**2.** Composé selon la revendication 1, dans lequel $R_1$ est

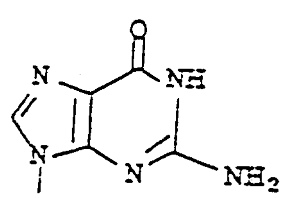 ou 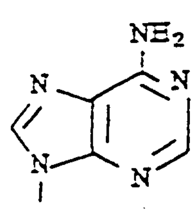 ,

3. Composé selon la revendication 2, dans lequel $R_1$ est

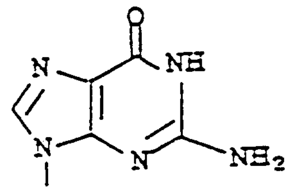

4. Composé selon la revendication 2, dans lequel $R_1$ est

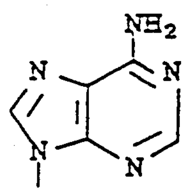

5. Composé selon la revendication 1, qui est la (1α,2β,3α,4β)-2-amino-9-[2-fluoro-3,4-bis(hydroxyméthyl)cyclobutyl]-1,9-dihydro-6H-purin-6-one.

6. Composé selon la revendication 5, qui est la [1S-(1α,2β,3α,4β)]-2-amino-9-[2-fluoro-3,4-bis(hydroxyméthyl)cyclobutyl]-1,9-dihydro-6H-purin-6-one.

7. Composé selon la revendication 5, qui est la [1R-(1α,2β,3α,4β)]-2-amino-9-[2-fluoro-3,4-bis(hydroxyméthyl)cyclobutyl]-1,9-dihydro-6H-purin-6-one.

8. Composé selon la revendication 1, qui est (1α,2β,3α,4β)-3-(6-amino-9H-purin-9-yl)-4-fluoro-1,2-cyclobutanediméthanol.

9. Composé selon la revendication 1, qui est la (1α,2β,3α,4β)-1-[2-fluoro-3,4-bis(hydroxyméthyl)cyclobutyl]-5-méthyl-2,4-(1H,3H)-pyrimidinedione.

10. Composé selon la revendication 1, qui est la (1α,2β,3α,4β)-1-[2-fluoro-3,4-bis(hydroxyméthyl)cyclobutyl]-5-iodo-2,4(1H,3H)-pyrimidinedione.

**11.** Composé selon la revendication 1, dans lequel $R_1$ est

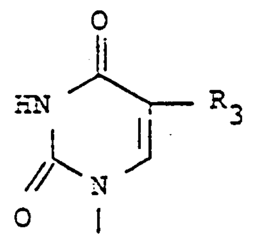

;

$R_3$ est

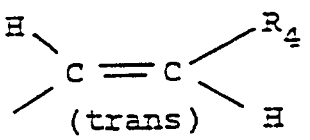

;

et

$R_4$ est le brome ou l'iode.

**12.** Composé selon la revendication 1, qui est la [1S-(1α(E),2β,3α,4β)]-1-[2-fluoro-3,4-bis(hydroxyméthyl)cyclobutyl]-5-(2-iodoéthényl)-2,4(1H,3H)pyrimidinedione.

**13.** Composé selon la revendication 1, qui est la [1S-(1α(E),2β,3α,4β)]-1-[2-fluoro-3,4-bis(hydroxyméthyl)cyclobutyl]-5-(2-bromoéthényl)-2,4-(1H,3H)-pyrimidinedione.

**14.** Composé selon la revendication 1, qui est la [1S-(1α,2β,3α,4β,)]-4-amino-1-[2-fluoro-3,4-bis(hydroxyméthyl)cyclobutyl]-2(1H)pyrimidinone.

**15.** Composé de formule

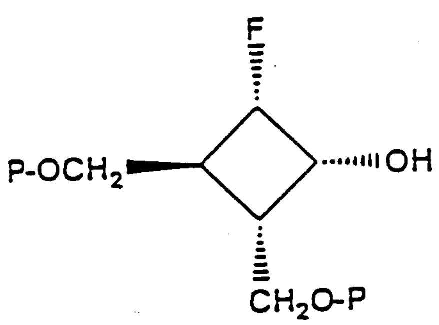

dans laquelle P est un groupe protecteur de la fonction hydroxy, choisi entre les groupes benzyle, t-butyldiméthylsilyle, t-butyldiphénylsilyle, (triphénylméthyl)diméthylsilyle, méthyldiisopropylsilyle et triisopropylsilyle, et la stéréochimie absolue sur la position qui porte le groupe hydroxy secondaire est R.

**16.** Composé de formule

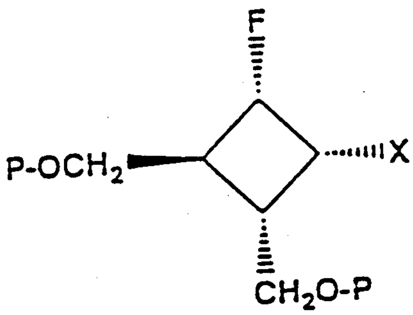

dans laquelle X est un groupe labile choisi entre les groupes benzènesulfonyloxy, p-toluènesulfonyloxy, méthanesulfonyloxy, p-nitrophénylsulfonyloxy, et trifluorométhylsulfonyloxy; et

P est un groupe protecteur de la fonction hydroxy, choisi entre les groupes benzyle, t-butyldiméthylsilyle, t-butyldiphénylsilyle, (triphénylméthyl)diméthylsilyle, méthyldiisopropylsilyle, et triisopropylsilyle; et la stéréochimie absolue sur la position qui porte le groupe X est R.

**17.** Procédé de préparation du composé optiquement actif de formule 10a

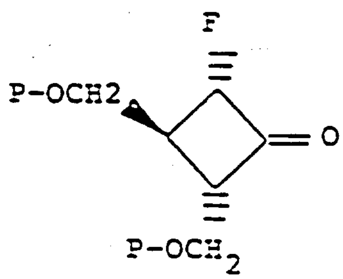

10a

dans laquelle P est un groupe protecteur de la fonction hydroxy, choisi entre les groupes benzyle, t-butyldiméthylsilyle, t-butyldiphénylsilyle, (triphénylméthyl)diméthylsilyle, méthyldiisopropylsilyle, et triisopropylsilyle, qui consiste

(a) à faire réagir un composé de formule 30

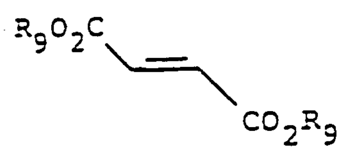

30

avec un composé de formule 31

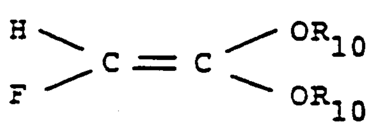

31

en présence d'un acide de Lewis, pour obtenir un mélange d'isomères de formules 32a et 32b

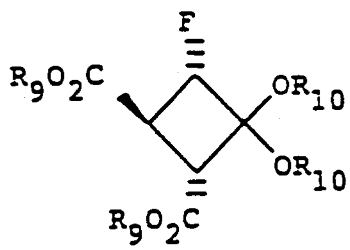

32a

et

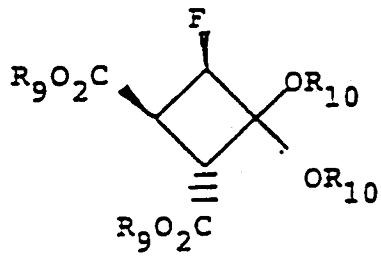

32b

dans lesquelles $R_9$ est

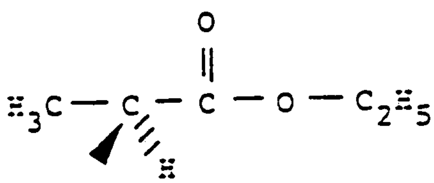

,

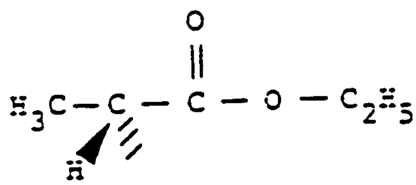

,

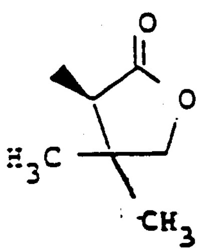

,

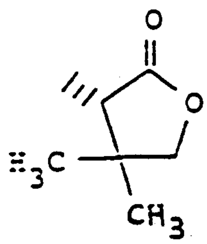

,

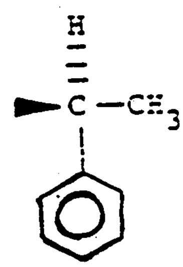

.

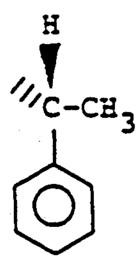

,

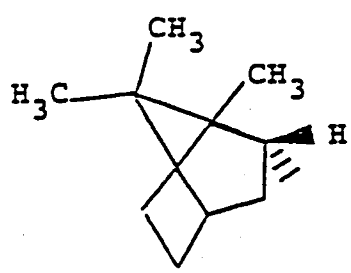

,

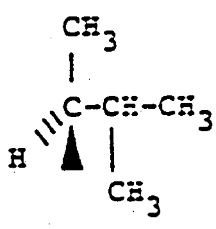

,

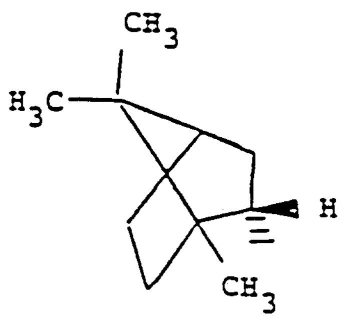 , 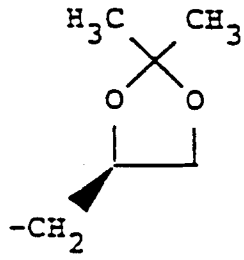 , 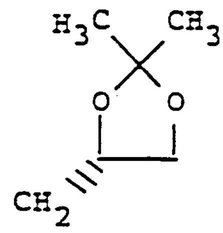

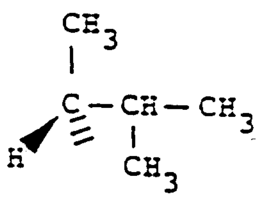 ,

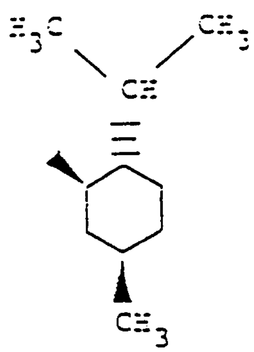 , 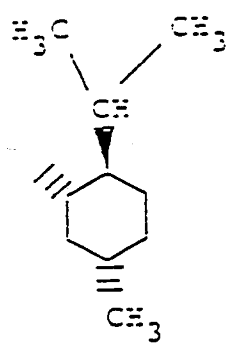 ,

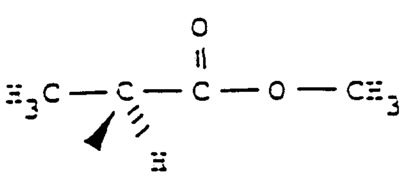 ou 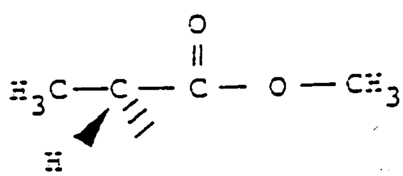 ,

$R_{10}$ est un groupe alkyle inférieur de 1 à 5 atomes de carbone, ou bien les deux groupes $R_{10}$ sont réunis par un groupe alkylène de 2 ou 3 atomes de carbone;

(b) à faire réagir le mélange de 32a et 32b avec un réducteur pour former le mélange correspondant de composés de formules 33a et 33b

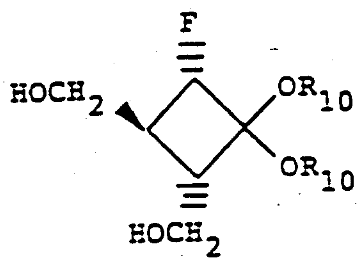

33a

et

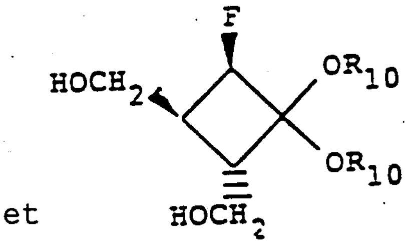

33b

(c) à protéger les groupes hydroxy du mélange de composés 33a et 33b avec un groupe (P) pour former le mélange correspondant de composés de formules 34a et 34b

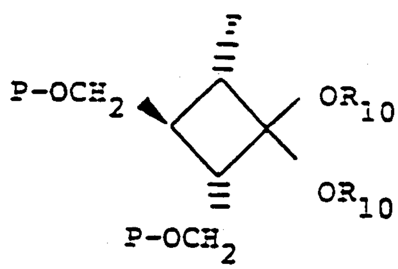

34a

et

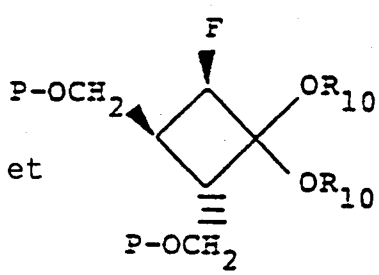

34b

(d) à hydrolyser le mélange de composés de formules 34a et 34b, en utilisant un acide comme catalyseur, pour former le mélange de composés de formules 10a et 10b

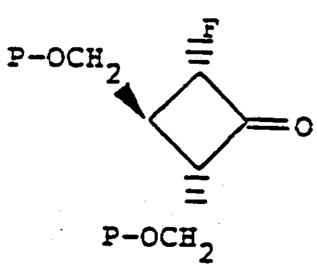

10a

et

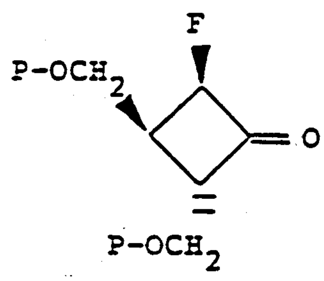

10b

(e) à traiter le mélange d'isomères 10a et 10b par un catalyseur basique pour provoquer l'épimérisation du fluor de telle sorte que 10a devienne l'isomère prédominant ou unique du mélange.

**18.** Procédé selon la revendication 17, dans lequel l'acide de Lewis est choisi dans le groupe composé du chlorure de diéthylaluminium, du chlorure de diisobutylaluminium, du dichlorure d'éthyaluminium, du dichlorure d'isopropoxyaluminium, du trichlorure d'aluminium, du trifluorure de bore, du trichlorure de bore, du tétrachlorure de titane, du dichlorodiisopropoxytitane, du tétrachlorure d'étain et du trichlorure d'étain.

**19.** Procédé selon la revendication 17, dans lequel l'acide de Lewis est un chlorure de di(alkyl inférieur)aluminium, dans lequel chaque groupe alkyle inférieur a de 2 à 4 atomes de carbone, et $R_{10}$ est le groupe méthyle ou éthyle.

**20.** Procédé selon la revendication 17, dans lequel

$R_9$ est

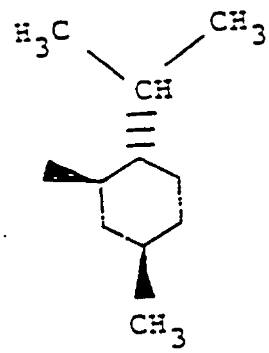

;

$R_{10}$ est le groupe éthyle; et

l'acide de Lewis est le chlorure de diéthylaluminium.

**21.** Composé de formule 32a ou 32b

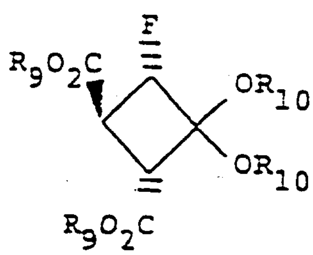

32a

ou

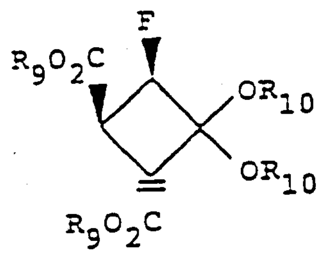

32b

dans laquelle $R_9$ et $R_{10}$ sont tels que définis dans la revendication 17.

**22.** Composé selon la revendication 21, dans lequel

$R_9$ est

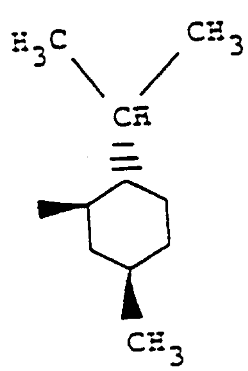

;

et

$R_{10}$ est le groupe éthyle.

**23.** Composé selon la revendication 22, qui est l'ester bis-(-)-menthylique de l'acide [1S-(1α,2β,4α)]-4-fluoro-3,3-diéthoxy-1,2-cyclobutanedicarboxylique ou l'ester bis-(-)-menthylique de l'acide [1S-(1α,2β,4β)]-4-fluoro-3,3-diéthoxy-1,2-cyclobutanedicarboxylique.

**24.** Composé de formule 10a ou 10b

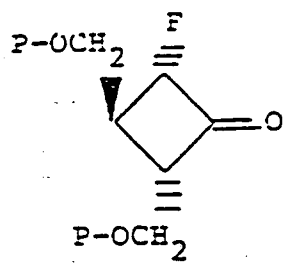

10a

ou

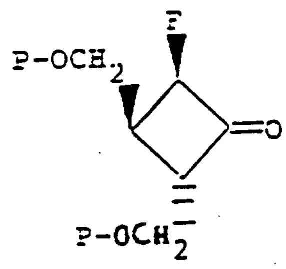

10b

où P est tel que défini dans la revendication 17 et où la stéréochimie absolue sur la position 2 de 10a est S, et la stéréochimie absolue sur la position 2 de 10b est R.

**25.** Composé selon la revendication 24, qui est la [2R-(2α,3α,4β)-2-fluoro-3,4-bis[(phénylméthoxy)méthyl]cyclobutanone [2S-(2α,3β,4α)-2-fluoro-3,4-bis[(phénylméthoxy)méthyl]cyclobutanone.

**26.** Composition pharmaceutique comprenant un composé selon l'une quelconque des revendications 1 à 14.

**27.** Agent antiviral comprenant un composé selon l'une quelconque des revendications 1 à 14.

**28.** Utilisation d'un composé selon l'une quelconque des revendications 1 à 14 pour la préparation d'un agent antiviral.

**29.** Procédé de préparation de composés de formule 1 selon la revendication 1

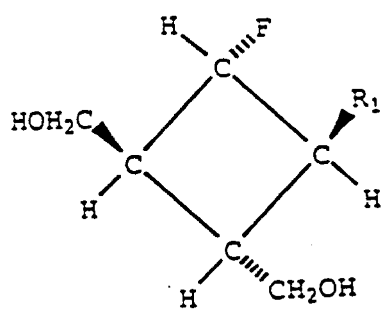

1

et de leurs sels pharmaceutiquement acceptables, consistant

a) lorsque $R_1$ est

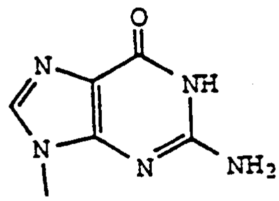

à faire réagir un intermédiaire de formule 2

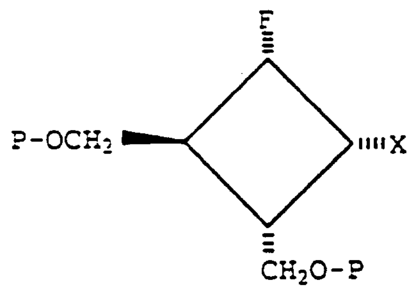

2

dans laquelle X est un groupe labile choisi entre les groupes benzènesulfonyloxy, p-toluènesulfonyloxy, méthanesulfonyloxy, p-nitrophénylsulfonyloxy et trifluorométhylsulfonyloxy; et

P est un groupe protecteur de la fonction hydroxy, choisi entre les groupes benzyle, t-butyldiméthylsilyle, t-butyldiphénylsilyle, (triphénylméthyl )diméthylsilyle, méthyldiisopropylsilyle et triisopropylsilyle;

avec une guanine, à groupement protecteur benzyle, de formule 3

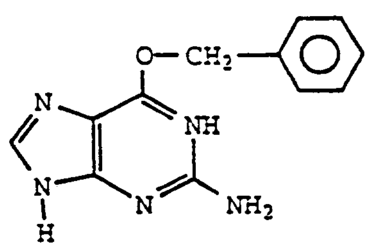

,

3.

puis à éliminer les groupes protecteurs P de la fonction hydroxy et le groupe protecteur benzyle de la guanine;

b) lorsque $R_1$ est

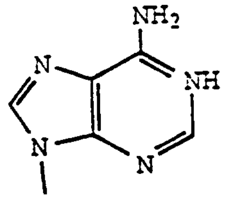

,

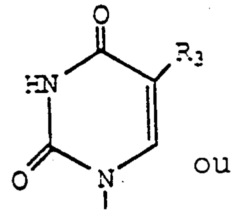

ou

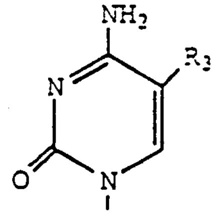

,

et que $R_3$ est l'hydrogène, le groupe méthyle, le fluor, ou le groupe éthyle, n-propyle, 2-chloréthyle, ou 2-fluoréthyle, à faire réagir un intermédiaire de formule 2 avec un composé de formule

$R_1$-H

puis à éliminer les groupes protecteurs P de la fonction hydroxy;
c) lorsque $R_1$ est

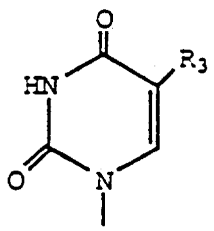 ou 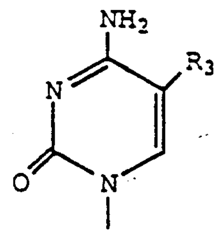

et que $R_3$ est le chlore, le brome ou l'iode, à faire réagir un composé de formule 1 dans laquelle $R_1$ est

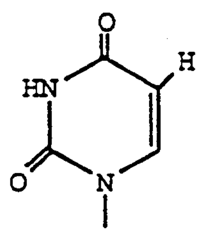 ou 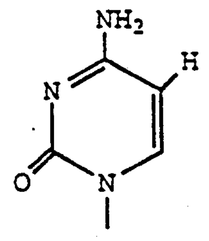

avec une source de chlore, de brome ou d'iode; et
d) lorsque $R_1$ est

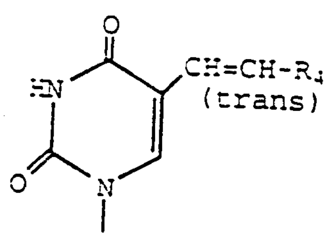 ou 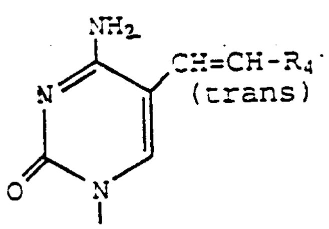

et que $R_4$ est le chlore, le brome, l'iode, l'hydrogène ou le groupe méthyle ou trifluorométhyle, à faire réagir un composé de formule 1 dans laquelle $R_1$ est

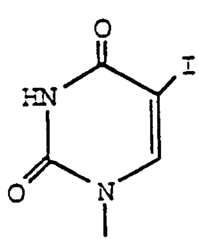 ou 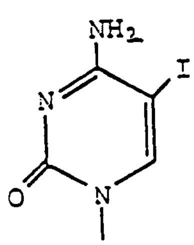

avec un composé organopalladien,
par des procédés connus dans la technique.